(19) Europäisches Patentamt
European Patent Office
Office européen des brevets

(11) EP 3 271 402 B1

(12) **EUROPEAN PATENT SPECIFICATION**

(45) Date of publication and mention of the grant of the patent:
**28.04.2021 Bulletin 2021/17**

(21) Application number: **16715793.2**

(22) Date of filing: **16.03.2016**

(51) Int Cl.:
***C07K 16/44*** $^{(2006.01)}$ ***A61K 39/395*** $^{(2006.01)}$

(86) International application number:
**PCT/DK2016/050073**

(87) International publication number:
**WO 2016/146134 (22.09.2016 Gazette 2016/38)**

(54) **ANTIBODIES TOWARDS AN EXTRACELLULAR REGION OF NBCN1**

ANTIKÖRPER GEGEN EIN EXTRAZELLULÄRES REGION NBCN1

ANTICORPS DIRIGÉ CONTRE UNE REGION EXTRACELLULAIRE DE NBCN1

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **16.03.2015 DK 201570148**

(43) Date of publication of application:
**24.01.2018 Bulletin 2018/04**

(73) Proprietor: **Aarhus Universitet**
**8000 Aarhus C (DK)**

(72) Inventor: **BØDTKJER, Ebbe, Briggs**
**8660 Skanderborg (DK)**

(74) Representative: **Høiberg P/S**
**Adelgade 12**
**1304 Copenhagen K (DK)**

(56) References cited:

- **Helle Hasager Damkier ET AL: "An anti-NH 2 -terminal antibody localizes NBCn1 to heart endothelia and skeletal and vascular smooth muscle cells", Am J Physiol Heart Circ Physiol First published August, January 2006 (2006-01), pages 172-180, XP55275089, Retrieved from the Internet: URL:http://ajpheart.physiology.org/content /ajpheart/290/1/H172.full.pdf [retrieved on 2016-05-20]**
- **LEE SOOJUNG ET AL: "Na+,HCO3--cotransport is functionally upregulated during human breast carcinogenesis and required for the inverted pH gradient across the plasma memb", PFLUEGERS ARCHIV: EUROPEAN JOURNAL OF PHYSIOLOGY, SPRINGER VERLAG, BERLIN, DE, vol. 467, no. 2, 2 May 2014 (2014-05-02), pages 367-377, XP035426602, ISSN: 0031-6768, DOI: 10.1007/S00424-014-1524-0 [retrieved on 2014-05-02]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 271 402 B1

Printed by Jouve, 75001 PARIS (FR)

SEQUENCE LISTING

<110> Aarhus Universitet

<120> NBCn1 directed agents

<130> P3746PC00

<160> 15

<170> PatentIn version 3.5

<210> 1
<211> 3399
<212> DNA
<213> human

<400> 1

```
atggaaagat ttcgtctgga gaagaagtta cctggtcctg atgaagaagc tgttgtggat      60
cttggcaaaa ctagctcaac tgtgaacacc aagtttgaaa aagaagaact agaaagtcat     120
agagctgtat atattggtgt tcacgtcccg tttagtaaag agagtcgtcg gcgtcatagg     180
catcgcggac acaaacatca ccaccggaga agaaaagata aagaatcaga taaagaagat     240
ggacgggaat ctccttctta tgatacacca tcccagagag ttcagtttat ccttggtact     300
gaagatgatg atgaagaaca tattccccat gatctcttca cggaaatgga tgaactgtgt     360
tacagagatg gagaagaata tgaatggaaa gaaactgcta gatggctgaa atttgaagag     420
gatgttgaag atggcggtga ccgatggagt aaaccttatg tggcaactct ctctttgcac     480
agtctttttg aactaaggag ttgcatcctc aatggaacag tcatgctgga tatgagagca     540
agcactctag atgaaatagc agatatggta ttagacaaca tgatagcttc tggccaatta     600
gacgagtcca tacgagagaa tgtcagagaa gctcttctga agagacatca tcatcagaat     660
gagaaaagat tcaccagtcg gattcctctt gttcgatctt ttgcagatat aggcaagaaa     720
cattctgacc ctcacttgct tgaaaggaat ggtattttgg cctctcccca gtctgctcct     780
ggaaacttgg acaatagtaa aagtggagaa attaaaggta atggaagtgg tggaagcaga     840
gaaaatagta ctgttgactt cagcaaggtt gatatgaatt tcatgagaaa aattcctacg     900
ggtgctgagg catccaacgt cctggtgggc gaagtagact ttttggaaag gccaataatt     960
gcatttgtga gactggctcc tgctgtcctc cttacagggt tgactgaggt ccctgttcca    1020
accaggtttt tgtttttgtt attgggtcca gcgggcaagg caccacagta ccatgaaatt    1080
ggacgatcaa tagccactct catgacagat gagattttcc atgatgtagc ttataaagca    1140
aaagacagaa atgacctctt atctggaatt gatgaatttt tagatcaagt aactgtccta    1200
cctccaggag agtgggatcc ttctatacgc atagaaccac caaaaagtgt cccttctcag    1260
gaaaagagaa agattcctgt gtttcacaat ggatctaccc ccacactggg tgagactcct    1320
aaagaggccg ctcatcatgc tgggcctgag ctacagagga ctggacggct tttggtggt     1380
```

ttgatacttg acatcaaaag gaaagcacct tttttcttga gtgacttcaa ggatgcatta 1440
agcctgcagt gcctggcctc gattcttttc ctatactgtg cctgtatgtc tcctgtaatc 1500
acttttggag ggctgcttgg agaagctaca gaaggcagaa taagtgcaat agagtctctt 1560
tttggagcat cattaactgg gattgcctat tcattgtttg ctgggcaacc tctaacaata 1620
ttggggagca caggtccagt tctagtgttt gaaaaaattt tatataaatt ctgcagagat 1680
tatcaacttt cttatctgtc tttaagaacc agtattggtc tgtggacttc ttttttgtgc 1740
attgttttgg ttgcaacaga tgcaagcagc cttgtgtgtt atattactcg atttacagaa 1800
gaggcttttg cagcccttat ttgcatcata ttcatctacg aggctttgga gaagctcttt 1860
gatttaggag aaacatatgc atttaatatg cacaacaact tagataaact gaccagctac 1920
tcatgtgtat gtactgaacc tccaaacccc agcaatgaaa ctctagcaca atggaagaaa 1980
gataatataa cagcacacaa tatttcctgg agaaatctta ctgtttctga atgtaaaaaa 2040
cttcgtggtg tattcttggg gtcagcttgt ggtcatcatg gaccttatat tccagatgtg 2100
ctcttttggt gtgtcatctt gtttttcaca acatttttc tgtcttcatt cctcaagcaa 2160
tttaagacca agcgttactt tcctaccaag gtgcgatcga caatcagtga ttttgctgta 2220
tttctcacaa tagtaataat ggttacaatt gactaccttg taggagttcc atctcctaaa 2280
cttcatgttc ctgaaaaatt tgagcctact catccagaga gagggtggat cataagccca 2340
ctgggagata atccttggtg gaccttatta atagctgcta ttcctgcttt gctttgtacc 2400
attctcatct ttatggatca acaaatcaca gctgtaatta taaacagaaa ggaacacaaa 2460
ttgaagaaag gagctggcta tcaccttgat ttgctcatgg ttggcgttat gttgggagtt 2520
tgctctgtca tgggacttcc atggtttgtg gctgcaacag tgttgtcaat aagtcatgtc 2580
aacagcttaa aagttgaatc tgaatgttct gctccagggg aacaacccaa gtttttggga 2640
attcgtgaac agcgggttac agggctaatg atttttattc taatgggcct ctctgtgttc 2700
atgacttcag tcctaaagtt tattccaatg cctgttctgt atggtgtttt cctttatatg 2760
ggagtttcct cattaaaagg aatccagtta tttgaccgta taaaattatt tggaatgcct 2820
gctaagcatc agcctgattt gatatacctc cgttatgtgc cgctctggaa ggtccatatt 2880
ttcacagtca ttcagcttac ttgtttggtc cttttatggg tgataaaagt ttcagctgct 2940
gcagtggttt ttcccatgat ggttcttgca ttagtgtttg tgcgcaaact catggacctg 3000
tgtttcacga agagagaact tagttggctt gatgatctta tgccagaaag taagaaaaag 3060
aaagaagatg acaaaaagaa aaaagagaaa gaggaagctg aacggatgct tcaagatgat 3120
gatgatactg tgcaccttcc atttgaaggg ggaagtctct tgcaaattcc agtcaaggcc 3180
ctaaaatata gtcctgataa acctgtgagt gtgaaaataa gttttgaaga tgaaccaaga 3240

aagaaatacg tggatgctga aacttcatta tagaattgaa ccaagaggca ttatacatat 3300

agatatatac atatgtaatg tgtgcgtatc atgtcactat atataagaat attgtatgtc 3360

atgctgttta tgtgtgacta ccgggttttt aaaagtagt 3399

<210> 2
<211> 3600
<212> DNA
<213> human

<400> 2

```
atggaggctg atggggccgg cgagcagatg agaccgctac tcacccgggg tcctgatgaa      60
gaagctgttg tggatcttgg caaaactagc tcaactgtga acaccaagtt tgaaaaagaa     120
gaactagaaa gtcatagagc tgtatatatt ggtgttcacg tcccgtttag taaagagagt     180
cgtcggcgtc ataggcatcg cggacacaaa catcaccacc ggagaagaaa agataaagaa     240
tcagataaag aagatggacg ggaatctcct tcttatgata caccatccca gagagttcag     300
tttatccttg gtactgaaga tgatgatgaa gaacatattc cccatgatct cttcacggaa     360
atggatgaac tgtgttacag agatggagaa gaatatgaat ggaaagaaac tgctagatgg     420
ctgaaatttg aagaggatgt tgaagatggc ggtgaccgat ggagtaaacc ttatgtggca     480
actctctctt tgcacagtct tttgaacta aggagttgca tcctcaatgg aacagtcatg     540
ctggatatga gagcaagcac tctagatgaa atagcagata tggtattaga caacatgata     600
gcttctggcc aattagacga gtccatacga gagaatgtca gagaagctct tctgaagaga     660
catcatcatc agaatgagaa aagattcacc agtcggattc ctcttgttcg atcttttgca     720
gatataggca agaaacattc tgaccctcac ttgcttgaaa ggaatggtat tttggcctct     780
ccccagtctg ctcctggaaa cttggacaat agtaaaagtg gagaaattaa aggtaatgga     840
agtggtggaa gcagagaaaa tagtactgtt gacttcagca aggttgatat gaatttcatg     900
agaaaaattc ctacgggtgc tgaggcatcc aacgtcctgg tgggcgaagt agactttttg     960
gaaaggccaa taattgcatt tgtgagactg gctcctgctg tcctccttac agggttgact    1020
gaggtccctg ttccaaccag gtttttgttt ttgttattgg gtccagcggg caaggcacca    1080
cagtaccatg aaattggacg atcaatagcc actctcatga cagatgagat tttccatgat    1140
gtagcttata aagcaaaaga cagaaatgac ctcttatctg gaattgatga atttttagat    1200
caagtaactg tcctacctcc aggagagtgg gatccttcta tacgcataga accaccaaaa    1260
agtgtccctt ctcaggaaaa gagaaagatt cctgtgtttc acaatggatc taccccccaca    1320
ctgggtgaga ctcctaaaga ggccgctcat catgctgggc ctgagctaca gaggactgga    1380
cggctttttg gtggtttgat acttgacatc aaaaggaaag cacctttttt cttgagtgac    1440
ttcaaggatg cattaagcct gcagtgcctg gcctcgattc ttttcctata ctgtgcctgt    1500
```

atgtctcctg taatcacttt tggagggctg cttggagaag ctacagaagg cagaataagt 1560

gcaatagagt ctctttttgg agcatcatta actgggattg cctattcatt gtttgctggg 1620

caacctctaa caatattggg gagcacaggt ccagttctag tgtttgaaaa aattttatat 1680

aaattctgca gagattatca actttcttat ctgtctttaa gaaccagtat tggtctgtgg 1740

acttcttttt tgtgcattgt tttggttgca acagatgcaa gcagccttgt gtgttatatt 1800

actcgattta cagaagaggc ttttgcagcc cttatttgca tcatattcat ctacgaggct 1860

ttggagaagc tctttgattt aggagaaaca tatgcattta atatgcacaa caacttagat 1920

aaactgacca gctactcatg tgtatgtact gaacctccaa accccagcaa tgaaactcta 1980

gcacaatgga agaaagataa tataacagca cacaatattt cctggagaaa tcttactgtt 2040

tctgaatgta aaaaacttcg tggtgtattc ttggggtcag cttgtggtca tcatggacct 2100

tatattccag atgtgctctt ttggtgtgtc atcttgtttt tcacaacatt ttttctgtct 2160

tcattcctca agcaatttaa gaccaagcgt tactttccta ccaaggtgcg atcgacaatc 2220

agtgattttg ctgtatttct cacaatagta ataatggtta caattgacta ccttgtagga 2280

gttccatctc ctaaacttca tgttcctgaa aaatttgagc ctactcatcc agagagaggg 2340

tggatcataa gcccactggg agataatcct tggtggacct tattaatagc tgctattcct 2400

gctttgcttt gtaccattct catctttatg gatcaacaaa tcacagctgt aattataaac 2460

agaaaggaac acaaattgaa gaaaggagct ggctatcacc ttgatttgct catggttggc 2520

gttatgttgg gagtttgctc tgtcatggga cttccatggt ttgtggctgc aacagtgttg 2580

tcaataagtc atgtcaacag cttaaaagtt gaatctgaat gttctgctcc aggggaacaa 2640

cccaagtttt tgggaattcg tgaacagcgg gttacagggc taatgatttt tattctaatg 2700

ggcctctctg tgttcatgac ttcagtccta aagtttattc caatgcctgt tctgtatggt 2760

gttttccttt atatgggagt ttcctcatta aaaggaatcc agttatttga ccgtataaaa 2820

ttatttggaa tgcctgctaa gcatcagcct gatttgatat acctccgtta tgtgccgctc 2880

tggaaggtcc atattttcac agtcattcag cttacttgtt tggtcctttt atgggtgata 2940

aaagtttcag ctgctgcagt ggtttttccc atgatggttc ttgcattagt gtttgtgcgc 3000

aaactcatgg acctgtgttt cacgaagaga gaacttagtt ggcttgatga tcttatgcca 3060

gaaagtaaga aaaagaaaga agatgacaaa aagaaaaaag agaaagagga agctgaacgg 3120

atgcttcaag atgatgatga tactgtgcac cttccatttg aagggggaag tctcttgcaa 3180

attccagtca aggccctaaa atatagtgtt gatccctcaa ttgttaacat atcagatgaa 3240

atggccaaaa ctgcacagtg gaaggcactt tccatgaata ctgagaatgc caaagtaacc 3300

agatctaaca tgagtcctga taaacctgtg agtgtgaaaa taagttttga agatgaacca 3360

agaaagaaat acgtggatgc tgaaacttca ttatagaatt gaaccaagag gcattataca 3420

```
tatagatata tacatatgta atgtgtgcgt atcatgtcac tatatataag aatattgtat     3480

gtcatgctgt ttatgtgtga ctaccgggtt tttaaaagta gtgtctggag tttgtaatga     3540

gcaccgtgga gactatgtat ttaatgaaat gctctctttg aagtgaggta catggttctt     3600
```

<210> 3
<211> 1090
<212> PRT
<213> human

<400> 3

```
Met Glu Arg Phe Arg Leu Glu Lys Lys Leu Pro Gly Pro Asp Glu Glu
1               5                   10                  15

Ala Val Val Asp Leu Gly Lys Thr Ser Ser Thr Val Asn Thr Lys Phe
            20                  25                  30

Glu Lys Glu Glu Leu Glu Ser His Arg Ala Val Tyr Ile Gly Val His
        35                  40                  45

Val Pro Phe Ser Lys Glu Ser Arg Arg Arg His Arg His Arg Gly His
    50                  55                  60

Lys His His His Arg Arg Arg Lys Asp Lys Glu Ser Asp Lys Glu Asp
65                  70                  75                  80

Gly Arg Glu Ser Pro Ser Tyr Asp Thr Pro Ser Gln Arg Val Gln Phe
                85                  90                  95

Ile Leu Gly Thr Glu Asp Asp Asp Glu Glu His Ile Pro His Asp Leu
            100                 105                 110

Phe Thr Glu Met Asp Glu Leu Cys Tyr Arg Asp Gly Glu Glu Tyr Glu
        115                 120                 125

Trp Lys Glu Thr Ala Arg Trp Leu Lys Phe Glu Glu Asp Val Glu Asp
    130                 135                 140

Gly Gly Asp Arg Trp Ser Lys Pro Tyr Val Ala Thr Leu Ser Leu His
145                 150                 155                 160

Ser Leu Phe Glu Leu Arg Ser Cys Ile Leu Asn Gly Thr Val Met Leu
                165                 170                 175

Asp Met Arg Ala Ser Thr Leu Asp Glu Ile Ala Asp Met Val Leu Asp
            180                 185                 190
```

```
Asn Met Ile Ala Ser Gly Gln Leu Asp Glu Ser Ile Arg Glu Asn Val
        195                 200                 205

Arg Glu Ala Leu Leu Lys Arg His His His Gln Asn Glu Lys Arg Phe
    210                 215                 220

Thr Ser Arg Ile Pro Leu Val Arg Ser Phe Ala Asp Ile Gly Lys Lys
225                 230                 235                 240

His Ser Asp Pro His Leu Leu Glu Arg Asn Gly Ile Leu Ala Ser Pro
                245                 250                 255

Gln Ser Ala Pro Gly Asn Leu Asp Asn Ser Lys Ser Gly Glu Ile Lys
            260                 265                 270

Gly Asn Gly Ser Gly Gly Ser Arg Glu Asn Ser Thr Val Asp Phe Ser
        275                 280                 285

Lys Val Asp Met Asn Phe Met Arg Lys Ile Pro Thr Gly Ala Glu Ala
    290                 295                 300

Ser Asn Val Leu Val Gly Glu Val Asp Phe Leu Glu Arg Pro Ile Ile
305                 310                 315                 320

Ala Phe Val Arg Leu Ala Pro Ala Val Leu Leu Thr Gly Leu Thr Glu
                325                 330                 335

Val Pro Val Pro Thr Arg Phe Leu Phe Leu Leu Leu Gly Pro Ala Gly
            340                 345                 350

Lys Ala Pro Gln Tyr His Glu Ile Gly Arg Ser Ile Ala Thr Leu Met
        355                 360                 365

Thr Asp Glu Ile Phe His Asp Val Ala Tyr Lys Ala Lys Asp Arg Asn
    370                 375                 380

Asp Leu Leu Ser Gly Ile Asp Glu Phe Leu Asp Gln Val Thr Val Leu
385                 390                 395                 400

Pro Pro Gly Glu Trp Asp Pro Ser Ile Arg Ile Glu Pro Pro Lys Ser
                405                 410                 415

Val Pro Ser Gln Glu Lys Arg Lys Ile Pro Val Phe His Asn Gly Ser
            420                 425                 430

Thr Pro Thr Leu Gly Glu Thr Pro Lys Glu Ala Ala His His Ala Gly
        435                 440                 445
```

Pro Glu Leu Gln Arg Thr Gly Arg Leu Phe Gly Gly Leu Ile Leu Asp
450 455 460

Ile Lys Arg Lys Ala Pro Phe Phe Leu Ser Asp Phe Lys Asp Ala Leu
465 470 475 480

Ser Leu Gln Cys Leu Ala Ser Ile Leu Phe Leu Tyr Cys Ala Cys Met
485 490 495

Ser Pro Val Ile Thr Phe Gly Gly Leu Leu Gly Glu Ala Thr Glu Gly
500 505 510

Arg Ile Ser Ala Ile Glu Ser Leu Phe Gly Ala Ser Leu Thr Gly Ile
515 520 525

Ala Tyr Ser Leu Phe Ala Gly Gln Pro Leu Thr Ile Leu Gly Ser Thr
530 535 540

Gly Pro Val Leu Val Phe Glu Lys Ile Leu Tyr Lys Phe Cys Arg Asp
545 550 555 560

Tyr Gln Leu Ser Tyr Leu Ser Leu Arg Thr Ser Ile Gly Leu Trp Thr
565 570 575

Ser Phe Leu Cys Ile Val Leu Val Ala Thr Asp Ala Ser Ser Leu Val
580 585 590

Cys Tyr Ile Thr Arg Phe Thr Glu Glu Ala Phe Ala Ala Leu Ile Cys
595 600 605

Ile Ile Phe Ile Tyr Glu Ala Leu Glu Lys Leu Phe Asp Leu Gly Glu
610 615 620

Thr Tyr Ala Phe Asn Met His Asn Asn Leu Asp Lys Leu Thr Ser Tyr
625 630 635 640

Ser Cys Val Cys Thr Glu Pro Pro Asn Pro Ser Asn Glu Thr Leu Ala
645 650 655

Gln Trp Lys Lys Asp Asn Ile Thr Ala His Asn Ile Ser Trp Arg Asn
660 665 670

Leu Thr Val Ser Glu Cys Lys Lys Leu Arg Gly Val Phe Leu Gly Ser
675 680 685

Ala Cys Gly His His Gly Pro Tyr Ile Pro Asp Val Leu Phe Trp Cys
690 695 700

Val Ile Leu Phe Phe Thr Thr Phe Phe Leu Ser Ser Phe Leu Lys Gln
705 710 715 720

Phe Lys Thr Lys Arg Tyr Phe Pro Thr Lys Val Arg Ser Thr Ile Ser
725 730 735

Asp Phe Ala Val Phe Leu Thr Ile Val Ile Met Val Thr Ile Asp Tyr
740 745 750

Leu Val Gly Val Pro Ser Pro Lys Leu His Val Pro Glu Lys Phe Glu
755 760 765

Pro Thr His Pro Glu Arg Gly Trp Ile Ile Ser Pro Leu Gly Asp Asn
770 775 780

Pro Trp Trp Thr Leu Leu Ile Ala Ala Ile Pro Ala Leu Leu Cys Thr
785 790 795 800

Ile Leu Ile Phe Met Asp Gln Gln Ile Thr Ala Val Ile Ile Asn Arg
805 810 815

Lys Glu His Lys Leu Lys Lys Gly Ala Gly Tyr His Leu Asp Leu Leu
820 825 830

Met Val Gly Val Met Leu Gly Val Cys Ser Val Met Gly Leu Pro Trp
835 840 845

Phe Val Ala Ala Thr Val Leu Ser Ile Ser His Val Asn Ser Leu Lys
850 855 860

Val Glu Ser Glu Cys Ser Ala Pro Gly Glu Gln Pro Lys Phe Leu Gly
865 870 875 880

Ile Arg Glu Gln Arg Val Thr Gly Leu Met Ile Phe Ile Leu Met Gly
885 890 895

Leu Ser Val Phe Met Thr Ser Val Leu Lys Phe Ile Pro Met Pro Val
900 905 910

Leu Tyr Gly Val Phe Leu Tyr Met Gly Val Ser Ser Leu Lys Gly Ile
915 920 925

Gln Leu Phe Asp Arg Ile Lys Leu Phe Gly Met Pro Ala Lys His Gln
930 935 940

Pro Asp Leu Ile Tyr Leu Arg Tyr Val Pro Leu Trp Lys Val His Ile

```
945                 950                 955                 960

Phe Thr Val Ile Gln Leu Thr Cys Leu Val Leu Leu Trp Val Ile Lys
                965                 970                 975

Val Ser Ala Ala Ala Val Val Phe Pro Met Met Val Leu Ala Leu Val
            980                 985                 990

Phe Val Arg Lys Leu Met Asp Leu  Cys Phe Thr Lys Arg  Glu Leu Ser
        995                 1000                1005

Trp Leu  Asp Asp Leu Met Pro  Glu Ser Lys Lys Lys  Lys Glu Asp
    1010                 1015                 1020

Asp Lys  Lys Lys Lys Glu Lys  Glu Glu Ala Glu Arg  Met Leu Gln
    1025                 1030                 1035

Asp Asp  Asp Asp Thr Val His  Leu Pro Phe Glu Gly  Gly Ser Leu
    1040                 1045                 1050

Leu Gln  Ile Pro Val Lys Ala  Leu Lys Tyr Ser Pro  Asp Lys Pro
    1055                 1060                 1065

Val Ser  Val Lys Ile Ser Phe  Glu Asp Glu Pro Arg  Lys Lys Tyr
    1070                 1075                 1080

Val Asp  Ala Glu Thr Ser Leu
    1085                 1090
```

<210> 4
<211> 1131
<212> PRT
<213> human

<400> 4

```
Met Glu Ala Asp Gly Ala Gly Glu Gln Met Arg Pro Leu Leu Thr Arg
1               5                   10                  15

Gly Pro Asp Glu Glu Ala Val Val Asp Leu Gly Lys Thr Ser Ser Thr
            20                  25                  30

Val Asn Thr Lys Phe Glu Lys Glu Glu Leu Glu Ser His Arg Ala Val
        35                  40                  45

Tyr Ile Gly Val His Val Pro Phe Ser Lys Glu Ser Arg Arg Arg His
    50                  55                  60

Arg His Arg Gly His Lys His His His Arg Arg Arg Lys Asp Lys Glu
```

65 70 75 80

Ser Asp Lys Glu Asp Gly Arg Glu Ser Pro Ser Tyr Asp Thr Pro Ser
85 90 95

Gln Arg Val Gln Phe Ile Leu Gly Thr Glu Asp Asp Asp Glu Glu His
100 105 110

Ile Pro His Asp Leu Phe Thr Glu Met Asp Glu Leu Cys Tyr Arg Asp
115 120 125

Gly Glu Glu Tyr Glu Trp Lys Glu Thr Ala Arg Trp Leu Lys Phe Glu
130 135 140

Glu Asp Val Glu Asp Gly Gly Asp Arg Trp Ser Lys Pro Tyr Val Ala
145 150 155 160

Thr Leu Ser Leu His Ser Leu Phe Glu Leu Arg Ser Cys Ile Leu Asn
165 170 175

Gly Thr Val Met Leu Asp Met Arg Ala Ser Thr Leu Asp Glu Ile Ala
180 185 190

Asp Met Val Leu Asp Asn Met Ile Ala Ser Gly Gln Leu Asp Glu Ser
195 200 205

Ile Arg Glu Asn Val Arg Glu Ala Leu Leu Lys Arg His His His Gln
210 215 220

Asn Glu Lys Arg Phe Thr Ser Arg Ile Pro Leu Val Arg Ser Phe Ala
225 230 235 240

Asp Ile Gly Lys Lys His Ser Asp Pro His Leu Leu Glu Arg Asn Gly
245 250 255

Ile Leu Ala Ser Pro Gln Ser Ala Pro Gly Asn Leu Asp Asn Ser Lys
260 265 270

Ser Gly Glu Ile Lys Gly Asn Gly Ser Gly Gly Ser Arg Glu Asn Ser
275 280 285

Thr Val Asp Phe Ser Lys Val Asp Met Asn Phe Met Arg Lys Ile Pro
290 295 300

Thr Gly Ala Glu Ala Ser Asn Val Leu Val Gly Glu Val Asp Phe Leu
305 310 315 320

Glu Arg Pro Ile Ile Ala Phe Val Arg Leu Ala Pro Ala Val Leu Leu
325 330 335

Thr Gly Leu Thr Glu Val Pro Val Pro Thr Arg Phe Leu Phe Leu Leu
340 345 350

Leu Gly Pro Ala Gly Lys Ala Pro Gln Tyr His Glu Ile Gly Arg Ser
355 360 365

Ile Ala Thr Leu Met Thr Asp Glu Ile Phe His Asp Val Ala Tyr Lys
370 375 380

Ala Lys Asp Arg Asn Asp Leu Leu Ser Gly Ile Asp Glu Phe Leu Asp
385 390 395 400

Gln Val Thr Val Leu Pro Pro Gly Glu Trp Asp Pro Ser Ile Arg Ile
405 410 415

Glu Pro Pro Lys Ser Val Pro Ser Gln Glu Lys Arg Lys Ile Pro Val
420 425 430

Phe His Asn Gly Ser Thr Pro Thr Leu Gly Glu Thr Pro Lys Glu Ala
435 440 445

Ala His His Ala Gly Pro Glu Leu Gln Arg Thr Gly Arg Leu Phe Gly
450 455 460

Gly Leu Ile Leu Asp Ile Lys Arg Lys Ala Pro Phe Phe Leu Ser Asp
465 470 475 480

Phe Lys Asp Ala Leu Ser Leu Gln Cys Leu Ala Ser Ile Leu Phe Leu
485 490 495

Tyr Cys Ala Cys Met Ser Pro Val Ile Thr Phe Gly Gly Leu Leu Gly
500 505 510

Glu Ala Thr Glu Gly Arg Ile Ser Ala Ile Glu Ser Leu Phe Gly Ala
515 520 525

Ser Leu Thr Gly Ile Ala Tyr Ser Leu Phe Ala Gly Gln Pro Leu Thr
530 535 540

Ile Leu Gly Ser Thr Gly Pro Val Leu Val Phe Glu Lys Ile Leu Tyr
545 550 555 560

Lys Phe Cys Arg Asp Tyr Gln Leu Ser Tyr Leu Ser Leu Arg Thr Ser
565 570 575

Ile Gly Leu Trp Thr Ser Phe Leu Cys Ile Val Leu Val Ala Thr Asp
580 585 590

Ala Ser Ser Leu Val Cys Tyr Ile Thr Arg Phe Thr Glu Glu Ala Phe
595 600 605

Ala Ala Leu Ile Cys Ile Ile Phe Ile Tyr Glu Ala Leu Glu Lys Leu
610 615 620

Phe Asp Leu Gly Glu Thr Tyr Ala Phe Asn Met His Asn Asn Leu Asp
625 630 635 640

Lys Leu Thr Ser Tyr Ser Cys Val Cys Thr Glu Pro Pro Asn Pro Ser
645 650 655

Asn Glu Thr Leu Ala Gln Trp Lys Lys Asp Asn Ile Thr Ala His Asn
660 665 670

Ile Ser Trp Arg Asn Leu Thr Val Ser Glu Cys Lys Lys Leu Arg Gly
675 680 685

Val Phe Leu Gly Ser Ala Cys Gly His His Gly Pro Tyr Ile Pro Asp
690 695 700

Val Leu Phe Trp Cys Val Ile Leu Phe Phe Thr Thr Phe Phe Leu Ser
705 710 715 720

Ser Phe Leu Lys Gln Phe Lys Thr Lys Arg Tyr Phe Pro Thr Lys Val
725 730 735

Arg Ser Thr Ile Ser Asp Phe Ala Val Phe Leu Thr Ile Val Ile Met
740 745 750

Val Thr Ile Asp Tyr Leu Val Gly Val Pro Ser Pro Lys Leu His Val
755 760 765

Pro Glu Lys Phe Glu Pro Thr His Pro Glu Arg Gly Trp Ile Ile Ser
770 775 780

Pro Leu Gly Asp Asn Pro Trp Trp Thr Leu Leu Ile Ala Ala Ile Pro
785 790 795 800

Ala Leu Leu Cys Thr Ile Leu Ile Phe Met Asp Gln Gln Ile Thr Ala
805 810 815

Val Ile Ile Asn Arg Lys Glu His Lys Leu Lys Lys Gly Ala Gly Tyr
820 825 830

His Leu Asp Leu Leu Met Val Gly Val Met Leu Gly Val Cys Ser Val
835 840 845

Met Gly Leu Pro Trp Phe Val Ala Ala Thr Val Leu Ser Ile Ser His
850 855 860

Val Asn Ser Leu Lys Val Glu Ser Glu Cys Ser Ala Pro Gly Glu Gln
865 870 875 880

Pro Lys Phe Leu Gly Ile Arg Glu Gln Arg Val Thr Gly Leu Met Ile
885 890 895

Phe Ile Leu Met Gly Leu Ser Val Phe Met Thr Ser Val Leu Lys Phe
900 905 910

Ile Pro Met Pro Val Leu Tyr Gly Val Phe Leu Tyr Met Gly Val Ser
915 920 925

Ser Leu Lys Gly Ile Gln Leu Phe Asp Arg Ile Lys Leu Phe Gly Met
930 935 940

Pro Ala Lys His Gln Pro Asp Leu Ile Tyr Leu Arg Tyr Val Pro Leu
945 950 955 960

Trp Lys Val His Ile Phe Thr Val Ile Gln Leu Thr Cys Leu Val Leu
965 970 975

Leu Trp Val Ile Lys Val Ser Ala Ala Ala Val Val Phe Pro Met Met
980 985 990

Val Leu Ala Leu Val Phe Val Arg Lys Leu Met Asp Leu Cys Phe Thr
995 1000 1005

Lys Arg Glu Leu Ser Trp Leu Asp Asp Leu Met Pro Glu Ser Lys
1010 1015 1020

Lys Lys Lys Glu Asp Asp Lys Lys Lys Lys Glu Lys Glu Glu Ala
1025 1030 1035

Glu Arg Met Leu Gln Asp Asp Asp Asp Thr Val His Leu Pro Phe
1040 1045 1050

Glu Gly Gly Ser Leu Leu Gln Ile Pro Val Lys Ala Leu Lys Tyr
1055 1060 1065

Ser Val Asp Pro Ser Ile Val Asn Ile Ser Asp Glu Met Ala Lys

```
    1070                1075                1080

Thr Ala  Gln Trp Lys Ala Leu  Ser Met Asn Thr Glu  Asn Ala Lys
    1085                1090                1095

Val Thr  Arg Ser Asn Met Ser  Pro Asp Lys Pro Val  Ser Val Lys
    1100                1105                1110

Ile Ser  Phe Glu Asp Glu Pro  Arg Lys Lys Tyr Val  Asp Ala Glu
    1115                1120                1125

Thr Ser  Leu
    1130
```

<210> 5
<211> 30
<212> PRT
<213> human

<400> 5

```
Ser Pro Val Ile Thr Phe Gly Gly Leu Leu Gly Glu Ala Thr Glu Gly
1               5                   10                  15

Arg Ile Ser Ala Ile Glu Ser Leu Phe Gly Ala Ser Leu Thr
            20                  25                  30
```

<210> 6
<211> 78
<212> PRT
<213> human

<400> 6

```
Lys Leu Phe Asp Leu Gly Glu Thr Tyr Ala Phe Asn Met His Asn Asn
1               5                   10                  15

Leu Asp Lys Leu Thr Ser Tyr Ser Cys Val Cys Thr Glu Pro Pro Asn
            20                  25                  30

Pro Ser Asn Glu Thr Leu Ala Gln Trp Lys Lys Asp Asn Ile Thr Ala
        35                  40                  45

His Asn Ile Ser Trp Arg Asn Leu Thr Val Ser Glu Cys Lys Lys Leu
    50                  55                  60

Arg Gly Val Phe Leu Gly Ser Ala Cys Gly His His Gly Pro
65                  70                  75
```

<210> 7

<211> 78
<212> PRT
<213> mouse

<400> 7

```
Lys Leu Phe His Leu Gly Glu Ile Tyr Ala Phe Asn Met His Asn Asn
1               5                   10                  15

Leu Asp Glu Leu Thr Ser Tyr Thr Cys Val Cys Ala Glu Pro Ser Asn
            20                  25                  30

Pro Ser Asn Glu Thr Leu Glu Leu Trp Lys Arg Lys Asn Ile Thr Ala
        35                  40                  45

Tyr Ser Val Ser Trp Gly Asn Leu Thr Val Ser Glu Cys Lys Thr Phe
    50                  55                  60

His Gly Met Phe Val Gly Ser Ala Cys Gly Pro His Gly Pro
65                  70                  75
```

<210> 8
<211> 30
<212> PRT
<213> human

<400> 8

```
Pro Ser Pro Lys Leu His Val Pro Glu Lys Phe Glu Pro Thr His Pro
1               5                   10                  15

Glu Arg Gly Trp Ile Ile Ser Pro Leu Gly Asp Asn Pro Trp
            20                  25                  30
```

<210> 9
<211> 30
<212> PRT
<213> mouse

<400> 9

```
Pro Ser Pro Lys Leu His Val Pro Glu Lys Phe Glu Pro Thr Asp Pro
1               5                   10                  15

Ser Arg Gly Trp Ile Ile Ser Pro Leu Gly Asp Asn Pro Trp
            20                  25                  30
```

<210> 10
<211> 30
<212> PRT
<213> human

<400> 10

Ser Ile Ser His Val Asn Ser Leu Lys Val Glu Ser Glu Cys Ser Ala
1               5                   10                  15

Pro Gly Glu Gln Pro Lys Phe Leu Gly Ile Arg Glu Gln Arg
            20                  25                  30

<210> 11
<211> 608
<212> PRT
<213> human

<400> 11

```
Met Glu Arg Phe Arg Leu Glu Lys Lys Leu Pro Gly Pro Asp Glu Glu
1               5                   10                  15

Ala Val Val Asp Leu Gly Lys Thr Ser Ser Thr Val Asn Thr Lys Phe
            20                  25                  30

Glu Lys Glu Glu Leu Glu Ser His Arg Ala Val Tyr Ile Gly Val His
        35                  40                  45

Val Pro Phe Ser Lys Glu Ser Arg Arg Arg His Arg His Arg Gly His
    50                  55                  60

Lys His His His Arg Arg Arg Lys Asp Lys Glu Ser Asp Lys Glu Asp
65                  70                  75                  80

Gly Arg Glu Ser Pro Ser Tyr Asp Thr Pro Ser Gln Arg Val Gln Phe
                85                  90                  95

Ile Leu Gly Thr Glu Asp Asp Asp Glu Glu His Ile Pro His Asp Leu
            100                 105                 110

Phe Thr Glu Met Asp Glu Leu Cys Tyr Arg Asp Gly Glu Glu Tyr Glu
        115                 120                 125

Trp Lys Glu Thr Ala Arg Trp Leu Lys Phe Glu Glu Asp Val Glu Asp
    130                 135                 140

Gly Gly Asp Arg Trp Ser Lys Pro Tyr Val Ala Thr Leu Ser Leu His
145                 150                 155                 160

Ser Leu Phe Glu Leu Arg Ser Cys Ile Leu Asn Gly Thr Val Met Leu
                165                 170                 175

Asp Met Arg Ala Ser Thr Leu Asp Glu Ile Ala Asp Met Val Leu Asp
            180                 185                 190

Asn Met Ile Ala Ser Gly Gln Leu Asp Glu Ser Ile Arg Glu Asn Val
```

```
            195                 200                 205

Arg Glu Ala Leu Leu Lys Arg His His His Gln Asn Glu Lys Arg Phe
    210                 215                 220

Thr Ser Arg Ile Pro Leu Val Arg Ser Phe Ala Asp Ile Gly Lys Lys
225                 230                 235                 240

His Ser Asp Pro His Leu Leu Glu Arg Asn Gly Glu Gly Leu Ser Ala
                245                 250                 255

Ser Arg His Ser Leu Arg Thr Gly Leu Ser Ala Ser Asn Leu Ser Leu
            260                 265                 270

Arg Gly Glu Ser Pro Leu Ser Leu Leu Leu Gly His Leu Leu Pro Ser
        275                 280                 285

Ser Arg Ala Gly Thr Pro Ala Gly Ser Arg Cys Thr Thr Pro Val Pro
    290                 295                 300

Thr Pro Gln Asn Ser Pro Pro Ser Ser Pro Ser Ile Ser Arg Leu Thr
305                 310                 315                 320

Ser Arg Ser Ser Gln Glu Ser Gln Arg Gln Ala Pro Glu Leu Leu Val
                325                 330                 335

Ser Pro Ala Ser Asp Asp Ile Pro Thr Val Val Ile His Pro Pro Glu
            340                 345                 350

Glu Asp Leu Glu Ala Ala Leu Lys Gly Glu Glu Gln Lys Asn Glu Glu
        355                 360                 365

Asn Val Asp Leu Thr Pro Gly Ile Leu Ala Ser Pro Gln Ser Ala Pro
    370                 375                 380

Gly Asn Leu Asp Asn Ser Lys Ser Gly Glu Ile Lys Gly Asn Gly Ser
385                 390                 395                 400

Gly Gly Ser Arg Glu Asn Ser Thr Val Asp Phe Ser Lys Val Asp Met
                405                 410                 415

Asn Phe Met Arg Lys Ile Pro Thr Gly Ala Glu Ala Ser Asn Val Leu
            420                 425                 430

Val Gly Glu Val Asp Phe Leu Glu Arg Pro Ile Ile Ala Phe Val Arg
        435                 440                 445
```

```
Leu Ala Pro Ala Val Leu Leu Thr Gly Leu Thr Glu Val Pro Val Pro
    450                 455                 460

Thr Arg Phe Leu Phe Leu Leu Leu Gly Pro Ala Gly Lys Ala Pro Gln
465                 470                 475                 480

Tyr His Glu Ile Gly Arg Ser Ile Ala Thr Leu Met Thr Asp Glu Ile
                485                 490                 495

Phe His Asp Val Ala Tyr Lys Ala Lys Asp Arg Asn Asp Leu Leu Ser
            500                 505                 510

Gly Ile Asp Glu Phe Leu Asp Gln Val Thr Val Leu Pro Pro Gly Glu
        515                 520                 525

Trp Asp Pro Ser Ile Arg Ile Glu Pro Pro Lys Ser Val Pro Ser Gln
    530                 535                 540

Glu Lys Arg Lys Ile Pro Val Phe His Asn Gly Ser Thr Pro Thr Leu
545                 550                 555                 560

Gly Glu Thr Pro Lys Glu Ala Ala His His Ala Gly Pro Glu Leu Gln
                565                 570                 575

Arg Thr Gly Arg Leu Phe Gly Gly Leu Ile Leu Asp Ile Lys Arg Lys
            580                 585                 590

Ala Pro Phe Phe Leu Ser Asp Phe Lys Asp Ala Leu Ser Leu Gln Cys
        595                 600                 605
```

<210> 12
<211> 36
<212> PRT
<213> human

<400> 12

```
Ala Thr Val Leu Ser Ile Ser His Val Asn Ser Leu Lys Val Glu Ser
1               5                   10                  15

Glu Cys Ser Ala Pro Gly Glu Gln Pro Lys Phe Leu Gly Ile Arg Glu
            20                  25                  30

Gln Arg Val Thr
        35
```

<210> 13
<211> 37
<212> PRT

<213> human

<400> 13

Asp Arg Ile Lys Leu Phe Gly Met Pro Ala Lys His Gln Pro Asp Leu
1 5 10 15

Ile Tyr Leu Arg Tyr Val Pro Leu Trp Lys Val His Ile Phe Thr Val
20 25 30

Ile Gln Leu Thr Cys
35

<210> 14
<211> 35
<212> PRT
<213> human

<400> 14

His Asn Asn Leu Asp Lys Leu Thr Ser Tyr Ser Cys Val Cys Thr Glu
1 5 10 15

Pro Pro Asn Pro Ser Asn Glu Thr Leu Ala Gln Trp Lys Lys Asp Asn
20 25 30

Ile Thr Ala
35

<210> 15
<211> 35
<212> PRT
<213> human

<400> 15

Leu Ala Gln Trp Lys Lys Asp Asn Ile Thr Ala His Asn Ile Ser Trp
1 5 10 15

Arg Asn Leu Thr Val Ser Glu Cys Lys Lys Leu Arg Gly Val Phe Leu
20 25 30

Gly Ser Ala
35

## Description

### Technical field

The present disclosure relates to the use of NBCn1 (SLC4A7) as a target for treatment of breast cancer, atherosclerosis and/or restenosis.

### Background

The lifetime risk of developing breast cancer is more than 1 in 8 for women in the Western world and globally more than 500,000 women die of breast cancer each year. Like most solid cancers, breast carcinomas are characterized by high metabolic rates and prominent glycolytic activities. Metabolic production of $CO_2$ and lactic acid represent substantial sources of intracellular acid loading that in breast cancer cells are compensated by upregulated cellular net acid extrusion. High rates of net transmembrane acid efflux in breast carcinomas maintain intracellular pH ($pH_i$) equal to or higher than in normal breast tissue. In combination with an insufficient blood supply, the upregulated cellular acid extrusion also creates an acidic and hypoxic extracellular tumor microenvironment. This characteristic compartmentalization of acid-base equivalents has been proposed to promote cancer progression; and as a consequence, cancer metabolism and mechanisms of acidic waste product elimination could be relevant targets for anti-cancer treatment.

The high proliferative activity and growth rate of cancer tissue are energy demanding, and inhibition of glycolytic activity is a possible therapeutic approach. Phosphofructokinase 1 (PFK1) is the rate limiting step of glycolysis and has prominent pH sensitivity *in vitro. In vivo,* $pH_i$ may also regulate glycolytic activity. Thus, interfering with acid-base regulation - to lower $pH_i$ selectively in cancer cells - may potentially inhibit glycolytic energy production in cancer tissue without major effects on normal tissue.

Targeting $Na^+/H^+$-exchangers (Loo SY, Chang MK, Chua CS, Kumar AP, Pervaiz S, Clement MV. NHE-1: a promising target for novel anti-cancer therapeutics. Curr. Pharm. Des 2012;18:1372-1382), monocarboxylate transporters (Chiche J et al. In vivo pH in metabolic-defective Ras-transformed fibroblast tumors: key role of the monocarboxylate transporter, MCT4, for inducing an alkaline intracellular pH. Int. J. Cancer 2012;130:1511-1520) or $H^+$-pumps (De Milito A, Marino ML, Fais S. A rationale for the use of proton pump inhibitors as antineoplastic agents. Curr. Pharm. Des 2012;18:1395-1406), interfering with acid-base buffering by $NaHCO_3$ supplementation (Silva AS, Yunes JA, Gillies RJ, Gatenby RA. The potential role of systemic buffers in reducing intratumoral extracellular pH and acid-mediated invasion. Cancer Res. 2009;69:2677-2684) or inhibiting carbonic anhydrases (Robertson N, Potter C, Harris AL. Role of carbonic anhydrase IX in human tumor cell growth, survival, and invasion. Cancer Res. 2004;64:6160-6165; Swietach P, Patiar S, Supuran CT, Harris AL, Vaughan-Jones RD. The role of carbonic anhydrase 9 in regulating extracellular and intracellular ph in three-dimensional tumor cell growths. J. Biol. Chem. 2009;284:20299-20310) have been suggested as possible treatment strategies for different types of cancer. For human breast cancer, $Na^+,HCO_3^-$-cotransport is involved in acid extrusion and is also functionally upregulated during breast carcinogenesis (Lee S, Mele M, Vahl P, Christiansen PM, Jensen VED, Boedtkjer E. $Na^+,HCO_3^-$-cotransport is functionally upregulated during human breast carcinogenesis and required for the inverted pH gradient across the plasma membrane. Pflugers Arch. 2014; Boedtkjer E et al. Contribution of Na+,HCO3--cotransport to cellular pH control in human breast cancer: a role for the breast cancer susceptibility locus NBCn1 (SLC4A7). Int. J. Cancer 2013;132:1288-1299). However, nothing is known about the consequences of inhibiting $Na^+,HCO_3^-$-cotransport for development and progression of breast cancer or for atherosclerosis or restenosis.

Known and putative $Na^+$-dependent $HCO_3^-$-transporters are gathered in the SLC4 family, which comprise 7 members. There are no suitable selective pharmacological inhibitors of $Na^+,HCO_3^-$-cotransporters. Thus, there is a need for identifying selective inhibitors of $Na^+,HCO_3^-$-cotransporters and evaluation of their potential as anticancer agents.

A paper by Damkier et al. (Damkier, H.H., Nielsen, S. and Praetorius, J. 2006. An anti-NH2-terminal antibody localizes NBCn1 to heart endothelia and skeletal and vascular smooth muscle cells. Am J Physiol Heart Circ Physiol, 290, H172-80) discloses the development of the $NH_2$-terminal antibody allowed the localization of NBCn1 protein to major cardiovascular tissue where NBCn1 mRNA was previously detected.

### Summary

The disclosure relates to the use of the $Na^+,HCO_3^-$-cotransporter NBCn1 (SLC4A7) as a target for breast cancer, atherosclerosis and/or restenosis treatment. The inventor has found in a mouse breast cancer model that knockout of NBCn1 leads to reduced tumor growth and increased survival. NBCn1 is involved in intracellular pH control and the inventor has found that the intracellular pH in breast tumors of NBCn1 knockout mice is lower compared to wild type mice. These observations support a role of NBCn1 in maintaining a steady state intracellular pH in breast cancer tissue, which thereby allows a high metabolic activity even in hypoxic tumors.

In particular, the disclosure relates to antibody-based therapy for breast cancer, where the antibodies specifically target extracellular domains of NBCn1. The present disclosure provides novel antibodies with specificity against specific extracellular regions of the NBCn1 transmembrane protein.

**Description of Drawings**

Figure 1. Disrupting NBCn1 expression delays breast cancer development and tumor growth rate following treatment with MPA and DMBA. A. Breast tumor-free survival of NBCn1 KO and WT mice. Day 0 corresponds to the day of the last DMBA treatment by gavage. The Kaplan-Meier curves were compared by a Gehan-Breslow-Wilcoxon test. B. Representative images of breast tumors from WT and NBCn1 KO mice and average tumor sizes (n=13-16) measured 14 days after the tumors were first detectable. Tumors were typically 3-4 mm at first detection consistent with previous studies. Due to the delayed breast tumor development in the NBCn1 KO mice, they were on average 41 days older than the WT mice on the day of tumor size determination. Log-transformed data for tumor latency and tumor burden were compared between genotypes by unpaired two-tailed Student's *t*-test. *$P<0.05$, **$P<0.01$ *vs.* WT.

Figure 2. Histopathology of chemically induced breast cancers differs between WT and NBCn1 KO mice. A. The distribution between histopathological breast cancer subtypes in WT and NBCn1 KO mice (n=13-16). B-F. Hematoxylin and eosin stained histological images representative of the identified breast cancer subtypes: adenosquamous carcinoma (B), squamous carcinoma (C), Wnt type tumor (D), adenomyoepithelioma (E) and adenocarcinoma (F). G. Representative images of hematoxylin and eosin stained (upper panel) and anti-CD45 labeled (lower panel) lymphomas. H. The left panel displays matched values for tumor burden and tumor latency for the individual histopathological breast cancer subtypes. The right panel shows aggressiveness score' defined as the ratio between tumor burden and tumor latency for each breast tumor subtype. The aggressiveness score was significantly lower ($P<0.05$, two-way ANOVA) for tumors of NBCn1 KO than WT mice. The scale bar in B represents 200 $\mu$m; all images are shown at the same magnification.

Figure 3. NBCn1 is responsible for net acid extrusion from breast cancer and for the alkaline shift in $pH_i$ occurring during breast carcinogenesis. A. Epithelial organoids freshly isolated from breast cancer and matched normal breast tissue. Similar images were obtained from NBCn1 KO and WT mice (not shown). The left panels show cytokeratin 19 (CK-19) immunofluorescence images, center panels show negative control images without primary antibody (no 1° AB), and the right panels show images of BCECF-loaded organoids. B. Average traces of the $pH_i$ dynamics during $NH_4^+$ prepulse experiments (n=7). For simplicity, standard errors are shown for a few points only. Recordings were collected at 0.2 Hz. C. Buffering capacities calculated from the change in $pH_i$ upon washout of $NH_4Cl$ (n=10-11). D+E. $Na^+$-dependent cellular net acid extrusion (or equivalently, base uptake) in epithelial organoids from WT (D) and NBCn1 KO (E) mice as functions of $pH_i$. Experiments were performed on organoids isolated from breast cancer or matched normal breast tissue (n=7-9) in the presence and absence of $CO_2/HCO_3^-$. F. Steady-state $pH_i$ of epithelial organoids isolated from breast cancer and matched normal breast tissue from NBCn1 KO and WT mice (n=7-9). Comparisons were performed by least-squares linear regression analyses (panel D+E) or by two-tailed unpaired Student's *t*-test (panel F). *$P<0.05$ vs. normal breast tissue under similar conditions or as specified.

Figure 4. Protein expression of NBCn1, MCT1 and MCT4-but not of NHE1-is upregulated during breast carcinogenesis. A-D. Representative immunoblots and average protein expression levels of NBCn1 (A), NHE1 (B), MCT1 (C) and MCT4 (D) in organoids isolated from breast cancer and matched normal breast tissue from NBCn1 KO and WT mice (n=8-10). In the right hand panels, the tumor values are re-plotted as functions of tumor volume. β-actin and dynactin subunit $p150^{Glued}$ were used as loading controls. The representative immunoblots are from the same gels and illustrate samples of matched breast cancer and normal breast tissue. Expression levels are expressed relative to normal breast tissue from WT mice. Log-transformed data were compared by repeated measures two-way ANOVA followed by Bonferroni post-tests (left panels) or by least-squares liner regression analyses (right panels). If the fitted lines were not significantly different between WT and NBCn1 KO mice, only the combined line is shown. *$P<0.05$, **$P<0.01$, ***$P<0.001$, NS: not significantly different vs. organoids of similar source (normal breast or breast cancer) from WT mice or between organoids from normal and cancer tissue as indicated.

Figure 5. *In vivo,* glycolytic metabolism is inhibited in breast cancer of NBCn1 KO compared to WT mice. A+B. Interstitial concentrations of glucose (A, n=11-12) and lactate (B, n=12) measured in microdialysis samples obtained with probes placed in breast cancer and matched normal breast tissue of NBCn1 KO and WT mice. C. [lactate]/[glucose] ratios in breast cancer and normal breast tissue (n=11-12; left panel). In the right hand panel, the cancer values are re-plotted as a function of tumor volume (right panel). D+E. Interstitial concentrations of pyruvate (D, n=6-13) and glycerol (E, n=12) measured in microdialysis samples obtained with probes placed in breast cancer and matched normal breast tissue of NBCn1 KO and WT mice. Log-transformed data were compared by repeated measures two-way ANOVA followed by Bonferroni post-tests or by least-squares linear regression analysis (right part of panel C). Because the fitted lines were not significantly different between WT and NBCn1 KO mice, only the combined line is shown. *$P$<0.05, **$P$<0.01, NS: not significantly different vs. WT under similar conditions or between organoids from normal and cancer tissue as indicated.

Figure 6. Protein expression levels and phosphorylation states differ in breast cancer from NBCn1 KO and WT mice. A-E. Representative immunoblots and average protein expression levels of phosphorylated ErbB2 (A), cleaved PARP-1 (B), phosphorylated AMPK (C), phosphorylated Akt (D) and phosphorylated ERK1/2 (E) in organoids isolated from breast cancer and matched normal breast tissue from NBCn1 KO and WT mice (n=8-10). For each protein, the right hand panel shows the tumor values re-plotted as a function of tumor volume. $\beta$-actin was used as loading control. The representative immunoblots are from the same gels and illustrate samples of matched breast cancer and normal breast tissue. Expression levels are expressed relative to normal breast tissue from WT mice. Log-transformed data were compared by repeated measures two-way ANOVA followed by Bonferroni post-tests (left panels) or by least-squares linear regression analyses (right panels). Because the fitted lines were not significantly different between WT and NBCn1 KO mice, only the combined lines are shown. *$P$<0.05, **$P$<0.01, NS: not significantly different *vs.* WT under similar conditions or between organoids from normal and cancer tissue as indicated.

Figure 7. Cell proliferation is reduced in breast cancer from NBCn1 KO compared to WT mice. A+B. Representative immunohistochemical staining for Ki-67 (A) and $pHH_3$ (B) in sections of breast cancer tissue from NBCn1 KO and WT mice. Examples of positively stained cells are indicated by arrowheads. C+D. Fraction of epithelial cells positive for Ki-67 (C) or $pHH_3$ (D) in sections of cancer tissue and normal breast tissue from NBCn1 KO and WT mice (n=12-13). From each of the cancer samples, we evaluated 3174$\pm$1147 (mean$\pm$SD) cells for Ki-67 and 5043$\pm$1609 cells for $pHH_3$ staining. The data were compared by two-way ANOVA followed by Bonferroni post-tests. E. The cancer values from panel C and D were re-plotted as functions of tumor volume. The shaded area highlights the tumors within the interval between the smallest tumor from WT mice and the largest tumor from NBCn1 KO mice. Following normalization to the average cancer value, the matched data for Ki-67 and $pHH_3$ within the shaded area were compared between WT and NBCn1 KO by repeated measures two-way ANOVA. **$P$<0.01, ***$P$<0.001, NS: not significantly different vs. WT under similar conditions, between organoids from normal and cancer tissue, or as indicated. The scale bar in A represents 50 $\mu$m; all images are shown at the same magnification.

Figure 8. NBCn1 mediates the $Na^+$,$HCO_3$ -cotransport in VSMCs of mouse carotid arteries and is important for steady-state $pH_i$ regulation. **A+B.** Average $pH_i$ traces from $NH_4^{+}$-prepulse experiments showing the response of carotid arteries from NBCn1 KO and WT mice (n=3-5) investigated in the presence (A) or absence (B) of $CO_2/HCO_3^-$. SEM is shown for a few points only to improve readability; measurements were performed at 0.2 Hz. **C.** Rates of $pH_i$ recovery in VSMCs of carotid arteries from NBCn1 KO and WT mice after $NH_4^+$-prepulses in presence or absence of $CO_2/HCO_3$-(n=3-5). **D.** Resting steady-state $pH_i$ in carotid arteries from NBCn1 KO and WT mice (n=3-9) in presence and absence of $CO_2/HCO_3^-$. Data were compared by two-way ANOVA followed by Bonferroni post-tests. *$P$<0.05, NS: Not significantly different.

Figure 9. NBCn1 KO inhibits remodeling following carotid artery ligation. **A.** Representative images of non-ligated, partially ligated, and completely ligated common carotid arteries four weeks after surgery. Arrows indicate the outer boundaries of the lumen (green), media (red) and adventitia (black). The asterisks indicate intraluminal blood clots. The scale bar represents 100 $\mu$m, all images are shown at the same magnification. **B-D.** Relative differences in internal diameter (B), media thickness (C) and media area (D) between ligated and contralateral, non-ligated common carotid arteries. Effects of partial ligation (n=12-13), complete ligation (n=12-14) and sham operation (n=5-6) are shown. For the decrease in lumen diameter, the effect of partial ligation was greater than the effect of complete ligation (P<0.05, two-way ANOVA). The increase in media thickness ($P$=0.88) and area ($P$=0.36) did not differ significantly between the complete and partial ligation models (two-way ANOVA). **E.** Cross-sectional areas of adherent adventitia in ligated common carotid arteries (n=9-13). The amount of adherent adventitia was significantly larger after partial than complete carotid artery ligation ($P$<0.05, two-way ANOVA) but did not differ between NBCn1 KO and WT mice ($P$=0.50). Non-ligated common carotid arteries had negligible amounts of adherent adventitia (see panel A). Comparisons were performed by two-way ANOVA and the overall significance level reported. *$P$<0.05, NS: Not significantly different.

Figure 10. $Na^+$,$HCO_3^-$-cotransport via NBCn1 is important for $pH_i$ regulation in primary aortic explants. **A.** Rates of

$pH_i$ recovery in primary explants investigated in presence of $CO_2/HCO_3^-$ and 1 mM amiloride (n=8). $Na^+$-dependent $pH_i$ recovery rates were calculated at average $pH_i$ levels of 6.54±0.12 (WT) and 6.53±0.13 (NBCn1 KO). **B.** Rates of $pH_i$ recovery in primary explants investigated in absence of $CO_2/HCO_3^-$ (n=7-12). $Na^+$-dependent $pH_i$ recovery rates were calculated at average $pH_i$ levels of 6.59±0.03 (WT) and 6.59±0.06 (NBCn1 KO). **C.** Steady-state $pH_i$ in primary explants (n=8). **D.** Rates of $pH_i$ recovery in primary explants from WT mice in presence of $CO_2/HCO_3^-$ with or without S0859 (n=4-5). $Na^+$-dependent $pH_i$ recovery rates were calculated at average $pH_i$ levels of 6.19±0.08 (control), 6.10±0.02 (5 μM S0859), and 6.10±0.03 (30 μM S0859). **E.** Effects of S0859 on steady-state $pH_i$ (n=6). Curves show mean values obtained five minutes after addition of S0859. Asterisks indicate significant difference vs. WT (panel A and B), vs. control conditions (panel D), *vs.* baseline and washout conditions (panel E), or as indicated (panel C). Comparisons were performed by paired (panel C) or unpaired (panel A,B) two-tailed Student's *t*-test, or one-way ANOVA followed by Bonferroni post-tests (panel D,E). **P*<0.05, ***P*<0.01, NS: Not significantly different.

Figure 11. Inhibition of $Na^+,HCO_3^-$-cotransport attenuates wound healing in scratch assays. **A.** Representative images showing the extent of wound healing over a 3-hour period in primary aortic explants from WT and NBCn1 KO mice. Red arrows indicate the migrating front. Original series of time-lapse images are included in the online supplement. The scale bar represents 200 μm, all images are shown at the same magnification. **B-G.** Progression of wound healing after scratch infliction through primary explants (n=5-11). Panels B and D-G report data from aortic explants, whereas panel C reports data from carotid artery explants. Genotype and experimental conditions are given in the individual panels. PSS was used to avoid binding of S0859 to medium and serum components. Comparisons were performed by repeated measures two-way ANOVA. ****P*<0.001, NS: Not significantly different.

Figure 12. Global $pH_i$ modulates VSMC proliferation *in vitro.* **A+B.** Fraction of cells from primary aortic (A) and carotid (B) explants undergoing mitosis in serum-containing medium or PSS during 6-hour time-lapse recordings (n=5-12). The relative number of cell divisions was significantly lower in the absence (shaded blue) than presence of $CO_2/HCO_3^-$ (*P*<0.01, two-way ANOVA). The image sequence illustrates a cell division. The scale bar represents 20 μm, all images are shown at the same magnification. **C.** Fraction of BrdU-positive cells in primary explants incubated for six hours with the thymidine-analogue in serum-containing medium (n=7). Representative images are shown. The scale bar represents 200 μm, all images are shown at the same magnification. Red represents anti-BrdU fluorescence; green represents SYTO® 16 nuclear fluorescence. **D.** Media hypertrophy (i.e., difference in media area between ligated and contralateral, non-ligated carotid arteries; left ordinate) and VSMC proliferation (i.e., fraction of dividing cells in primary aortic explants; right ordinate) as function of global $pH_i$ measured in carotid arteries and primary aortic explants, respectively, under the given conditions. Open symbols indicate experiments performed in PSS rather than serum-containing culture medium but otherwise under conditions corresponding to the filled symbols. **E.** Projected cell areas in primary aortic explants from WT and NBCn1 KO mice (n=16-19) investigated in serum-supplemented medium. **F.** Images of primary explants from WT and NBCn1 KO mice 18 hours after scratch infliction. Images are representative of 10-11 experiments performed in serum-containing medium with $CO_2/HCO_3^-$. The scale bar represents 200 μm, both images are shown at the same magnification. Numbers in individual panels represent the relative number of cells with normal cell morphology after 18 hours of imaging compared to immediately after scratch infliction. **G+H.** Images of primary explants under control conditions or during exposure to S0859. The scale bars represent 200 μm, all images are shown at same magnification. Experiments were performed in PSS with (G) or without (H) $CO_2/HCO_3^-$ to avoid binding of S0859 to medium and serum components. The images are representative of 5-10 experiments. Numbers in individual panels represent the relative number of cells with normal cell morphology after 18 hours of imaging compared to immediately after scratch infliction; the gradual decay in proportion of viable cells in presence of 30 μM S0859 is displayed in Supplemental Figure 2. Data in panels A (medium + serum), B, C, F and H were compared by unpaired two-tailed Student's *t*-test, data in panel A (PSS) and G by one-way ANOVA, and data in panel D by linear regression analysis. ****P*<0.001, NS: Not significantly different vs. WT under similar conditions (without S0859).

Figure 13. NBCn1 KO inhibits $pH_i$ gradients and filopodia in migrating VSMCs. **A.** Relative lengths of cellular protrusions at the migrating front of primary aortic explants quantified two hours after scratch infliction. Representative phase contrast images are shown for experiments performed in presence and absence of $CO_2/HCO_3^-$ as indicated. The scale bar represents 100 μm, all images are shown at the same magnification. The experiments were performed in medium containing serum (n=6-10). **B.** Relative lengths of cellular protrusions at the migrating front of primary carotid explants quantified three hours after scratch infliction. The experiments were performed in medium containing serum (n=8-11). **C.** Relative lengths of cellular protrusions at the migrating front of primary aortic explants quantified two hours after scratch infliction. Experiments were performed in PSS (n=5-11) to avoid binding of S0859 to medium and serum components. **D.** Fluorescence images of migrating VSMCs recorded two hours after scratch infliction through primary aortic explants from WT and NBCn1 KO mice. From the time of scratch infliction until image acquisition, explants were incubated in presence or absence of $CO_2/HCO_3^-$ as specified. The scale bar represents 50 $\mu$m, all images are shown at the same magnification. **E.** Fluorescence image of filopodium and original traces of $pH_i$ along filopodia in explants from WT and NBCn1 KO mice. **F.** Average $pH_i$ levels at the tip of filopodia (upper horizontal line) and in the perinuclear cytosolic region (lower horizontal line); n=4-10, each represented by the average of ~5 filopodia. The height of the bars represents the magnitude of the axial $pH_i$ gradient. Acetazolamide (ATZ, 100 $\mu$M) was applied to inhibit carbonic anhydrase activity. For the first two hours after scratch infliction, the primary aortic explants were incubated in absence of $CO_2/HCO_3^-$ to allow for equivalent filopodia formation in explants from NBCn1 KO and WT mice. Then, explants were exposed to the specified condition (i.e., $CO_2/HCO_3^-$, $CO_2/HCO_3^-$-free, S0859, ATZ) for 15 minutes prior to initiation of $pH_i$ recordings. Data were compared using one-way ANOVA or two-tailed Student's *t*-tests (paired or unpaired, as appropriate). Bonferroni corrections were applied to adjust for multiple testing. In panel A, B and C: **P*<0.05, ***P*<0.01, ****P*<0.001, NS: Not significantly different vs. WT under similar conditions. In panel F: **P*<0.05 comparing $pH_i$ at tip of filopodia vs. WT with $CO_2/HCO_3^-$, ¤*P*<0.05 comparing the $pH_i$ gradient vs. WT with $CO_2/HCO_3^-$, ##*P*<0.01 comparing the $pH_i$ gradient vs. NBCn1 KO with $CO_2/HCO_3^-$, NS: not significantly different vs. WT without $CO_2/HCO_3^-$.

Figure 14. Carbonic anhydrase inhibitor acetazolamide promotes wound healing and filopodia in explants from NBCn1 KO mice. **A.** Progression of wound healing after scratch infliction through primary aortic explants from NBCn1 KO mice with and without 100 $\mu$M acetazolamide (ATZ, n=9). Experiments were performed in $CO_2/HCO_3^-$-containing medium added serum. **B.** Original images showing the extent of wound healing over a 3-hour period in primary explants from NBCn1 KO mice in presence or absence of 100 $\mu$M acetazolamide. The scale bar represents 200 $\mu$m, all images are shown at the same magnification. Experiments were performed in $CO_2/HCO_3^-$-containing medium added serum. **C.** Average relative lengths of cellular protrusions at the migrating front quantified two hours after scratch infliction and original phase-contrast images illustrating the cellular morphology during wound healing in aortic explants from NBCn1 KO mice with or without 100 $\mu$M acetazolamide in presence of $CO_2/HCO_3^-$. The scale bar represents 100 $\mu$m, both images are shown at the same magnification. **D.** Summary of the association between the axial $pH_i$ gradient and the relative rate of wound healing. Open symbols indicate experiments performed in PSS rather than serum-containing medium but otherwise under conditions corresponding to the filled symbols. Data were compared by repeated measures two-way ANOVA (panel A), unpaired two-tailed Student's *t*-test (panel C) or linear regression analysis (panel D). **P*<0.05, ****P*<0.001 *vs.* NBCn1 KO without acetazolamide or as indicated.

Figure 15. Messenger RNA transcripts for known and putative $Na^+$,$HCO_3^-$-cotransporters of the Slc4-family are detected in arteries and primary explants from 129/SvJ WT and NBCn1 KO mice. Gel images (representative of three experiments on independent tissue preparations) showing the results of RT-PCR analyses. 'Positive control' represents reactions performed on tissue with known expression of the given transporter (NBCn1: kidney medulla, NBCe1: kidney cortex, NBCe2: choroid plexus, NDCBE: cerebral cortex, NCBE/NBCn2: choroid plexus, AE4: kidney cortex, BTR1: kidney medulla). For each sample, a parallel reaction without addition of reverse transcriptase produced no band (not shown) excluding amplification of genomic DNA. Bands of appropriate sizes were sequenced to confirm specificity. Despite detecting mRNA transcripts for multiple $Na^+$,$HCO_3^-$-cotransporters of the Slc4-family (NBCn1, NBCe1, NDCBE and BTR1) in arteries and VSMCs from 129/SvJ mice-which is in contrast to our previous findings from mesenteric, coronary and cerebral small arteries of NMRI mice where only NBCn1 was detected at mRNA level[4]-the $Na^+$,$HCO_3^-$-cotransport was completely abolished in carotid arteries from NBCn1 KO mice (see Figure 1A-C of the printed article). The finding that NBCn1 is responsible for the $Na^+$,$HCO_3$-cotransport in carotid arteries is consistent with our previous findings from murine resistance-sized mesenteric and middle cerebral arteries.

Figure 16. S0859 causes loss of cell viability in primary explants. Experiments were performed in $CO_2/HCO_3^-$-containing physiological saline solutions (PSS). Data were compared by repeated measures 2-way ANOVA. ****P*<0.001.

Figure 17. Predicted antigenic determinants in extracellular loop 3 of NBCn1. Based on the calculated antigenic index, we selected two overlapping peptides (NBCn1_EL3h1.1 and NBCn1_EL3h2.1) that together cover the regions of high estimated antigenicity.

Figure 18. Titers of serum samples collected from rabbits immunized with KLH-conjugated peptides corresponding to the predicted extracellular loop 3 of human NBCn1. **A+B.** Binding of sera from rabbits injected with KLH-conjugated NBCn1_EL3h1.1 (A) or NBCn1_EL3h2.1 (B) to the corresponding BSA-conjugated peptides evaluated by ELISA. The anti-BSA antibody was used as positive control and pre-immune serum served as negative control. Each measurement was made in duplicate from both of the rabbits injected with the respective KLH-conjugated peptide.

B1, B2, B3 and B4 refer to serum samples separated from blood drawn at week 7, 8, 10 and 11, respectively.

Figure 19. The polyclonal antibodies raised against extracellular loop 3 of NBCn1 completely inhibit $Na^+$,$HCO_3^-$-cotransport activity in MCF-7 human breast cancer cells. **A+B.** Representative $pH_i$ traces in response to $NH_4^+$-prepulses and the subsequent recovery with and without the raised antibodies (A) and 30 μM S0859 (B). To improve clarity, the $pH_i$ recovery phases (marked by the dotted gray squares) are displayed at twice the magnification of the initial parts of the traces and only the control curve is shown for the period prior to washout of $NH_4Cl$. The illustrated experiments were performed in the presence of $CO_2/HCO_3^-$. The antibodies were added (at 1:100 dilution) at the time of $NH_4Cl$ addition and S0859 was added at the time of $NH_4Cl$ washout and included in all subsequent solutions until the end of the experiment. **C+D.** Relative initial rates of $Na^+$-dependent $pH_i$ recovery after intracellular acid-loading with $NH_4^+$-prepulses. Equivalent inhibition of net acid extrusion by anti-NBCn1_EL3h2.1 (C, n=11-20) and 30 μM S0859 (D, n=5-6) in the presence of $CO_2/HCO_3^-$ supports that the developed antibody completely inhibits $Na^+$,$HCO_3^+$cotransport in these cells. This finding is further supported by the finding that anti-NBCn1_EL3h2.1 had no effect in the absence of $CO_2/HCO_3^-$ (C, n=6) and the finding from previous studies that siRNA-mediated down-regulation of NBCn1 inhibits net acid extrusion to the same extent as S0859. The remaining net acid-extrusion in the presence of S0859 has been found sensitive to the $Na^+/H^+$-exchange inhibitor 5-(*N*-ethyl-*N*-isopropyl)amiloride (Lauritzen et al., 2010). The data regarding S0859 in panels B and D are from (Steinkamp et al., 2015). Data were compared by one-way ANOVA followed by Dunnet's post-tests (experiments with $CO_2/HCO_3^-$ in panel C) or by unpaired, two-tailed Student's *t*-test (panel D and experiments without $CO_2/HCO_3^-$ in panel C). *$P$<0.05, ***$P$<0.001, NS: not significantly different vs. control under similar $CO_2/HCO_3^-$ conditions.

## Detailed description

The present disclosure relates to agents, which are capable of inhibiting the activity of the $Na^+$,$HCO_3^-$-cotransporter NBCn1 (SLC4A7). The present inventors have surprisingly found that inhibition of NBCn1 mediated $HCO_3^-$ transport is associated with reduced growth of breast tumors and increased survival. The disclosure, therefore, specifically relates to the use of such agent in medicine, in particular in the treatment of cancer, such as breast cancer. NBCn1 is also involved in migration of smooth muscle cells, which is important in restenosis and atherosclerosis; cf. example 2. Therefore, the disclosure also relates to the use of such agent in the treatment of atherosclerosis and/or restenosis. The agent of the disclosure may be any suitable agent capable of inhibiting NBCn1 activity and in a preferred aspect of the present disclosure, the agent is an antibody.

### Definitions

Antibodies: An antibody according to the disclosure is a polypeptide or protein capable of recognising and binding an antigen comprising at least one antigen binding site. Said antigen binding site preferably comprises at least one complementarity determining region (CDR). The antibody may be a naturally occurring antibody, a fragment of a naturally occurring antibody or a synthetic antibody.

Naturally occurring antibody: The term "naturally occurring antibody" refers to heterotetrameric glycoproteins capable of recognising and binding an antigen and comprising two identical heavy (H) chains and two identical light (L) chains inter-connected by disulfide bonds. Each heavy chain comprises a heavy chain variable region (abbreviated herein as $V_H$) and a heavy chain constant region (abbreviated herein as $C_H$). Each light chain comprises a light chain variable region (abbreviated herein as $V_L$) and a light chain constant region (abbreviated herein as $C_L$). The $V_H$ and $V_L$ regions can be further subdivided into regions of hypervariability, termed complementarity determining regions (CDRs), interspersed with regions that are more conserved, termed framework regions (FRs). Antibodies may comprise several identical heterotetramers.

Antigen: Molecule comprising at least one epitope. The antigen may for example be a polypeptide, polysaccharide, protein, lipoprotein or glycoprotein.

Epitope: An epitope is a determinant capable of specific binding to an antibody. Epitopes may for example be comprised within polypeptides, polysaccharide, proteins, lipoproteins or glycoproteins. Epitopes usually consist of chemically active surface groupings of molecules such as amino acids or sugar side chains and usually have specific three dimensional structural characteristics, as well as specific charge characteristics. Epitopes may be conformational or non-conformational, wherein binding to the former but not the latter is lost in the presence of denaturing solvents. Epitopes may be continuous or discontinuous, wherein a discontinuous epitope is a conformational epitope on a protein antigen which is formed from at least two separate regions in the primary sequence of the protein.

Monoclonal Antibody: Monoclonal antibodies (Mab's) are populations of antibodies, wherein every antibody molecule is similar and thus recognises and binds the same epitope. Monoclonal antibodies are in general produced by a host cell line and frequently by a hybridoma cell line. Methods of making monoclonal antibodies and antibody-synthesizing hybridoma cells are well known to those skilled in the art. Antibody producing hybridomas may for example be prepared by fusion of an antibody producing B lymphocyte with an immortalized B-lymphocyte cell line. Monoclonal antibodies according to the present disclosure may for example be prepared by the standard somatic cell hybridization technique of Kohler and Milstein, Nature 256:495 (1975) or as described in Antibodies: A Laboratory Manual, By Ed Harlow and David Lane, *Cold Spring Harbor Laboratory Press, 1988*. Said monoclonal antibodies may be derived from any suitable mammalian species, however frequently the monoclonal antibodies will be rodent antibodies for example murine or rat monoclonal antibodies. ¤It is preferred that the antibodies according to the present disclosure are monoclonal antibodies or derived from monoclonal antibodies.

Polyclonal antibodies: Polyclonal antibodies is a population of antibodies comprising a mixture of different antibody molecules recognising and binding a specific given antigen, hence polyclonal antibodies may recognise different epitopes within said antigen. In general polyclonal antibodies are purified from serum of an animal, preferably a mammal, which previously has been immunized with the antigen. Polyclonal antibodies may for example be prepared by any of the methods described in Antibodies: A Laboratory Manual, By Ed Harlow and David Lane, Cold Spring Harbor Laboratory Press, 1988.

Antibody or antigen binding fragment

An antibody or antigen binding fragment thereof is provided which is capable of inhibiting cellular NBCn1-mediated $Na^+$-dependent $HCO_3^-$ transport. An antibody or antigen binding fragment thereof is also provided which specifically recognizes and binds an extracellular polypeptide region of human NBCn1. In a preferred aspect of the present disclosure, the extracellular polypeptide region comprises or consists of a sequence selected from any one of SEQ ID NO: 5, 6, 7, 8, 9, 10, 11, 12, 13, 14, 15 or a fragment thereof. In one particularaspect of the present disclosure, the extracellular polypeptide region comprises or consists of a human sequence selected from any one of SEQ ID NO: 14, 15 or a fragment thereof, such as a fragment of 5-25, such as 10-25, such as 10-20 amino acids thereof. In oneaspect of the present disclosure, the antibody or antigen binding fragment thereof is capable of inhibiting cellular NBCn1-mediated $Na^+$-dependent $HCO_3^-$ transport.

The antibody according to the present disclosure may be any polypeptide or protein capable of recognising and binding an antigen in a polypeptide region of human NBCn1, preferably an extracellular polypeptide region. Preferably, the antibody is capable of specifically binding said antigen. By the term "specifically binding" is meant binding with at least 10 times higher affinity to the antigen than to a non-specific antigen (e.g. BSA). Typically, the antibody binds with an affinity corresponding to a $K_D$ of about $10^{-7}$ M or less, such as about $10^{-8}$ M or less, such as about $10^{-9}$ M or less, for example about $10^{-10}$ M or less, or even about $10^{-11}$ M or even less, when measured as apparent affinities based on $IC_{50}$ values.

Preferably said antibody is a naturally occurring antibody or a functional homologue thereof. A naturally occurring antibody is a heterotetrameric glycoprotein capable of recognising and binding an antigen comprising two identical heavy (H) chains and two identical light (L) chains inter-connected by disulfide bonds. Each heavy chain comprises or preferably consists of a heavy chain variable region (abbreviated herein as $V_H$) and a heavy chain constant region (abbreviated herein as $C_H$). Each light chain comprises, or preferably consists of, a light chain variable region (abbreviated herein as $V_L$) and a light chain constant region (abbreviated herein as $C_L$). The $V_H$ and $V_L$ regions can be further subdivided into regions of hypervariability, termed complementarity determining regions (CDRs), interspersed with regions that are more conserved, termed framework regions (FRs). Each $V_H$ and $V_L$ comprises and preferably consists of three CDRs and four FRs, arranged from amino-terminus to carboxy-terminus in the following order: FR1, CDR1, FR2, CDR2, FR3, CDR3, FR4.

The naturally occurring antibody may also be a heavy-chain antibody (HCAbs) as produced by camelids (camels, dromedaries and llamas). HCAbs are homodimers of heavy chains only, devoid of light chains and the first constant domain (Hamers-Casterman et al., 1993).

Another possibility is New or Nurse Shark Antigen Receptor (NAR) protein, which exists as a dimer of two heavy chains with no associated light chains. Each chain is composed of one variable (V) and five constant domains. The NAR proteins constitute a single immunoglobulin variable-like domain (Greenberg, A. S., Avila, D., Hughes, M., Hughes, A., McKinney, E. C. & Flajnik, M. F. (1995) Nature (London) 374, 168-173.) which is much lighter than an antibody molecule.

Naturally occurring antibodies according to the disclosure may consist of one heterotetramer or they may comprise several identical heterotetramers. Thus, the naturally occurring antibody according to the disclosure may for example be selected from the group consisting of IgG, IgM, IgA, IgD and IgE. The subunit structures and three-dimensional configurations of these different classes of immunoglobulins are well known.

Naturally occurring antibodies according to the disclosure may be antibodies of a particular species, for example the antibody may be a murine, a rat, a rabbit, a goat, a sheep, a chicken, a donkey, a camelid or a human antibody. The antibody according to the disclosure may however also be a hybrid between antibodies from several species, for example the antibody may be a chimeric antibody, such as a humanised antibody.

The antibody according to the disclosure may be a monoclonal antibody, such as a naturally occurring monoclonal antibody or it may be polyclonal antibodies, such as naturally occurring polyclonal antibodies.

The antibody may be any protein or polypeptide containing an antigen binding site, such as a single polypeptide, a protein or a glycoprotein. Preferably, the antigen binding site comprises at least one CDR, or more preferably a variable region.

Thus the antigen binding site may comprise a $V_H$ and/or $V_L$. In an antibody, the $V_H$ and $V_L$ together may contain the antigen binding site, however, either one of the $V_H$ or the $V_L$ may comprise an antigen binding site. In particular, the CDRs may identify the specificity of the antibody and accordingly it is preferred that the antigen binding site comprises one or more CDRs, preferably at least 1, more preferably at least 2, yet more preferably at least 3, even more preferably at least 4, yet more preferably at least 5, even more preferably 6 CDRs. It is preferable that the antigen binding site comprises at least one CDR3, more preferably at least the CDR3 of the heavy chain.

The antibody may for example be an antigen binding fragment of an antibody, preferably an antigen binding fragment of a naturally occurring antibody, a heterospecific antibody, a single chain antibody or a recombinant antibody. An antibody according to the disclosure may comprise one or more antigen binding sites. Naturally occurring heterotetrameric antibodies comprises two antigen binding sites.

*Heterospecific antibodies*

The antibody according to the disclosure may also be a "heterospecific antibody", such as a bispecific antibody. A bispecific antibody is a protein or polypeptide, which comprises two different antigen binding sites with different specificities. For example, the bispecific antibody may recognise and bind to two different antigens or it may recognise and bind to two different epitopes within the same antigen. The term "heterospecific antibody" is intended to include any protein or polypeptide, which has more than two different antigen binding sites with different specificities. For example, the heterospecific antibody may recognise and bind to three different antigens or it may recognise and bind to different epitopes on the same antigen. Accordingly, the disclosure includes, but is not limited to, bispecific, trispecific, tetraspecific, and other multispecific antibodies, which are directed to extracellular polypeptide fragments of human NBCn1.

Bispecific antibodies may for example be prepared starting from monoclonal antibodies, for example by fusing two hybridomas in order to combine their specificity, by chemical crosslinking or using recombinant technologies.

For example the $V_H$ and $V_L$ of two different antibodies (1 and 2) may be linked by recombinant means to form "cross-over" chains $V_H1$-$V_L2$ and $V_H2$-$V_L1$, and then dimerised to reassemble both antigen-binding sites (see WO 94/09131). Bispecific antibodies may also be prepared by linking two single chain antibodies with different specificity genetically as for example described in WO 94/13806. Also, two antigen binding fragments of an antibody may be linked.

*Human and humanised antibodies*

It is not always desirable to use non-human antibodies for human therapy, and accordingly, the antibody of the disclosure may be a human antibody or a humanised antibody.

Thus, the antibody according to the disclosure may be a human or a humanised antibody. A human antibody as used herein is an antibody, which is obtained from a system using human immunoglobulin sequences. Human antibodies may for example be antibodies isolated from an animal (*e.g.*, a mouse) that is transgenic or transchromosomal for human immunoglobulin genes or a hybridoma prepared therefrom. Human antibodies may also be isolated from a host cell transformed to express the antibody, *e.g.*, from a transfectoma. Human antibodies may also be isolated from a recombinant, combinatorial human antibody library.

Human antibodies have variable and constant regions derived from human germline immunoglobulin sequences. In certainaspects of the present disclosure, however, such recombinant human antibodies can be subjected to *in vitro* mutagenesis or *in vivo* somatic mutagenesis and thus the amino acid sequences of the $V_H$ and $V_L$ regions of the recombinant antibodies are sequences that, while derived from and related to human germline $V_H$ and $V_L$ sequences, may not naturally exist within the human antibody germline repertoire *in vivo.*

A human antibody is preferably at least 90%, more preferably at least 95%, even more preferably at least 96%, 97%, 98%, or 99% identical in amino acid sequence to the amino acid sequence encoded by a wild type human immunoglobulin gene.

Said transgenic of transchromosomal animal may contain human immunoglobulin gene miniloci that encode unrearranged human heavy ($\mu$ and/or $\gamma$) and $\kappa$ light chain immunoglobulin sequences. Furthermore, the animal may contain one or more mutations that inactivate the endogenous heavy and light chain loci. Examples of such animals are described in Lonberg, N. et al. (1994) Nature 368 (6474):856-859 and WO 02/43478.

The antibody according to the disclosure may be a chimeric antibody, i.e. an antibody comprising regions derived from different species. The chimeric antibody may for example comprise variable regions from one species of animal and constant regions from another species of animal. For example, a chimeric antibody can be an antibody having variable regions which derive from a mouse monoclonal antibody and constant regions which are human. Such antibodies may also be referred to as humanised antibodies.

Thus, the antibody according to the disclosure may also be a humanised antibody, which is encoded partly by sequences obtained from human germline immunoglobulin sequences and partly from other sequences. Said other sequences are preferably germline immunoglobulins from other species, more preferably from other mammalian species. In particular a humanised antibody may be an antibody in which the antigen binding site is derived from an immunoglobulin from a non-human species, preferably from a non-human mammal, e.g. from a mouse or a rat, whereas some or all of the remaining immunoglobulin-derived parts of the molecule is derived from a human immunoglobulin. The antigen binding site from said non-human species may for example consist of a complete $V_L$ or $V_H$ or both or one or more CDRs grafted onto appropriate human framework regions in $V_L$ or $V_H$ or both. Thus, in a humanized antibody, the CDRs can be from a mouse or rat monoclonal antibody and the other regions of the antibody are of human origin.

*Recombinant antibodies*

The antibody according to the present disclosure may also be a recombinant antibody, i.e. an antibody prepared, expressed, created or isolated by recombinant means.

*Single chain antibodies*

Naturally occurring antibodies are heterotetramers. However, the antibody according to the present disclosure may also be a single polypeptide comprising one or more antigen binding sites. Such antibodies are also referred to as "single chain antibodies". Thus the antibody according to the present disclosure may also be a single chain antibody.

Single chain antibodies may comprise the two domains of the Fv fragment, $V_L$ and $V_H$. To obtain such single chain antibodies the genes encoding the $V_L$ and $V_H$ may be joined, using recombinant methods. Usually they are separated by a synthetic linker, for example a linker of 5 to 100, such as of 5 to 50, for example of 10 to 25 amino acids. Said linker may either connect the N-terminus of the $V_H$ with the C-terminus of the $V_L$, or *vice versa.* This enables production of a single protein chain in which the $V_L$ and $V_H$ regions pair to form monovalent antibody-like molecules (also known as single chain antibodies or single chain Fv (scFv); see *e.g.,* Bird et al. (1988) Science 242:423-426; and Huston et al. (1988) Proc. Natl. Acad. Sci. USA 85:5879-5883).

The single chain antibody may also be a divalent antibody, e.g. a single peptide chain comprising two $V_H$ and two $V_L$ regions, which may each be linker by linker. The single chain antibody may also be a multivalent antibody, e.g. a single peptide chain comprising multiple $V_H$ and multiple $V_L$ regions, which may each be linker by linker. The $V_H$ and $V_L$ regions may be identical or different, yielding monospecific or heterospecific antibodies, respectively.

Said $V_H$ and said $V_L$ may be a naturally occurring $V_H$ or $V_L$, or a synthetic $V_H$ and $V_L$ comprising at least one antigen binding site. Preferably said $V_H$ and $V_L$ are naturally occurring $V_H$ and $V_L$.

Antigen binding fragments of antibodies

Antigen binding fragments of antibodies are fragments of antibodies retaining the ability to specifically bind to an antigen. Preferably, said fragment is an antigen binding fragment of a naturally occurring antibody.

It has been shown that the antigen-binding function of an antibody can be performed by fragments of a full-length antibody. Examples of antigen binding fragments of naturally occurring antibodies include for example (i) a Fab fragment, a monovalent fragment consisting of the $V_L$, $V_H$, $C_L$ and $C_{H1}$ domains; (ii) a $F(ab')_2$ fragment, a bivalent fragment comprising two Fab fragments linked by a disulfide bridge at the hinge region; (iii) a Fd fragment consisting of the $V_H$ and $C_{H1}$ domains; (iv) a Fv fragment consisting of the $V_L$ and $V_H$ domains of a single arm of an antibody or (v) a dAb fragment (Ward et a/., (1989) Nature 341:544-546), which consists of a $V_H$ domain. Fab fragments may be prepared by papain digestion. $F(ab')_2$ fragments may be prepared by pepsin treatment.

The antigen binding fragment of an antibody preferably comprise at least one complementarity determining region (CDR) or more preferably a combination of two or more isolated CDRs which may optionally be joined by a synthetic linker.

A further example of an antigen binding fragment of an antibody is binding-domain immunoglobulin fusion proteins comprising (i) an antigen binding site fused to an immunoglobulin hinge region polypeptide, (ii) an immunoglobulin heavy chain CH2 constant region fused to the hinge region, and (iii) an immunoglobulin heavy chain CH3 constant region fused to the CH2 constant region. The antigen binding site can be a heavy chain variable region or a light chain variable region. Such binding-domain immunoglobulin fusion proteins are further disclosed in US 2003/0118592 and US 2003/0133939. These antibody fragments are obtained using conventional techniques known to those with skill in the art, and the fragments are screened for utility in the same manner as are intact antibodies.

The antigen binding fragment of an antibody may also be diabodies, which are small antibody fragments with two antigen-binding sites. Diabodies preferably comprises a heavy chain variable domain (VH) connected to a light chain variable domain (VL) in the same polypeptide chain (VH-VL). By using a linker that is too short to allow pairing between the two domains on the same chain, the domains are forced to pair with the complementary domains of another chain and create two antigen-binding sites. Diabodies are described more fully in, for example, EP 404,097; WO 93/11161; and Hollinger et al., Proc. Natl. Acad Sci. USA 90: 6444-6448 (1993).

**Method of producing antibody**

In general, methods of producing antibodies are well-known to those of skill in the art. Antibodies can be produced using antigenic epitope-bearing peptides or polypeptides derived from NBCn1. Antigenic epitope-bearing peptides and polypeptides of the present disclosure preferably contain a sequence of at least four, or between 5 and 100, such as between 10 and 100, such as between 15 and 80, for example between 15 and 70, such as between 15 and 60, such as between 15 and 60, such as between 15 and 40, such as between 15 and 30 amino acids contained within NBCn1, such as SEQ ID NO: 1 or 2 or 14 and/or 15. However, peptides or polypeptides comprising a larger portion of NBCn1, for example containing from 100 amino acids and any length up to and including the entire amino acid sequence of NBCn1 are also useful as antigenic epitope-bearing peptides or polypeptides for producing antibodies of the present disclosure.

The antibodies of the present disclosure are preferably produced using peptides or polypeptides comprising a region of an extracellular loop of NBCn1 or a fragment thereof, such as at least four, or between 5 and 100, such as between 10 and 100, such as between 15 and 80, for example between 15 and 70, such as between 15 and 60, such as between 15 and 60, such as between 15 and 40, such as between 15 and 30 amino acids thereof. In one aspect of the present disclosure, the antigenic epitope-bearing peptides or polypeptides comprise or consists of:

a) EL2, human and mouse: SPVITFGGLLGEATEGRISAIESLFGASLT (SEQ ID NO: 5)
b) EL3, human:

KLFDLGETYAFNMHNNLDKLTSYSCVCTEPPNPSNETLAQWKKDNITAHNISW
RNLTVSECKKLRGVFLGSACGHHGP (SEQ ID NO: 6)

c) EL3, mouse:

KLFHLGEIYAFNMHNNLDELTSYTCVCAEPSNPSNETLELWKRKNITAYSVSW
GNLTVSECKTFHGMFVGSACGPHGP (SEQ ID NO: 7)

d) EL4, human: PSPKLHVPEKFEPTHPERGWIISPLGDNPW (SEQ ID NO: 8)
e) EL4, mouse: PSPKLHVPEKFEPTDPSRGWIISPLGDNPW (SEQ ID NO: 9)
f) EL5, human and mouse: SISHVNSLKVESECSAPGEQPKFLGIREQR (SEQ ID NO: 10)
g) Human:

MERFRLEKKLPGPDEEAVVDLGKTSSTVNTKFEKEELESHRAVYIGVHVPFSK
ESRRRHRHRGHKHHHRRRKDKESDKEDGRESPSYDTPSQRVQFILGTEDDD
EEHIPHDLFTEMDELCYRDGEEYEWKETARWLKFEEDVEDGGDRWSKPYVA
TLSLHSLFELRSCILNGTVMLDMRASTLDEIADMVLDNMIASGQLDESIRENVR
EALLKRHHHQNEKRFTSRIPLVRSFADIGKKHSDPHLLERNGEGLSASRHSLR
TGLSASNLSLRGESPLSLLLGHLLPSSRAGTPAGSRCTTPVPTPQNSPPSSPS
ISRLTSRSSQESQRQAPELLVSPASDDIPTVVIHPPEEDLEAALKGEEQKNEEN
VDLTPGILASPQSAPGNLDNSKSGEIKGNGSGGSRENSTVDFSKVDMNFMRK
IPTGAEASNVLVGEVDFLERPIIAFVRLAPAVLLTGLTEVPVPTRFLFLLLGPAG
KAPQYHEIGRSIATLMTDEIFHDVAYKAKDRNDLLSGIDEFLDQVTVLPPGEWD
PSIRIEPPKSVPSQEKRKIPVFHNGSTPTLGETPKEAAHHAGPELQRTGRLFG
GLILDIKRKAPFFLSDFKDALSLQC (SEQ ID NO: 11)

h) Human: ATVLSISHVNSLKVESECSAPGEQPKFLGIREQRVT (SEQ ID NO: 12)
i) Human: DRIKLFGMPAKHQPDLIYLRYVPLWKVHIFTVIQLTC (SEQ ID NO: 13)
j) NBCn1_EL3h1.1: [Hz]-HNNLDKLTSYSCVCTEPPNPSNETLAQWKKDNITA-amide (SEQ ID NO: 14)
k) NBCn1_EL3h2.1: [Hz]-LAQWKKDNITAHNISWRNLTVSECKKLRGVFLGSA-amide (SEQ ID NO: 15).

However, any fragment of any of SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14 and SEQ ID NO: 15 may be also be used, for example a fragment comprising at least four amino acids, such as between 5 and 75, such as between 10 and 75, such as between 15 and 70, for example between 15 and 60, such as between 15 and 60, such as between 15 and 50, such as between 15 and 40, such as between 15 and 30 amino acids thereof. In a preferredaspect of the present disclosure, the antibodies are produced using peptides comprising an extracellular polypeptide region of NBCn1 and preferably, the extracellular polypeptide region comprises or consists of a sequence selected from any one of SEQ ID NO: 14 and/or 15 or a fragment thereof, such as a fragment of 5-25, such as 10-25, such as 10-20 amino acids thereof.

In a preferred aspect of the present disclosure, the antigenic epitope-bearing peptides or polypeptides are human, such as SEQ ID NO: 5, 6, 8, 10, 11, 12, 13, 14 and 15.

It is desirable that the amino acid sequence of the epitope-bearing peptide is selected to provide substantial solubility in aqueous solvents (i.e., the sequence includes relatively hydrophilic residues, while hydrophobic residues are preferably avoided). Moreover, amino acid sequences containing proline residues may also be desirable for antibody production.

Recombinant antibodies of the disclosure may for example be produced using a synthetic library or by phage display, preferably using the extracellular domains of NBCn1 to generate antibodies against extracellular domains of NBCn1. Since selection of recombinant antibodies from phage display libraries and synthetic libraries do not build on immunogenicity principles, but solely on binding ability, it is capable of providing antibodies that bind to a more diverse array of epitopes. For example, antibodies binding to synthetic peptides corresponding to surface exposed regions of NBCn1 can be isolated. Next, antibodies binding to cells with high expression levels of NBCn1 may be isolated. The first step may follow any conventional selection protocol (e.g. Mandrup OA, Friis NA, Lykkemark S, Just J and Kristensen P. A novel heavy domain antibody library with functionally optimized complementarity determining regions. PLoS One 8: e76834, 2013), and the second step could utilize any selection procedures (e.g. Sorensen MD, Agerholm IE, Christensen B, Kolvraa S and Kristensen P. Microselection--affinity selecting antibodies against a single rare cell in a heterogeneous population. J Cell Mol Med 14: 1953-1961, 2010; or Sorensen MD and Kristensen P. Selection of antibodies against a single rare cell present in a heterogeneous population using phage display. Nat Protoc 6: 509-522, 2011; or Sorensen MD, Melchjorsen CJ, Mandrup OA and Kristensen P. Raising antibodies against circulating foetal cells from maternal peripheral blood. Prenat Diagn 33: 284-291, 2013.). Each selection step produces individual bacterial colonies from which monoclonal (phage) antibodies can be produced. The monoclonal phage antibodies are then typically tested in conventional cell ELISA, where antibody binding is compared between cells expressing high amounts of NBCn1 (e.g., breast cancer cells isolated from WT mice) and cells with no or very low NBCn1 expression (e.g., breast cancer cells isolated from NBCn1 knockout mice). In addition, the antibodies can be tested for binding to purified proteins. Following selection, the top monoclonal phage antibody fragments can be cloned into expression vectors, which comprise the constant regions of the IgG or different expression tags.

Recombinant antibodies may be produced in microbial host organisms, such as bacteria, yeast or cell cultures of cells derived from multicellular organisms. Frequently, Escherichia coli is useful as host organism. Frequently, recombinant antibodies are fragments of naturally occurring antibodies comprising at least one antigen binding site, such as a Fab fragment, a Fv fragment or the recombinant antibody is a scFV.

Recombinant antibodies may be identified using various systems, such as phage display or ribosome display. In a preferred aspect of the present disclosure, the present disclosure relates to methods of producing antibodies targeting extracellular regions of NBCn1 using phage display. The starting point of phage display is usually a library of antibodies, such as single chain antibodies or fragments of naturally occurring antibodies expressed by a phage. Various different kinds of phages are suitable for use in phage display, e.g. M13, fd filamentous phage, T4, T7 or λ phage. Phagemids may also be used, but that usually requires use of a helper phage. Typically, the library comprises in the range of $10^7$ to $10^{15}$, such as $10^9$ to $10^{11}$ different phages. The antibodies may be either of native or immune origin. The antibodies of the library may be fused to a phage coat protein (e.g. g3p or g8p) in order to ensure display on the surface. Thus, the antibody (fragment) may be encoded by a nucleic acid sequence, which is cloned upstream or downstream of a nucleic acid encoding a phage coat protein, which is operably linked to a suitable promoter.

The genomic information coding for antibody e.g. for the antibody variable domains may be obtained from B cells of non-immunised or immunised donors using recombinant DNA technology to amplify the VH and VL gene segments and cloning into an appropriate phage. Synthetic libraries may be prepared by rearranging VH and VL gene segments in vitro and/or by introducing artificial sequences into VH and VL gene segments. For example synthetic libraries may be prepared using a VH and VL gene framework, but introducing into this artificial complementarity determining regions (CDRs), which may be encoded by random oligonucleotides.

The library may also be different libraries, which are then combined in the host cell. Thus, one library may comprise heavy chain sequences, such as the heavy chain Fv fragment or Fab fragment or VH and the other light chain sequences, such as the light chain Fv fragment or Fab fragment VL.

Typically, several rounds of selection, e.g. 2, 3, 4, 5 or 5, such as 2 to 5 or 2 to 4 or 2 to 3 rounds of selection are performed. This may be done by immobilising the antigen, contacting the antigen with the phage and isolation of bound phages. The antigen may be immobilised on any suitable solid surface, such as a plastic surface, beads (such as magnetic beads)

In one aspect, the present disclosure relates to a method of selecting antibodies, said method comprising the steps of

a) Providing an antibody library, preferably a bacteriophage library, which may comprise in the range of $10^7$ to $10^{15}$, such as $10^9$ to $10^{11}$, different phages
b) Providing one or more NBCn1 antigens, preferably an antigen of an extracellular region of NBCn1,
c) Contacting said antibody library with said one or more NBCn1 antigens, and
d) Selecting antibodies, which bind said one or more NBCn1 antigens.

The method may comprise additional rounds of selection, such as 2, 3, 4, 5, 5 rounds of selection or more, where a library consisting of the selected antibodies in step d) are contacted with an NBCn1 antigen and again selecting antibodies, which bind the NBCn1 antigen. Thus, in one aspect of the present disclosure, the method comprises repeating steps a) -d) once or twice or more, preferably 1, 2, 3 times, where the antibody library in step a) of additional rounds corresponds to the antibodies selected in step d) of the previous round.

The antibodies of the library are in one aspect of the present disclosureselected for their ability to bind peptides or polypeptide fragments of NBCn1 as described elsewhere herein, in particular extracellular fragments (e.g. a polypeptide region comprising a sequence selected from any one of SEQ ID NO: 5-10 or SEQ ID NO: 14-15 or a fragment thereof), or the full-length NBCn1, preferably natively folded NBCn1. In a preferred aspect of the present disclosure, the antigens are immobilized on a solid surface, for example on solid beads.

The provided NBCn1 antigen may be provided as epithelial organoids, for example, epithelial organoids of breast cancer tissue. Thus, in one aspect of the present disclosure, the antigens are provided as epithelial organoids and suitable antibodies are selected by their binding ability to antigens presented on these epithelial organoids. For example, specific antibodies can be identified based on the different binding abilities to epithelial organoids of breast cancers from NBCn1 KO and WT mice.

Polyclonal antibodies to recombinant protein or isolated from natural sources can be prepared using methods well-known to those of skill in the art. See, for example, Green et al., "Production of Polyclonal Antisera," in Immunochemical Protocols (Manson, ed.), pages 1 to 5 (Humana Press 1992), and Williams et al., "Expression of foreign proteins in E. coli using plasmid vectors and purification of specific polyclonal antibodies," in DNA Cloning 2: Expression Systems, 2nd Edition, Glover et al. (eds.), page 15 (Oxford University Press 1995). The immunogenicity of a polypeptide can be increased through the use of an adjuvant, such as alum (aluminum hydroxide) or Freund's complete or incomplete adjuvant. Polypeptides useful for immunization also include fusion polypeptides, such as fusions of or a portion thereof with an immunoglobulin polypeptide or with maltose binding protein. The polypeptide immunogen may be a full-length molecule or a portion thereof. If the polypeptide portion is "hapten-like," such portion may be advantageously joined or linked to a macromolecular carrier (such as keyhole limpet hemocyanin (KLH), bovine serum albumin (BSA) or tetanus toxoid) for immunization.

Although polyclonal antibodies are typically raised in animals such as horses, cows, dogs, chicken, rats, mice, rabbits, guinea pigs, goats, or sheep, an antibody specific for a polypeptide according to the present disclosure may also be derived from a subhuman primate antibody. General techniques for raising diagnostically and therapeutically useful antibodies in baboons may be found, for example, in Goldenberg et al., international patent publication No. WO 91/11465, and in Losman et al., Int. J. Cancer 46:310 (1990).

In one aspect of the disclosure, a method is provided for producing an antibody specifically recognizing and binding an extracellular polypeptide region of human NBCn1, wherein said antibody is capable of inhibiting cellular NBCn1-mediated $Na^+$-dependent $HCO_3^-$ transport, said method comprising the steps of

a) administering to an animal, preferably a mammal, such as horses, cows, dogs, chicken, rats, mice, rabbits, guinea pigs, goats, or sheep, an extracellular polypeptide fragment of human NBCn1 (e.g. as defined by any of SEQ ID NO: 3-10 or 14-15) or a homolog at least 90% identical thereto, such as at least 91, 92, 93, 94, 95, 96, 97 or at least 98% or 99% identical thereto,
b) isolating antibodies from said mammal,
c) testing whether antibodies are capable of inhibiting cellular NBCn1-mediated $Na^+$-dependent $HCO_3^-$ transport and/or testing whether such antibodies are capable of specifically recognizing and binding an extracellular polypeptide region of human NBCn1 and
d) selecting antibodies capable of inhibiting cellular NBCn1-mediated $Na^+$-dependent $HCO_3^-$ transport and/or selecting antibodies capable of specifically recognizing and binding an extracellular polypeptide region of human NBCn1.

Alternatively, monoclonal antibodies specific for polypeptides according to the present disclosure can be generated. Rodent monoclonal antibodies to specific antigens may be obtained by methods known to those skilled in the art (see, for example, Kohler et al., Nature 256:495 (1975), Coligan et al. (eds.), Current Protocols in Immunology, Vol. 1, pages 2.5.1 2.6.7 (John Wiley & Sons 1991) ["Coligan"], Picksley et al., "Production of monoclonal antibodies against proteins expressed in E. coli," in DNA Cloning 2: Expression Systems, 2nd Edition, Glover et al. (eds.), page 93 (Oxford University Press 1995)).

Briefly, monoclonal antibodies can be obtained by injecting mice or rabbits with a composition comprising a gene product, verifying the presence of antibody production by removing a serum sample, removing the spleen to obtain B-lymphocytes, fusing the B-lymphocytes with myeloma cells to produce hybridomas, cloning the hybridomas, selecting positive clones which produce antibodies to the antigen, culturing the clones that produce antibodies to the antigen, and isolating the antibodies from the hybridoma cultures.

In addition, an antibody specific for polypeptides according to the present disclosure of the present disclosure may be derived from a human monoclonal antibody. Human monoclonal antibodies are obtained from transgenic mice that have been engineered to produce specific human antibodies in response to antigenic challenge. In this technique, elements of the human heavy and light chain locus are introduced into strains of mice derived from embryonic stem cell lines that contain targeted disruptions of the endogenous heavy chain and light chain loci. The transgenic mice can synthesize human antibodies specific for human antigens, and the mice can be used to produce human antibody-secreting hybridomas. Methods for obtaining human antibodies from transgenic mice are described, for example, by Green et al., Nature Genet. 7:13 (1994), Lonberg et al., Nature 368:856 (1994), and Taylor et al., Int. Immun. 6:579 (1994).

Monoclonal antibodies can be isolated and purified from hybridoma cultures by a variety of well-established techniques. Such isolation techniques include affinity chromatography with Protein-A Sepharose, size-exclusion chromatography, and ionexchange chromatography (see, for example, Coligan at pages 2.7.1 2.7.12 and pages 2.9.1 2.9.3; Baines et al., "Purification of Immunoglobulin G (IgG)," in Methods in Molecular Biology, Vol. 10, pages 79 104 (The Humana Press, Inc. 1992)).

For particular uses, it may be desirable to prepare fragments of antibodies specific for extracellular regions of NBCn1. Such antibody fragments can be obtained, for example, by proteolytic hydrolysis of the antibody. Antibody fragments can be obtained by pepsin or papain digestion of whole antibodies by conventional methods. As an illustration, antibody fragments can be produced by enzymatic cleavage of antibodies with pepsin to provide a 5S fragment denoted $F(ab')_2$. This fragment can be further cleaved using a thiol reducing agent to produce 3.5S Fab' monovalent fragments. Optionally, the cleavage reaction can be performed using a blocking group for the sulfhydryl groups that result from cleavage of disulfide linkages. As an alternative, an enzymatic cleavage using pepsin produces two monovalent Fab fragments and an $F_c$ fragment directly. These methods are described, for example, by Goldenberg, U.S. Pat. No. 4,331,647, Nisonoff et al., Arch Biochem. Biophys. 89:230 (1960), Porter, Biochem. J. 73:119 (1959), Edelman et al. and Coligan, both in Methods in Enzymology Vol. 1, (Academic Press 1967).

Other methods of cleaving antibodies, such as separation of heavy chains to form monovalent light-heavy chain fragments, further cleavage of fragments, or other enzymatic, chemical or genetic techniques may also be used, so long as the fragments bind to the antigen that is recognized by the intact antibody.

For example, Fv fragments comprise an association of $V_H$ and $V_L$ chains. This association can be noncovalent, as described by Inbar et al., Proc. Nat'l Acad. Sci. USA 69:2659 (1972). Alternatively, the variable chains can be linked by an intermolecular disulfide bond or cross-linked by chemicals such as glutaraldehyde (see, for example, Sandhu, Crit. Rev. Biotech. 12:437 (1992)).

The Fv fragments may comprise $V_H$ and $V_L$ chains, which are connected by a peptide linker. These single-chain antigen binding proteins (scFv) are prepared by constructing a structural gene comprising DNA sequences encoding the $V_H$ and $V_L$ domains which are connected by an oligonucleotide. The structural gene is inserted into an expression vector, which is subsequently introduced into a host cell, such as E. coli. The recombinant host cells synthesize a single polypeptide chain with a linker peptide bridging the two V domains. Methods for producing scFvs are described, for example, by Whitlow et al., Methods: A Companion to Methods in Enzymology 2:97 (1991) (also see, Bird et al., Science 242:423 (1988), Ladner et al., U.S. Pat. No. 4,946,778, Pack et al., Bio/Technology 11:1271 (1993), and Sandhu, supra).

As an illustration, a scFV can be obtained by exposing lymphocytes to polypeptide in vitro, and selecting antibody display libraries in phage or similar vectors (for instance, through use of immobilized or labeled protein or peptide). Genes encoding polypeptides having potential polypeptide binding domains can be obtained by screening random peptide libraries displayed on phage (phage display) or on bacteria, such as E. coli. Nucleotide sequences encoding the polypeptides can be obtained in a number of ways, such as through random mutagenesis and random polynucleotide synthesis. These random peptide display libraries can be used to screen for peptides, which interact with a known target which can be a protein or polypeptide, such as a ligand or receptor, a biological or synthetic macromolecule, or organic or inorganic substances. Techniques for creating and screening such random peptide display libraries are known in the art (Ladner et al., U.S. Pat. No. 5,223,409, Ladner et al., U.S. Pat. No. 4,946,778, Ladner et al., U.S. Pat. No. 5,403,484, Ladner et al., U.S. Pat. No. 5,571,698, and Kay et al., Phage Display of Peptides and Proteins (Academic Press, Inc. 1996)) and random peptide display libraries and kits for screening such libraries are available commercially, for instance from CLONTECH Laboratories, Inc. (Palo Alto, Calif.), Invitrogen Inc. (San Diego, Calif.), New England Biolabs, Inc. (Beverly, Mass.), and Pharmacia LKB Biotechnology Inc. (Piscataway, N.J.). Random peptide display libraries can be screened using the sequences disclosed herein to identify proteins which bind to.

Another form of an antibody fragment is a peptide coding for a single complementarity-determining region (CDR). CDR peptides ("minimal recognition units") can be obtained by constructing genes encoding the CDR of an antibody of interest. Such genes are prepared, for example, by using the polymerase chain reaction to synthesize the variable region from RNA of antibody-producing cells (see, for example, Larrick et al., Methods: A Companion to Methods in Enzymology 2:106 (1991), Courtenay-Luck, "Genetic Manipulation of Monoclonal Antibodies," in Monoclonal Antibodies: Production, Engineering and Clinical Application, Ritter et al. (eds.), page 166 (Cambridge University Press 1995), and Ward et al., "Genetic Manipulation and Expression of Antibodies," in Monoclonal Antibodies: Principles and Applications, Birch et al., (eds.), page 137 (Wiley-Liss, Inc. 1995)).

Alternatively, an antibody specific for a polypeptide according to the present disclosure may be derived from a "humanized" monoclonal antibody. Humanized monoclonal antibodies are produced by transferring mouse complementary determining regions from heavy and light variable chains of the mouse immunoglobulin into a human variable domain. Typical residues of human antibodies are then substituted in the framework regions of the murine counterparts. The use of antibody components derived from humanized monoclonal antibodies obviates potential problems associated with the immunogenicity of murine constant regions. General techniques for cloning murine immunoglobulin variable domains are described, for example, by Orlandi et al., Proc. Nat'l Acad. Sci. USA 86:3833 (1989). Techniques for producing humanized monoclonal antibodies are described, for example, by Jones et al., Nature 321:522 (1986), Carter et al., Proc. Nat'l Acad. Sci. USA 89:4285 (1992), Sandhu, Crit. Rev. Biotech. 12:437 (1992), Singer et al., J. Immun. 150:2844 (1993), Sudhir (ed.), Antibody Engineering Protocols (Humana Press, Inc. 1995), Kelley, "Engineering Therapeutic Antibodies," in Protein Engineering: Principles and Practice, Cleland et al. (eds.), pages 399 434 (John Wiley & Sons, Inc. 1996), and by Queen et al., U.S. Pat. No. 5,693,762 (1997).

Polyclonal anti-idiotype antibodies can be prepared by immunizing animals with antibodies or antibody fragments specific for a polypeptide according to the present disclosure, using standard techniques. See, for example, Green et al., "Production of Polyclonal Antisera," in Methods In Molecular Biology: Immunochemical Protocols, Manson (ed.), pages 1 12 (Humana Press 1992). Also, see Coligan at pages 241 to 247. Alternatively, monoclonal anti-idiotype antibodies can be prepared using antibodies or antibody fragments specific for a polypeptide according to the present disclosure as immunogens with the techniques, described above. As another alternative, humanized anti-idiotype antibodies or subhuman primate anti-idiotype antibodies can be prepared using the above-described techniques. Methods for producing anti-idiotype antibodies are described, for example, by Irie, U.S. Pat. No. 5,208,146, Greene, et. al., U.S. Pat. No. 5,637,677, and Varthakavi and Minocha, J. Gen. Virol. 77:1875 (1996).

## Analysis of antibody

The antibodies or antigen binding fragments provided herein specifically recognize and bind human NBCn1, in particular an extracellular polypeptide region of human NBCn1, such as a sequence selected from any one of SEQ ID NO: 5-15 or a fragment thereof. Relevant fragments are described elsewhere herein.

Such antibodies and antigen binding fragment thereof can be produced by methods described elsewhere herein. Suitable antibodies and antigen binding fragment thereof can be tested in a number of different assays. In particular, the binding abilities of an isolated antibody or antigen binding fragment thereof can be determined in an ELISA assay. Thus, in one aspect of the present disclosure, the antibody or antigen binding fragment thereof provided herein is capable of displacing one or more reference antibodies in a competitive ELISA assay.

Moreover, suitable antibodies and antigen binding fragment thereof can be analysed by immunoblotting, typically where isolated organoid membranes are incubated with the candidate antibody as primary antibody, and following washing detected by incubating with a detectable secondary antibody, which is typically conjugated to horseradish peroxidase for detection.

Suitable antibodies and antigen binding fragment thereof can also be analysed by Immunohistochemistry. For example paraffin-embedded sections of breast tissue can be used and incubated with the candidate antibodies for NBCn1, and antigen binding can subsequently be detected by incubation with a detectable secondary antibody, which is typically conjugated to horseradish peroxidase.

The function of suitable antibodies or antigen binding fragment thereof may also be determined on the basis of their ability to inhibit $Na^+$-dependent $HCO_3^-$ transport. Thus, in one aspect of the present disclosure, the antibody or antigen binding fragment thereof provided herein is capable of inhibiting cellular NBCn1-mediated $Na^+$-dependent $HCO_3^-$ transport. One approach could be to test the ability to inhibit NBCn1-mediated $Na^+$-dependent $HCO_3^-$ transport in cells, for example in cancer cells, such as epithelial breast cancer organoids. NBCn1 is involved in maintaining cellular pH and in particular in avoiding low pH in cancer cells. Therefore, the function of antibodies and antigen binding fragments thereof in recognizing and binding NBCn1 and inhibiting cellular $Na^+$-dependent $HCO_3^-$ transport can be determined by measuring intracellular pH in human cells and for example mice cells from NBCn1 KO vs. WT mice. Intracellular pH can also be determined in human cancer cell lines, such as MCF7. In particular, the intracellular pH may be determined in organoids from human and/or mice NBCn1 KO vs. WT, preferably in epithelial breast cancer organoids. Antibodies or antigen binding fragments thereof, for which the intracellular pH is decreased when supplied to cells or organoids, are functional as NBCn1 inhibitors, and are consequently suitable for use in medicine, in particular in anticancer therapy as described elsewhere herein.

Intracellular pH ($pH_i$) can be determined as described in the example. In particular, $pH_i$ can be determined as recovery of $pH_i$ from acidosis in isolated organoids loaded with BCECF-AM. Epifluorescence is typically measured with a camera-based fluorescence imaging system during alternating excitation at approximately 485 and 440 nm. The 485/440 BCECF fluorescence ratio can be converted to $pH_i$ using the high-$[K^+]$ nigericin calibration technique. Intracellular acidification can be induced with the $NH_4^+$ prepulse technique (traces are shown in Figure 3B), and acid-base transport activities calculated as the product of the $pH_i$ recovery rate and the buffering capacity of that same organoid. Intracellular buffering capacity can be calculated based on the change in $pH_i$ upon washout of $NH_4Cl$ with the assumption that $NH_3$ is in equilibrium across cell membranes.

## Medical use and treatment

The antibodies and compositions of the present disclosure are particularly useful in medicine and in therapeutic methods. In one aspect, the disclosure relates to an antibody of the present disclosure or antigen binding fragment thereof for use in medicine. In particular, the disclosure provides an antibody of the present disclosure or antigen binding fragment thereof for use in the treatment of cancer, in particular breast cancer. In fact, the antibody or antigen binding fragment thereof may be used for the treatment of any hyperproliferative disorder, including cancers, hyperproliferative/inflammatory skin conditions, such as psoriasis and other eczemas, and hyperproliferative disorders of the stomach and gastrointestinal tract.

The antibody or antigen binding fragment thereof may also be used in the treatment of atherosclerosis or restenosis.

In one aspect, the disclosure relates to the use of an antibody of the present disclosure or antigen binding fragment thereof in the manufacture of a medicament. In particular, the disclosure provides the use of an antibody of the present disclosure or antigen binding fragment thereof in the manufacture of a medicament for the treatment of any hyperproliferative disorder, preferably cancer, in particular breast cancer, or for the treatment of atherosclerosis or restenosis.

The disclosure also provides a method for treatment of a clinical condition, such as any hyperproliferative disorder, preferably cancer, in particular breast cancer, or atherosclerosis or restenosis, said method comprising administering a therapeutically effective amount of an antibody of the present disclosure or antigen binding fragment thereof to a subject in need thereof.

The uses, medicaments and methods of treatment are preferably intended for mammalian subjects and in particular human subjects.

The uses, medicaments and methods of treatment can be directed towards any cancer and in a preferred aspect of the present disclosure, the uses, medicaments and methods are directed to the treatment of breast cancer, atherosclerosis and/or restenosis. In particular, the treatment can be directed to those having a triple-negative breast cancer, which refers to breast cancers that do not express the genes for estrogen receptor (ER), progesterone receptor (PR) and Her2/neu. These cancers are difficult to treat since most available chemotherapies target one of the three receptors. However, in another aspect of the present disclosure, the medical uses and methods of treatment of the disclosure are directed towards HER2-positive breast cancers. Overexpression of HER2 in breast cancer cells is associated with a strong increase in the expression of NBCn1. In yet another aspect of the present disclosure, however, the medical uses and methods of treatment of the disclosure are directed towards estrogen receptor-positive breast cancers.

The treatment can be applied at any stage of a breast cancer and can also be used for prophylactic treatment of breast cancer. Thus, the treatment according to the present disclosure involves both prophylactic treatment as well as curative treatment, prevention and amelioration of symptoms associated with a clinical condition, such as cancer, in particular breast cancer.

The uses, medicaments and methods of treatment of the present disclosure may in addition to an antibody of the present disclosure or antigen binding fragment thereof involve at least one additional agent. For example, an additional agent could be an anticancer agent, in particular an agent suitable for use in breast cancer, such as a Her2 antibody. Other such suitable additional agents includes regulators of ion transport, such as $Na^+$,$H^+$-exchangers (NHEs), monocarboxylic acid transporters (MCTs) and proton pump inhibitors (PPIs).

When the treatment concerns atherosclerosis and/or restenosis, the additional agent may be chosen from active agents suitable for the treatment of atherosclerosis and/or restenosis. For example, statins (HMG-CoA reductase inhibitors) could be used as such additional agents, including Atorvastatin, Cerivastatin, Fluvastatin, Lovastatin, Mevastatin, Pitavastatin, Pravastatin, Rosuvastatin and/or Simvastatin.

The uses, medicaments and methods of treatment could be combined with chemotherapy and/or radiotherapy. The uses, medicaments and methods of treatment may also be used before or after surgical therapy.

**Inhibition of** $Na^+$-dependent $HCO_3^-$ **transport**

Generally, the present disclosure provides methods for inhibiting $Na^+$-dependent $HCO_3^-$ transport mediated by NCBn1. Thus, in one aspect, a method is provided for inhibiting cellular NBCn1-mediated $Na^+$-dependent $HCO_3^-$ transport, said method comprising providing a sufficient amount of an antibody of the present disclosure or an antigen-binding fragment thereof to target cells. This method is in one aspect of the present disclosure an in vitro method. The target cells are preferably a population of cancer cells, in particular a population of breast cancer cells, such as epithelial breast cancer.

**Administration**

In general, suitable methods of administering antibodies are well-known in the art. Thus, any suitable route of administration may be employed for providing a mammal, especially a human, with an effective dose of an antibody or antigen binding fragment thereof as provided herein. For example, oral, rectal, vaginal, topical, parenteral, ocular, pulmonary, nasal, and the like may be employed. Other examples of administration include sublingual, intravenous, intramuscular, intrathecal, subcutaneous, cutaneous and transdermal administration. In one embodiment the administration comprises inhalation, injection or implantation. The administration of the compound according to the present disclosure can result in a local (topical) effect or a body-wide (systemic) effect. In a preferred aspect of the present disclosure, the antibody or antigen binding fragment thereof is administered by intravenous injection/infusion.

Dosage forms include tablets, troches, dispersions, suspensions, solutions, capsules, creams, ointments, aerosols, and the like. Preferably compounds of the disclosure are administered orally or intravenously. The effective dose employed of antibody or antigen binding fragment thereof may vary depending on the particular compound, the mode of administration, the condition being treated and the severity of the condition being treated. Such dosage may be ascertained readily by a person skilled in the art.

In one aspect of the present disclosure the antibody or antigen binding fragment thereof of the present disclosure is administered at a dosage of from about 0.1 milligram to about 100 milligram per kilogram of body weight. For most large mammals, the total dosage is from about 1.0 milligrams to about 1000 milligrams, preferably from about 1 milligram to about 50 milligrams. In the case of a 70 kg adult human, the total dose will generally be from about 1 milligram to about 350 milligrams. For a particularly potent compound, the dosage for an adult human may be as low as 0.1 mg. The dosage regimen may be adjusted within this range or even outside of this range to provide the optimal therapeutic response.

The weekly dosage of antibody or antigen binding fragment thereof is preferably 4-8 mg/kg bodyweight. However, weekly dosage may vary over the course of treatment. Thus, an effective dose of antibody or antigen binding fragment thereof may be administered as an initial loading dose of 4-8 mg/kg bodyweight and subsequent doses of 1-5 mg/kg bodyweight, where e.g. the initial loading dose is administered in week 1, and the subsequent doses are administered weekly in the following weeks.

A complete course of treatment should preferably result in a total dose of 20-300 mg/kg bodyweight; more preferred 50-200 mg/kg bodyweight, such as about 110 mg/kg bodyweight regardless of the dosing regimen employed

The effective dose is preferably infused intravenously, for example by infusion in 5-180 minutes, preferably between 10 and 120 minutes, such as between 30 and 90 minutes.

## Composition

The present disclosure relates to an antibody or antigen binding fragment thereof specifically recognizing and binding an extracellular polypeptide region of human NBCn1 for use in medicine. When used in medicine, the antibody or antigen binding fragment thereof is usually provided as a composition, which in addition to the active agent, i.e. said antibody or antigen binding fragment thereof, comprises one or more additional components. Thus, in one aspect, the present disclosure relates to a composition, such as a pharmaceutical composition, comprising at least one antibody or antigen binding fragment thereof specifically recognizing and binding an extracellular polypeptide region of human NBCn1, as described elsewhere herein. The composition may comprise a combination of two or more such antibodies.

The pharmaceutical composition also preferably comprises a pharmaceutically acceptable excipient or carrier. The compositions may be prepared for parenteral administration, particularly in the form of liquid solutions or suspensions in aqueous physiological buffer solutions; for oral administration, particularly in the form of tablets or capsules; or for intranasal administration, particularly in the form of powders, nasal drops, or aerosols. Compositions for other routes of administration may be prepared as desired using standard methods.

Formulations for parenteral administration may contain as common excipients (i.e., pharmaceutically acceptable carriers) sterile water or saline, polyalkylene glycols such as polyethylene glycol, oils of vegetable origin, hydrogenated naphthalenes, and the like. In particular, biocompatible, biodegradable lactide polymer, lactide/glycolide copolymer, or polyoxethylene-polyoxypropylene copolymers are examples of excipients for controlling the release of a compound of the disclosure in vivo.Other suitable parenteral delivery systems include ethylene-vinyl acetate copolymer particles, osmotic pumps, implantable infusion systems, and liposomes. Formulations for inhalation administration may contain excipients such as lactose, if desired. Inhalation formulations may be aqueous solutions containing, for example, polyoxyethylene-9-lauryl ether, glycocholate and deoxycholate, or they may be oily solutions for administration in the form of nasal drops.If desired, the compounds can be formulated as gels to be applied intranasally.Formulations for parenteral administration may also include glycocholate for buccal administration

The composition may also comprise at least one additional active agent, such as another agent suitable for the treatment of cancer, in particular breast cancer, such as an additional anti breast cancer agent, a $Na^+$,$H^+$-exchanger (NHE), a monocarboxylic acid transporter (MCT) or a proton pump inhibitor (PPI).

## Examples

The present example shows that disruption of NBCn1 (Slc4a7) delays murine breast cancer development and establishes NBCn1 as a target for anti-cancer therapy. It is shown that NBCn1 maintains $pH_i$ of breast cancer tissue in a neutral to alkaline range that facilitates cancer development and progression.

## Example 1

Methods

*Mouse model and tumor induction*

NBCn1 KO mice were generated and backcrossed for at least 10 generations with WT C57BL/6j mice. Breast cancer was induced using a well-established model of breast carcinogenesis by implanting medroxyprogesterone acetate pellets (MPA, 50 mg, 90 days release; Innovative Research of America, FL, USA) subcutaneously in 6 weeks old female mice, and treating them with 1 mg dimethyl benz(a)anthracene (DMBA, in 100 μL cottonseed oil) by gavage at 9, 10, 12 and 13 weeks of age. Hormone pellets were replaced 90 and 180 days after first implantation unless tumors were detected prior to these dates. The treated mice were examined for tumor development by thorough bi-weekly palpations.

*Microdialysis sampling and analyses*

Mice were anaesthetized with an intraperitoneal injection of ketamine and xylazine on day 14 after first tumor detection. Microdialysis probes (CMA 20 Elite, CMA Microdialysis AB, Sweden) with 4 mm membrane length were placed in breast tumors and matched normal breast tissue of the same mice guided by a steel needle and split tubing. The probes were perfused by Harvard Pump 33 dual syringe pumps (Harvard Apparatus, MA, USA) at a rate of 0.5 μL/min. From each probe, 6 μL dialysate was collected and analyzed for [glucose], [lactate], [pyruvate] and [glycerol] using a CMA 600 Microdialysis Analyzer (Sweden).

*Tumor latency, growth rate and aggressiveness score*

Tumor latency was defined as the time from last oral gavage with DMBA to first tumor detection. At the end of the microdialysis procedure, mice were euthanized and tumor size(s) measured using calipers to determine tumor growth rate during the first 14 days after tumor detection. Total tumor burden was determined as the sum of individual tumor volumes (V) calculated as $V=W^2 \times L \times \pi/6$, where W is tumor width and L is tumor length. A composite "aggressiveness score" was calculated as the ratio between tumor burden and tumor latency.

*Histopathology*

Excised tumor parts and matched normal breast tissue were fixed for 30-60 minutes in 4% neutral-buffered formaldehyde (VWR, Denmark), paraffin embedded and cut to 3 μm thick sections. Deparaffinized and rehydrated slides were stained with hematoxylin and eosin. The histopathology of the breast tumors was evaluated and categorized by an experienced pathologist blinded for genotype.

*Breast epithelial organoids*

Epithelial organoids were isolated from primary breast cancer and matched normal breast tissue: Tissue samples were cut into 1 mm large pieces in phosphate-buffered saline (PBS, containing (in mM): 154.2 $Na^+$, 4.1 $K^+$, 140.6 $Cl^-$, 8.1 $HPO_4^{2-}$, and 1.5 $H_2PO_4^-$; pH 7.4) and transferred to T25 culture flasks containing advanced DMEM/F12 culture medium (Life Technologies, Denmark) supplemented with 10% fetal bovine serum (Biochrom AG, Germany)and a final concentration of 450 IU/mL collagenase type 3 (Worthington Biochemical Corporation, NJ, USA). The culture flasks were then transferred to a shaking incubator (at ~60 revolutions per minute) and left for 4 hours in an atmosphere of 5% $CO_2$ at 37°C. Next, portions of the tissue suspensions were transferred to Eppendorf tubes and organoids were allowed to sediment for 20 minutes in the incubator without shaking. The epithelial nature of the breast organoids was confirmed by staining with the epithelial cell marker CK-19 and the myofibroblast marker α-SMA (Figure 3A). To avoid culture-induced changes in cell function or protein expression patterns, all procedures were performed on freshly isolated organoids directly without cell culture.

*Intracellular pH measurements*

Recovery of $pH_i$ from acidosis was studied in freshly isolated organoids loaded for 20 minutes with 5 μM BCECF-AM. Epifluorescence was collected at 510 nm with a camera-based EasyRatioPro fluorescence imaging system (Photon Technology International, NJ, USA) during alternating excitation at 485 and 440 nm. The 485/440 BCECF fluorescence ratio was converted to $pH_i$ using the high-[$K^+$] nigericin calibration technique. Intracellular acidification was induced with the $NH_4^+$ prepulse technique (traces are shown in Figure 3B), and acid-base transport activities calculated as the product of the $pH_i$ recovery rate and the buffering capacity. Assuming that $NH_3$ is in equilibrium across cell membranes, intracellular intrinsic buffering capacities were calculated from the change in $pH_i$ upon washout of $NH_4Cl$ in absence of $CO_2/HCO_3^-$. Contribution from $CO_2/HCO_3^-$ to intracellular buffering capacity was calculated using the equation $\beta_{CO_2/HCO_3^-} = 2.3 \times [HCO_3^-]_i$. $NH_4Cl$ was initially washed out into a $Na^+$-free solution, and the rate of $Na^+$-dependent net base uptake was calculated based on the increase in $pH_i$ recovery rate upon subsequent re-addition of bath $Na^+$.

The $CO_2/HCO_3^-$-containing salt solution used for $pH_i$ recordings had the following composition (in mM): 140 $Na^+$, 4 $K^+$, 1.6 $Ca^{2+}$, 1.2 $Mg^{2+}$, 122 $Cl^-$, 24 $HCO_3^-$, 1.2 $SO_4^{2-}$, 1.18 $H_2PO_4^-$, 10 HEPES, 5.5 glucose, and 0.03 EDTA. In $Na^+$-free solutions, $Na^+$ was substituted with an equimolar amount of N-methyl-D-glucammonium ($NMDG^+$), except for $NaHCO_3$, which was replaced with choline-$HCO_3$. In $HCO_3^-$-containing solutions at pH 6.8, [$HCO_3^-$] was reduced to 6 mM by substitution with $Cl^-$. In $HCO_3^-$-free solutions, $HCO_3^-$ was substituted with an equimolar amount of $Cl^-$. $HCO_3^-$-containing solutions were aerated with a gas mixture of 5% $CO_2$ balance air, $HCO_3^-$-free solutions were gassed with atmospheric air (nominally $CO_2$-free); pH was adjusted to 7.4 or 6.8 at 37°C. All solutions contained 5 mM probenecid to inhibit cellular extrusion of BCECF by the organic anion transporter.

*Antibodies*

Primary antibodies against the following proteins were purchased from Cell Signaling Technology (MA, USA): Akt (#9272, diluted 1:200), Ser473 phospho-Akt (#4060, diluted 1:200), Thr172 phospho-AMPKα (#2535, diluted 1:1000), ERK1/2 (#9102, diluted 1:200), Thr202+Tyr204/Thr185+Tyr187 phospho-ERK1/2 (#9101, diluted 1:200), Tyr1221+1222 phospho-ErbB2 (#2243, diluted 1:200), and PARP-1 (#9542, diluted 1:500). Anti-NHE1 (#sc-136239, diluted 1:500) and anti-MCT4 (#sc-50329, diluted 1:2000) antibodies were from Santa Cruz Biotechnology (TX, USA), anti-MCT1 (#AB3538P, diluted 1:500) from Millipore (Denmark), anti-β-actin (#A5441, diluted 1:5,000) from Sigma-Aldrich (Denmark), anti-$p150^{Glued}$ (#610473, diluted 1:1,000) from BD Transduction Laboratories (Denmark) and anti-CK-19 (#ab15463, diluted 1:100), anti-α-SMA (#ab112022, 1:200), anti-Ki-67 (#ab16667, diluted 1:50) and anti-$pHH_3$ (#ab5176, diluted 1:800) from Abcam (UK). The anti-NBCn1 antibody, generously provided by Dr. Jeppe Praetorius, Aarhus University, was diluted 1:600, and the anti-AMPKα1 antibody (43), kindly donated by Dr. Grahame Hardie, University of Dundee, was diluted 1:10,000. Anti-mouse (#A1293, diluted 5,000), anti-rabbit (#A3937, diluted 1:5,000) and anti-sheep (#A5187, diluted 1:5,000) alkaline phosphatase-conjugated secondary antibodies were from Sigma-Aldrich. The horseradish peroxidase conjugated anti-rabbit secondary antibody (#7074, diluted 1:1000 (for Ki-67) or 1:2000 (for $pHH_3$)) was from Cell Signaling Technology and the Alexa Fluor® 488 coupled goat anti-rabbit secondary antibody (#A-11008) from Life Technologies.

*Immunoblotting*

Organoids isolated from primary breast cancer and matched normal breast tissue were snap frozen in liquid $N_2$, stored at -80°C and lysed. Protein concentrations were determined for each sample with the RC DC protein assay (Bio-Rad Laboratories, Denmark). Concentrations of extracted protein were equalized with double-distilled $H_2O$ and samples mixed with NuPAGE LDS Sample Buffer (Invitrogen, #NP0007) and dithiothreitol (DTT). Equal amounts of protein were loaded onto each lane of precast NuPAGE 10% Bis-Tris gels (NOVEX by Life Technologies, #NP0302BOX), separated by sodium dodecyl sulphate-polyacrylamide gel electrophoresis (SDS-PAGE) under denaturing and reducing conditions using a NOVEX system and NuPAGE MOPS SDS Running Buffer (NOVEX by Life Technologies, #NP0001). Benchmark protein ladder (Invitrogen, #10747-012) was used for identification of protein sizes. The separated proteins were transferred to nitrocellulose membranes (Invitrogen, #LC2000) in an XCel II Blot Module (Invitrogen, #EI9051) using NuPAGE Transfer Buffer (NOVEX by Life Technologies, #NP0006). The nitrocellulose membranes were stained with Ponceau S Solution (Sigma-Aldrich, #P7170-1L), blocked in blocking buffer (5% nonfat dry milk in TBST: 0.01 M Tris/HCl, 0.15 M NaCl, 0.1% Tween 20) for 1 hour at 37°C and incubated overnight at 4°C with primary antibodies diluted in blocking buffer. The membranes were washed 1 × 15 minutes and 3 × 5 minutes in TBST and incubated for 1-2 hours at room temperature with alkaline phosphatase-conjugated secondary antibodies diluted in blocking buffer. The membranes were washed in TBST and developed using BCIP/NBT Phosphatase Substrate (KPL, MD, USA; #50-81-08). The membranes were scanned and the intensity of the bands quantified using UN-SCAN-IT 6.1 (Silk Scientific, UT, USA). Individual band intensities were normalized to the average level for that membrane and expressed relative to the mean expression level for the normal breast tissue from WT mice.

*Immunohistochemistry and whole-mount immunofluorescence*

Mitotic activity was evaluated based on immunohistochemical detection of Ki-67 and $pHH_3$ expression. Excised breast tissue was fixed in neutral-buffered 4% formaldehyde for 30-60 minutes, paraffin-embedded and cut to 3 μm thick sections. Sections were heated at 80°C for 1 hour, then deparaffinized in xylene and rehydrated by graded alcohol rinses. Endogenous peroxidase activity was blocked by incubation in 3% $H_2O_2$ for 20 minutes. Heat-induced antigen retrieval was performed by microwave heating at 600 W in 10 mM citrate buffer (pH 6.0) for 2 × 10 minutes followed by blocking of non-specific sites with 1% bovine serum albumin (Ki-67) or 10% goat serum ($pHH_3$) in PBS. Following

incubation with primary antibodies (diluted in PBS with 1% bovine serum albumin and 0.1% Triton X-100) for 24 hours (Ki-67) or overnight ($pHH_3$) at 5°C, the preparations were washed vigorously and incubated with species-matched secondary antibodies for 60 minutes at room temperature. Color development was achieved using 3,3'-diaminobenzidine (DAB; DAKO, Denmark), and slides were counterstained with hematoxylin. Ki-67 and $pHH_3$ labeling indices were calculated as the fraction of cells positive for Ki-67 and $pHH_3$, respectively. Only glandular structures were evaluated in the normal breast tissue. Cell counts were obtained using ImageJ software (Rasband; NIH, MD, USA) by investigators blinded for NBCn1 genotype.
Expression of CK-19 and $\alpha$-SMA was evaluated by whole-mount immunofluorescence imaging.

*Statistics*

Data are expressed as mean$\pm$SEM, unless otherwise specified. Single variables were compared between groups by unpaired two-tailed Student's *t*-test. One-sample *t*-tests were performed to determine if the means of single distributions were different from hypothetical values. We used regular or repeated measures two-way ANOVA followed by Bonferroni post-tests to evaluate the effects of two variables (e.g., carcinogenesis and NBCn1 genotype) on the measured variable. Survival curves were compared by Gehan-Breslow-Wilcoxon test. Linear relationships (typically after log-transformation) were studied using least-squares linear regression analyses. A probability value (*P*-value) smaller than 0.05 was considered statistically significant; n equals number of mice. Statistical analyses were performed using GraphPad Prism 5.02 software.

Results

We investigated susceptibility of NBCn1 KO mice to breast cancer development after subcutaneous implantation of medroxyprogesterone acetate (MPA) pellets and treatment with 7,12-dimethylbenz($\alpha$)anthracene (DMBA) by gavage.

*KO of NBCn1 delays breast cancer development and inhibits tumor growth*

After completion of the chemical induction procedure, wild type (WT) mice developed breast cancer with an average latency of 83$\pm$8 days (Figure 1A). In NBCn1 KO mice, tumor latency was increased to 125$\pm$9 days (Figure 1A, $P<0.01$, unpaired two-tailed Student's *t*-test). At the end of the observation period, when the mice were 312 days old, a similar proportion of NBCn1 KO and WT mice had developed detectable breast tumors (Figure 1A). We evaluated growth rates of breast tumors by determining tumor burden 14 days after first tumor detection (Figure 1B). The lower detection limit by palpation is tumors 3-4 mm in diameter and is not expected to depend on NBCn1 genotype. By this approach, tumor growth rate was found ~65% lower in NBCn1 KO than WT mice (Figure 1B). Two mice were censored and excluded from further analysis because they developed lymphomas (Figure 2G) before breast tumors were detectable.

*Breast tumors in NBCn1 KO mice are histopathologically distinct from tumors in WT mice*

The histopathology of breast tumors developed in NBCn1 KO mice was more diverse than in WT mice (Figure 2A). All breast tumors detected in WT mice were squamous carcinomas (Figure 2B), adenosquamous carcinomas (Figure 2C) or Wnt type tumors (Figure 2D) whereas these tumor subtypes made up around 80% of breast tumors in NBCn1 KO mice (Figure 2A). The remaining breast tumors in NBCn1 KO mice were adenomyoepitheliomas (Figure 2E) or adenocarcinomas (Figure 2F). Histology and anti-CD45 immunohistochemical staining of the developed lymphomas are shown in Figure 2G.

To determine whether the effects of NBCn1 KO on tumor development and growth correlated with histopathology, we calculated for each histopathological tumor subtype an aggressiveness score defined as the ratio between tumor burden (at day 14 after first tumor detection) and tumor latency. As shown in Figure 2H, NBCn1 KO reduced the aggressiveness score for the three tumor types observed in both WT and NBCn1 KO mice ($P<0.001$, two-way ANOVA), and the additional tumor types observed only in NBCn1 KO mice had low aggressiveness scores (Figure 2H). These findings suggest that NBCn1 KO inhibits tumor aggressiveness also after correction for histopathology.

*KO of NBCn1 inhibits cellular net acid extrusion and causes intracellular acidification*

We investigated the mechanisms of net acid extrusion using organoids freshly isolated from primary breast cancer and matched normal breast tissue. As shown in Figure 3A, breast organoids were dominated by cells positive for the epithelial cell marker cytokeratin (CK)19 and showed low abundance of cells positive for the myofibroblast marker smooth muscle $\alpha$-actin (a-SMA). The average $pH_i$ recordings shown in Figure 3B illustrate that cellular net acid extrusion in organoids from breast cancer tissue was largely $Na^+$-dependent both in the presence (88$\pm$4% in WT and 92$\pm$3% in

NBCn1 KO, n=8) and in the absence (94±4% in WT and 101±5% in NBCn1 KO, n=8-9) of $CO_2/HCO_3^-$.

Intracellular intrinsic buffering capacity was increased in organoids from breast cancer compared to normal breast tissue but not systematically affected by NBCn1 KO (Figure 3C). Based on $pH_i$ recovery rates and intracellular buffering capacities, we calculated net $Na^+$-dependent acid-base transport activities as functions of $pH_i$ (Figure 3D,E). Cellular net acid extrusion increased approximately linearly when $pH_i$ was decreased in both normal and malignant breast tissue (Figure 3D,E). In breast cancer tissue from WT mice, net acid extrusion was strongly $CO_2/HCO_3^-$-dependent as demonstrated by high transport activities at alkaline $pH_i$ (i.e., above 7.2) only in the presence of $CO_2/HCO_3^-$ (Figure 3D). This $CO_2/HCO_3^-$-dependent transport activity was not observed in breast cancer tissue from NBCn1 KO mice (Figure 3E) nor in normal breast tissue from WT or NBCn1 KO mice (Figure 3D,E) where net acid extrusion was almost entirely $CO_2/HCO_3^-$-independent. In the absence of $CO_2/HCO_3^-$, net acid extrusion was very similar in normal breast tissue and breast cancer tissue whether from WT or NBCn1 KO mice (Figure 3D,E). These findings demonstrate that NBCn1 was responsible for the upregulated net acid extrusion in the breast cancer tissue whereas no major change in $Na^+/H^+$-exchange activity occurred during breast carcinogenesis.

Steady-state $pH_i$ of cancer tissue can be surprisingly high even in an acidic environment, often resulting in an inverted pH gradient (i.e., with $pH_i>pH_o$) across the plasma membrane. Indeed, we observed that $pH_i$ in breast cancer organoids from WT mice was higher than the pH (7.4) of the bath solution when investigated in the presence of $CO_2/HCO_3^-$ ($pH_i$=7.62±0.09; *P*<0.05 *vs.* 7.4, one-sample *t*-test). Furthermore, steady-state $pH_i$ in the presence of $CO_2/HCO_3^-$ was lower in breast cancer tissue from NBCn1 KO than WT mice (Figure 3F) demonstrating a critical role for NBCn1 in steady-state $pH_i$ control. In accordance, whereas steady-state $pH_i$ in breast cancer organoids from WT mice decreased dramatically (~0.4 units) upon omission of $CO_2/HCO_3^-$, steady-state $pH_i$ in breast cancer organoids from NBCn1 KO mice was similar in the presence and absence of $CO_2/HCO_3^-$ (Figure 3F). When $pH_o$ was reduced to 6.8, the differences in steady-state $pH_i$ between breast cancer organoids from WT and NBCn1 KO mice and their patterns of $CO_2/HCO_3^-$-dependency persisted (Figure 3F). Steady-state $pH_i$ in the normal breast tissue was lower than in the breast cancer tissue (Figure 3F) and-as expected for most normal cells-was below the pH of the bath solution (for WT: $pH_i$=7.20±0.03, for NBCn1 KO: $pH_i$=7.26±0.02, for both: *P*<0.001 *vs.* 7.4, one-sample *t*-tests). Also, in contrast to the $CO_2/HCO_3^-$-dependency of $pH_i$ in breast cancer tissue from WT mice, steady-state $pH_i$ in normal breast tissue from both WT and NBCn1 KO mice was unaffected by omission of $CO_2/HCO_3^-$ (Figure 3F).

*NBCn1 is strongly upregulated during breast carcinogenesis*

In WT mice, upregulation of $Na^+,HCO_3^-$-cotransport during breast carcinogenesis was associated with a 2.5-fold higher NBCn1 protein expression in organoids from breast cancer tissue compared to normal breast tissue (Figure 4A, left panel). No correlation was observed between NBCn1 expression level and tumor size (Figure 4A, right panel), suggesting that the increase in NBCn1 expression is an early event in carcinogenesis. Consistent with the lack of effect of carcinogenesis on the rate of net $Na^+$-dependent acid extrusion in the absence of $CO_2/HCO_3^-$ (Figure 3D,E), protein expression of the $Na^+/H^+$-exchanger NHE1 (Slc9a1) was similar in organoids from breast cancer and normal breast tissue whether from WT or NBCn1 KO mice (Figure 4B).

Protein expression of monocarboxylate transporters MCT1 (Slc16a1) and MCT4 (Slc16a3) was substantially upregulated during breast carcinogenesis (Figure 4C,D) consistent with the known roles of monocarboxylate transporters in elimination of intracellular lactate generated through glycolytic metabolism. Upregulation of MCT1 expression was smaller in tumors from NBCn1 KO than WT mice (Figure 4C, left panel) whereas expression of MCT4 was not significantly different in tumors from NBCn1 KO and WT mice (Figure 4D, left panel). These differences between MCT1 and MCT4 expression were reflected in their relation to tumor size: while MCT1 expression increased as a function of tumor size (Figure 4C, right panel), expression of MCT4 was tumor size independent (Figure 4D, right panel).

*Glycolytic metabolism is reduced in breast cancer of NBCn1 KO mice*

To assess tumor metabolism *in vivo,* we implanted microdialysis probes in breast tumors and matched normal breast tissue in the same mice. In breast tumors of WT mice, we found reduced concentrations of glucose ([glucose]) and increased concentrations of lactate ([lactate]) compared to normal breast tissue (Figure 5A,B) consistent with accelerated glycolytic metabolism. In breast tumors of NBCn1 KO mice, [glucose] was higher and [lactate] lower than in breast tumors of WT mice (Figure 5A,B). Combining these findings, we find that the [lactate]/[glucose] ratio is lower in breast tumors of NBCn1 KO than WT mice (Figure 5C, left panel) supporting that glycolysis in breast tumors of NBCn1 KO mice is inhibited compared to tumors of WT mice. The [lactate]/[glucose] ratio increased as a function of increasing breast tumor size (Figure 5C, right panel) consistent with higher flow of glucose to lactate in medium- and larger-sized breast tumors relative to small breast tumors. We observed no differences in the concentrations of pyruvate ([pyruvate]) or glycerol ([glycerol]) between breast tumors and matched normal breast tissue or between tissue from NBCn1 KO and WT mice (Figure 5D,E).

*Cell signaling is modified by breast carcinogenesis and NBCn1 KO*

Intracellular signaling is fundamentally altered in many types of cancer. We tested here whether tumors from NBCn1 KO mice differed from tumors from WT mice in their intracellular signaling profile. Overexpression of an N-terminally truncated ErbB2 receptor has previously been shown to cause expressional upregulation of NBCn1 in cultured MCF-7 human breast cancer cells; and using isolated organoids, we find that the level of phosphorylated ErbB2 is increased in breast cancer tissue compared to matched normal breast tissue (Figure 6A, left panel). No difference in ErbB2 phosphorylation was observed between breast tumors from NBCn1 KO and WT mice (Figure 6A, left panel) and the level of ErbB2 phosphorylation was independent of tumor size (Figure 6A, right panel).

The cleaved fraction of poly ADP-ribose polymerase (PARP)-1 is a measure of programmed cell death. Consistent with increased apoptosis in the harsh tumor microenvironment, it was found that proteolytic PARP-1 cleavage was increased in breast cancer tissue compared to normal breast tissue (Figure 6B, left panel). The cleaved fraction of PARP-1 was lower in tumors from NBCn1 KO than WT mice (Figure 6B, left panel) and increased as a function of tumor size (Figure 6B, right panel). AMPK, AKT and Erk1/2 signaling is prominent in many types of cancer where it is thought to link energy sensing to e.g., cell metabolism, proliferation and cell death. AMPK phosphorylation was increased in breast tumor tissue compared to normal breast tissue but did not differ between NBCn1 KO and WT mice (Figure 6C, left panel). No correlation between the level of AMPK phosphorylation and tumor size was observed (Figure 6C, right panel). AKT phosphorylation was reduced in breast tumors from WT mice compared to normal breast tissue (Figure 6D, left panel). This reduction in AKT phosphorylation did not occur during breast carcinogenesis in NBCn1 KO mice (Figure 6D, left panel), however, when AKT phosphorylation was plotted as a function of tumor volume, a significant negative correlation between tumor size and AKT phosphorylation was observed independently of NBCn1 genotype (Figure 6D, right panel). In WT mice, ERK1 phosphorylation was decreased in breast cancer tissue compared to normal breast tissue, whereas no significant change in ERK2 phosphorylation was seen (Figure 6E). The level of ERK1 phosphorylation in the breast cancer tissue decreased when tumor size increased independently of NBCn1 genotype (Figure 6E, second panel). No correlation between tumor volume and ERK2 phosphorylation was seen (Figure 6E, fourth panel).

*Cell proliferation is reduced in breast cancer of NBCn1 KO compared to WT mice*

To assess the proliferative activity in breast cancer and normal breast tissue, we performed immunohistochemical staining for Ki-67 and Ser10 phosphorylated histone $H_3$ ($pHH_3$). Representative images are shown in Figure 7A (Ki-67) and 7B ($pHH_3$). The fraction of cells positive for Ki-67 increased substantially during breast carcinogenesis (Figure 7C) whereas the fraction of cells positive for $pHH_3$ was similar in breast cancer and normal breast tissue (Figure 7D). In the smallest breast tumors (<100 $mm^3$) from NBCn1 KO mice, the Ki-67 and $pHH_3$ labeling indices were highly variable, whereas they were consistently low in medium- to large-sized breast tumors (Figure 7E). Due to tumor size heterogeneity between WT and NBCn1 KO mice, the overall Ki-67 and $pHH_3$ labeling indices did not differ between breast cancer tissue from NBCn1 KO and WT mice (Figure 7C and D). However, in the range of tumor sizes covered by both genotypes (i.e., in the size interval between the smallest breast tumor from WT mice and the largest breast tumor from NBCn1 KO mice; shaded area in Figure 7E, n=8-10), the fraction of cells positive for Ki-67 (3.5±0.8% in tumors from NBCn1 KO compared to 8.2±1.5% in tumors from WT mice) and $pHH_3$ (0.12±0.05% in tumors from NBCn1 KO compared to 0.36±0.11% in tumors from WT mice) was reduced by approximately 60% (Figure 7E).

Taken together, the data demonstrate that NBCn1 KO inhibits cell proliferation when tumor size is taken into account (Figure 7E). Since the effect of NBCn1 KO is most noticeable in medium- to large-sized breast tumors (Figure 7E), which have higher glycolytic activities (Figure 5C, right panel) and consequent higher rates of intracellular acid loading, our data are consistent with the interpretation that NBCn1 is required for neutralizing acidic waste products from tumor metabolism.

Discussion

The present example demonstrates a causal link between NBCn1 and breast cancer development and growth. The data show that disruption of NBCn1 expression results in a 50% longer tumor latency period and a 65% reduction in breast cancer growth rate. In breast cancer tissue from NBCn1 KO mice, $CO_2/HCO_3^-$-dependent net acid extrusion is abolished (Figure 3D,E), steady-state $pH_i$ is lower (Figure 3F), glycolytic activity is inhibited (Figure 5) and cell proliferation is reduced (Figure 7) compared to breast cancer tissue from WT mice. Although histopathologically different from typical human breast carcinomas (Figure 2), the breast cancer tissue in the investigated murine model accurately resembles human breast cancer tissue in terms of acid-base transporter expression, $Na^+,HCO_3^-$-cotransport dependency and steady-state $pH_i$ regulation.

These results illustrate the potential of inhibiting breast cancer development by targeting cancer cell metabolism and/or acidic waste product elimination. Previously, the $Na^+/H^+$-exchanger NHE1 has been mentioned in this connection.

NHE1 is important for $pH_i$ regulation in many cancer types (Loo SY, Chang MK, Chua CS, Kumar AP, Pervaiz S, Clement MV. NHE-1: a promising target for novel anti-cancer therapeutics. Curr. Pharm. Des 2012;18:1372-1382), but does not play a predominant role for overall $pH_i$ regulation in the current murine breast cancer model (Figure 3) or in human breast cancer biopsies. In human and murine (Figure 3) breast cancer tissue, $Na^+/H^+$-exchange activity apparently contributes to global $pH_i$ regulation mainly at very acidic $pH_i$ levels. At neutral $pH_i$ conditions, NHE1 may still play a role for pH control in local restricted intracellular or extracellular spaces or have transport-independent effects such as cytoskeletal anchoring.

By plotting protein expression levels and metabolic values as functions of tumor volume (Figure 4-7), we take into account that functional and expressional changes may occur during breast cancer progression. Due to the relation between breast cancer histopathology and tumor growth rate (Figure 2H), tumor volume becomes in part a surrogate measure for histopathology. Nevertheless, a distinct pattern arises where expression of some proteins (e.g., NHE1 and pERK2) is unaltered in cancer compared to normal tissue, whereas the expression of other proteins changes during breast carcinogenesis and is either unrelated (e.g., NBCn1, MCT-4, pErbB2 and pAMPK), positively related (MCT-1 and cleaved PARP-1) or negatively related (pAKT and pERK1) to tumor volume; Figure 4 and 6). These findings imply that while expression of some proteins (e.g., NBCn1) changes early in carcinogenesis, the change in expression of other proteins occurs later when the tumors start expanding. The upregulation of NBCn1 early in carcinogenesis, however, supports its essential role in breast cancer development.

NBCn1 expression in cultured MCF-7 breast cancer cells can be upregulated by enhanced signaling downstream of ErbB2 receptors. This mechanism may also contribute to NBCn1 upregulation in the current model of breast carcinogenesis because ErbB2 phosphorylation was increased in breast cancer compared to normal breast tissue (Figure 6A). Consistent with this notion, the levels of ErbB2 phosphorylation and NBCn1 expression were both independent of tumor volume (Figure 4A and 6A, right panels). Still, the pronounced effect of NBCn1 inhibition across multiple histopathological subtypes (Figure 2) supports a general importance of NBCn1 in breast carcinogenesis as described for human breast cancer tissue where upregulated NBCn1 expression and $Na^+,HCO_3^-$-cotransport activity are not restricted to tumors with particular receptor (i.e., HER2/ErbB2 or estrogen) expression profiles. Further supporting that $Na^+,HCO_3^-$-cotransport is important across multiple cancer types and conditions, a recent study found comparable levels of $Na^+$-dependent $HCO_3^-$-transport in multiple cultured cancer cell lines irrespective of oxygenation status (Hulikova A, Harris AL, Vaughan-Jones RD, Swietach P. Regulation of intracellular pH in cancer cell lines under normoxia and hypoxia. J Cell Physiol 2013;228:743-752.).

Tumor cell proliferative activity was assessed by immunohistochemical staining for Ki-67 and $pHH_3$ (Figure 7). The Ki-67 and $pHH_3$ labeling indices correlate inversely with prognosis for human cancer patients. Thus, the lower Ki-67 and $pHH_3$ labeling indices in tumors from NBCn1 KO mice support the implications of NBCn1 inhibition for breast cancer development and progression. It is well-accepted that glycolytic conversion of glucose to lactic acid increases with tumor size (Figure 5). Based on the effect of NBCn1 KO on proliferation particularly in medium- to larger-sized tumors (Figure 7E), it appears that NBCn1 could permit rapid tumor growth by facilitating acidic waste product elimination in tumors relying on high glycolytic activity.

The difference in histopathology between breast tumors from WT and NBCn1 KO mice is intriguing. The slower cancer development and growth (Figure 1) and lower proliferative activity (Figure 7E) in NBCn1 KO mice could provide time for less aggressive tumor types to develop. These less aggressive tumor types (e.g., adenomyoepitheliomas) would typically not be seen in WT mice because earlier development of more aggressive tumor types results in early euthanasia.

In conclusion, the present example shows that NBCn1 is causally related to breast cancer development. Genetic disruption of NBCn1 prolongs breast tumor latency, slows tumor growth and inhibits tumor cell proliferation. In congruence, NBCn1 mediates the upregulated cellular net acid extrusion in breast cancer tissue and maintains the alkaline steady-state $pH_i$ and inverted pH gradient across the plasma membrane. Also, compared to breast tumors in WT mice, the metabolic profile and protein expression pattern of breast tumors in NBCn1 KO mice are more similar to those of normal breast tissue. These findings establish NBCn1 as a target for anti-cancer therapy.

**Example 2**

$Na^+,HCO_3^-$-cotransporter NBCn1 increases $pH_i$ gradients, filopodia and migration of smooth muscle cells and promotes arterial remodeling

Abstract

Aims: Arterial remodeling can cause luminal narrowing and obstruct blood flow. We tested the hypothesis that cellular acid-base transport mediated by NBCn1 facilitates proliferation and migration of vascular smooth muscle cells (VSMCs) and enhances remodeling of conduit arteries.

Methods and Results: $Na^+,HCO_3^-$-cotransport via NBCn1 (Slc4a7) mediates net acid extrusion and controls steady-

state intracellular pH ($pH_i$) in VSMCs of mouse carotid arteries and primary aortic explants. Carotid arteries undergo hypertrophic inward remodeling in response to partial or complete ligation *in vivo* but the increase in media area and thickness and reduction in lumen diameter are attenuated in arteries from NBCn1 knockout compared to wild-type mice. With $CO_2/HCO_3^-$ present, gradients for $pH_i$ (~0.2 units magnitude) exist along the axis of VSMC migration in primary explants from wild-type but not NBCn1 knockout mice. Knockout or pharmacological inhibition of NBCn1 also reduces filopodia and lowers initial rates of VSMC migration after scratch-wound infliction. Interventions to reduce $H^+$-buffer mobility (omission of $CO_2/HCO_3^-$ or inhibition of carbonic anhydrases) reestablish axial $pH_i$ gradients, filopodia, and migration rates in explants from NBCn1 knockout mice. Omission of $CO_2/HCO_3^-$ also lowers global $pH_i$ and inhibits proliferation in primary explants.
Conclusions: Under physiological conditions (i.e., with $CO_2/HCO_3^-$ present), NBCn1-mediated $HCO_3^-$ uptake raises VSMC $pH_i$ and promotes filopodia, VSMC migration, and hypertrophic inward remodeling. Probabaly axial $pH_i$ gradients enhance VSMC migration whereas global acidification inhibits VSMC proliferation and media hypertrophy after carotid artery ligation. These findings support a key role of NBCn1 mediated acid-base transport in development of occlusive artery disease.

Background

Occlusive artery disease is a leading cause of human morbidity and mortality. Remodeling of conduit arteries typically occurs in response to altered hemodynamic forces. Although inward remodeling may serve important homeostatic functions, it can limit blood flow and contribute to ischemia.
Structural remodeling of arteries requires cell migration and possibly hyperplasia or hypertrophy. Ion transport across the plasma membrane modifies cell migration, proliferation, and growth. In addition to cell volume-which is controlled by multiple ion transport mechanisms-acid-base transporters regulate intracellular pH ($pH_i$) and local extracellular pH ($pH_o$). Recent studies suggest that local pH can change cell-matrix interactions (e.g., due to modifications of integrin binding), extracellular matrix degradation (e.g., by activation of matrix metalloproteinases), cytoskeletal dynamics (e.g., through cofilin-dependent changes in actin polymerization), and focal adhesion remodeling (e.g., due to changes in talin-actin interactions). As a result, local acid-base status may fundamentally modify tissue structure.
Investigations based on cultured non-vascular cells support that the ubiquitous $Na^+/H^+$-exchanger NHE1 (Slc9a1) promotes cell division and migration. NHE1-dependent $pH_o$ and $pH_i$ gradients along the axis of migration and predominant NHE1 expression at the leading edge of motile cells have been demonstrated. Studies of pulmonary and systemic resistance arteries and vascular smooth muscle cells (VSMCs) support the relevance of these findings for structural development and remodeling of the vascular wall. Yet, because effects of NHE1 on cell migration and cytoskeletal arrangement are at least partly independent of ion translocation, it remains controversial to what extent NHE1 modifies tissue structure via changes in pH. It should also be noted that most previous studies proposing a predominant role of NHE1 for subcellular pH regulation and cell migration were performed in absence of $CO_2/HCO_3^-$ where effects of $Na^+/H^+$-exchange will likely be overestimated.
In the wall of mouse mesenteric, coronary, and cerebral resistance arteries, omission of $CO_2/HCO_3^-$ has dramatic effects on $pH_i$. With $CO_2/HCO_3^-$ present, $Na^+,HCO_3^-$-cotransport mediated via NBCn1 (Slc4a7) dominates net acid extrusion in the near-neutral $pH_i$ range, whereas $Na^+/H^+$-exchange activity is important for acid extrusion primarily during pronounced intracellular acidification. Molecular mechanisms of global $pH_i$ regulation have been studied in detail for VSMCs, but mechanisms of pH regulation in subcellular compartments and local restricted spaces remain to be investigated. Although there is evidence for NBCn1 expression in rat and mouse aortas, the significance of NBCn1 for conduit artery function and structure is unresolved. NHE1 and NBCn1 mediate corresponding acid-base transport functions when the $CO_2/HCO_3^-$ buffer is equilibrated but it is still unclear whether they share activation patterns, play comparable roles for local pH regulation, and contribute similarly to cell signaling. Other than providing substrate for acid-base transporters, $CO_2$ and $HCO_3^-$ are important intracellular and extracellular buffers that increase the apparent $H^+$ mobility. It is plausible that $CO_2/HCO_3$-dependent processes modify relative acid-base transport activities at different subcellular locations (e.g., at the leading compared to rear end of migrating cells) and additionally contribute to dissipating pH gradients by facilitating diffusion of acid-base equivalents within the intra- and extracellular compartments.
In the current study, we tested the hypothesis that local $pH_i$ in VSMCs is controlled by the interplay between NBCn1-mediated $Na^+,HCO_3^-$-cotransport and mobile $H^+$ buffers to coordinate and promote cellular migration and proliferation that are key processes in conduit artery remodeling.

Methods

NBCn1 knockout (KO) mice were generated and maintained on the 129/SvJ genetic background. The experiments conformed to guidelines from Directive 2010/63/EU of the European Parliament on protection of animals used for scientific purposes and were approved by the Danish Animal Experiments Inspectorate (2012-15-2934-00286).

*In vitro analyses*

Primary explants were produced from thoracic aortas and common carotid arteries of NBCn1 KO and wild-type (WT) mice. To best maintain the cell morphology and phenotype of native VSMCs, cultured cells were never passaged but investigated directly in primary explants.
Using wide-field fluorescence microscopy, global $pH_i$ was recorded in arteries and primary explants loaded with the pH-sensitive fluorophore BCECF. Recordings of $pH_i$ in subcellular domains were performed by confocal fluorescence microscopy of cells loaded with the pH-sensitive fluorophore carboxy SNARF-1. Intracellular buffering capacities were calculated from changes in $pH_i$ upon washout of $NH_4Cl$. No difference in total intracellular buffering capacity (β) was seen between primary explants from NBCn1 KO and WT mice ($\beta_{WT}$=15±2 mM, $\beta_{KO}$=17±2 mM, n=8; P=0.45, unpaired two-tailed Student's *t*-test) evaluated at $pH_i$ 6.65-6.80.
Expression of transcripts coding for $Na^+$,$HCO_3^-$-cotransporters was investigated by reverse transcription and polymerase chain reaction. Experiments without addition of reverse transcriptase were performed to control for genomic amplification. Rates of wound healing in primary explants were calculated from time-lapse images captured at 5-minute intervals after scratch infliction. Experiments were performed in Minimum Essential Medium with 10% fetal calf serum or-to avoid binding of the non-selective $Na^+$,$HCO_3^-$-cotransport inhibitor S0859 to medium or serum components-in physiological saline solutions (PSS). Cell size was estimated based on cell density in confluent areas. Total filopodia length was determined relative to the length of the migrating front (i.e., twice the scratch length). Cell proliferation was evaluated (a) by counting the relative number of cells dividing during six hours time-lapse imaging and (b) as the proportion of cells positive for the thymidine analogue bromodeoxyuridine (BrdU) after six hours incubation.
The PSS was composed of (in mM) 134 $Na^+$, 4 $K^+$, 1.6 $Ca^{2+}$, 1.2 $Mg^{2+}$, 111 $Cl^-$, 24 $HCO_3^-$, 1.2 $SO_4^{2-}$, 1.18 $H_2PO_4^-$, 10 HEPES, 5.5 glucose, 0.03 EDTA. In $Na^+$-free solutions, $Na^+$ was substituted with an equimolar amount of *N*-methyl-D-glucammonium ($NMDG^+$), except for $NaHCO_3$, which was replaced with choline-$HCO_3$. In $HCO_3^-$-free solutions, $HCO_3^-$ was replaced with an equimolar amount of $Cl^-$. $HCO_3^-$-containing solutions were aerated with a gas mixture of 5% $CO_2$/balance air, whereas $HCO_3^-$-free solutions were gassed with air (i.e., 21% $O_2$/balance $N_2$). All solutions were titrated to pH 7.4 at 37°C.

*In vivo experiments*

NBCn1 KO and WT mice were anaesthetized with isoflurane and the right common carotid artery dissected free of surrounding tissue. A ligature was placed around the common carotid artery near the bifurcation (complete ligation) or around the internal and external carotid arteries above the occipital artery but below the superior thyroid artery (partial ligation). In sham-operated animals, the carotid artery was surgically isolated but no ligature was placed. The degree of remodeling was analyzed four weeks after the surgical procedure.

*Statistics*

Data are given as mean±SEM; n equals number of mice. $P<0.05$ was considered statistically significant. Single variables were compared between two groups by paired or unpaired two-tailed Student's *t*-test and between more than two groups by one-way ANOVA followed by Bonferroni post-tests. Regular or repeated measures two-way ANOVA was performed to compare effects of two variables on a third variable and followed by Bonferroni post-tests. Statistical tests were performed using Microsoft Excel 2010 or GraphPad Prism 5.02 software.

Results

*Global $pH_i$ and Slc4 transcripts in conduit arteries*

We evaluated $pH_i$ regulatory function after intracellular acid loading with the $NH_4^+$-prepulse technique. Washout of $NH_4Cl$ caused intracellular acidification followed by gradual recovery towards resting $pH_i$ levels (Figure 8A,B). Acid-base transporters are regulated by $pH_i$, and to allow for meaningful comparison of transporter activities under different experimental conditions, we plotted $pH_i$ recovery rates as function of $pH_i$ (Figure 8C). $Na^+$,$HCO_3^-$-cotransport activity was prominent in VSMCs of carotid arteries from WT mice: the $pH_i$ recovery rate from intracellular acidification increased as function of decreasing $pH_i$ and was higher in presence than absence of $CO_2$/$HCO_3^-$ when compared at equivalent $pH_i$ levels (Figure 8A-C). All experiments were performed at $pH_o$ 7.4. The critical role of $Na^+$,$HCO_3^-$-cotransport for $pH_i$ regulation in VSMCs of mouse conduit arteries is consistent with previous results from rat and mouse resistance arteries. As shown in Supplemental Figure 8, we detected mRNA transcripts for the $Na^+$,$HCO_3^-$-cotransporters NBCn1, NBCe1 (Slc4a4), NDCBE (Slc4a8) and BTR1 (Slc4a11, weak bands) in segments of aorta, carotid and mesenteric arteries of 129/SvJ WT mice. NBCn1 mRNA transcripts were absent in arteries from NBCn1 KO mice whereas expression of the other Slc4-family transporters appeared unaltered or increased (Figure 16).
Despite expression of transcripts for multiple $Na^+$,$HCO_3^-$-cotransporters, NBCn1 functionally predominated net acid extrusion during intracellular acidification as evidenced by the abolished $Na^+$,$HCO_3^-$-cotransport activity in carotid arteries from NBCn1 KO mice (Figure 8A-C). Omission of $CO_2$/$HCO_3^-$ strongly attenuated the $Na^+$-dependent $pH_i$ recovery rate from intracellular acidification in carotid arteries from WT mice but had no effect on the $pH_i$ recovery rate in carotid arteries from NBCn1 KO mice (Figure 8A-C) even at low $pH_i$ where the contribution from $CO_2$/$HCO_3^-$ to intracellular buffering is minor.
Steady-state $pH_i$ in carotid arteries depended on NBCn1-mediated $HCO_3^-$-transport (Figure 8D): in presence of $CO_2$/$HCO_3^-$ at $pH_o$ 7.4, steady-state $pH_i$ of VSMCs was -0.3 lower in arteries from NBCn1 KO than WT mice (Figure 8A,D). In the absence of $CO_2$/$HCO_3^-$-with $pH_o$ maintained at 7.4-we observed no difference in steady-state $pH_i$ between carotid arteries from NBCn1 KO and WT mice (Figure 8B,D).
Taken together, these findings show that NBCn1 is responsible for the $Na^+$,$HCO_3^-$-cotransport and required for normal $pH_i$ regulation in VSMCs of carotid arteries.

*Remodeling of carotid arteries*

We studied morphological changes of carotid arteries in response to altered shear stress *in vivo* following complete or partial unilateral ligation. Compared to the complete ligation model-where carotid blood flow is completely obstructed-the partial ligation model employed here results in -90% reduction in carotid blood flow and limits thrombosis and endothelial cell denudation. In 129/SvJ WT mice, significant hypertrophic inward remodeling was observed after ligation (Figure 9). Representative images are shown in Figure 9A. The reduction in lumen diameter (Figure 9B) and increase in media thickness (Figure 9C) and area (Figure 9D) were all attenuated in carotid arteries from NBCn1 KO compared to WT mice. Whereas non-ligated arteries had very little adherent adventitia, artery ligation caused significant adventitial thickening (Figure 9A). The adventitial cross-sectional area was larger after partial than complete ligation but did not differ between arteries from NBCn1 KO and WT mice (Figure 9E). Thus, the consequences of NBCn1 KO for arterial structure were confined to the media. The remodeling responses observed after complete or partial ligation were equivalent in nature (Figure 9). These findings demonstrate that NBCn1 plays a central role for structural adaptations in the arterial wall in response to hemodynamic disturbances under *in vivo* conditions.

*Global $pH_i$ and Slc4 transcripts in primary VSMCs*

To study the contribution of NBCn1 to cellular proliferation and migration, we next produced primary explants of VSMCs from aortas of NBCn1 KO and WT mice. We reduced phenotypic effects of cell culture by performing all subsequent analyses directly on primary explants without subcultivation. The mRNA expression profile for $Na^+$,$HCO_3^-$-cotransporters in primary explants from NBCn1 KO and WT mice largely resembled the profile in isolated arteries (Figure 15).
$Na^+$,$HCO_3^-$-cotransport activity-evaluated as the amiloride-insensitive, $Na^+$-dependent $pH_i$ recovery rate-contributed substantially to $pH_i$ regulation in primary explants from WT mice (Figure 10A). In explants from NBCn1 KO mice, $Na^+$,$HCO_3^-$-cotransport activity was ~60% lower (Figure 10A). Consistent with our previous findings from mesenteric arteries, $Na^+/H^+$-exchange activity-evaluated as the $Na^+$-dependent, $CO_2$/$HCO_3^-$-independent $pH_i$ recovery rate-was ~30% higher in explants from NBCn1 KO compared to WT mice (Figure 10B). In mesenteric arteries, the increase in $Na^+/H^+$-exchange activity occurs without changes in NHE1 expression.
Global steady-state $pH_i$ was only marginally reduced (by less than 0.1) in explants from NBCn1 KO compared to WT mice, reaching statistical significance only in paired tests where experiments were matched by experimental day (Figure 10C). In contrast, omission of $CO_2$/$HCO_3^-$ reduced steady-state $pH_i$ substantially in explants from both NBCn1 KO and WT mice (Figure 10C).
We previously showed that the non-selective $Na^+$,$HCO_3^-$-cotransport inhibitor S0859 does not affect cellular net acid extrusion in isolated arteries. Because S0859 inhibits $Na^+$,$HCO_3^-$-cotransport in cultures of MCF7 breast cancer cells, has no effect in breast cancer slices or freshly isolated breast cancer organoids, and shows unusually strong protein binding, we concluded that the inability of S0859 to inhibit $Na^+$,$HCO_3^-$-cotransport in arteries is explained by poor penetration into tissue preparations. Consistent with this interpretation, we show here that addition of S0859 concentration-dependently attenuates $Na^+$,$HCO_3^-$-cotransport activity (Figure 10D) and steady-state $pH_i$ (Figure 10E) in primary aortic explants. The effect of 5 $\mu$M S0859 on global $Na^+$,$HCO_3^-$-cotransport activity (Figure 10D) and steady-state $pH_i$ (Figure 10E) was roughly comparable to the difference between explants from WT and NBCn1 KO mice (Figure 10A,C).
Together these findings demonstrate that NBCn1 plays a prominent-although not exclusive-role for $Na^+$,$HCO_3^-$-cotransport in primary explants and that $Na^+/H^+$-exchange activity is upregulated in explants from NBCn1 KO mice.

*Wound healing*

We studied VSMC migration in primary aortic and carotid explants after scratch infliction. Time-lapse movies were made to show the wound healing process and representative images of the wound healing process are shown in Figure 11A. VSMCs from NBCn1 KO mice healed scratch-wounds slower than VSMCs from WT mice when studied in $CO_2/HCO_3^-$-containing serum-supplemented medium (Figure 4B,C) or PSS
(Figure 11D). The initial rate of wound healing was reduced by 40-60% in explants from NBCn1 KO compared to WT mice when investigated in presence of $CO_2/HCO_3^-$ (Figure 11B-D), whereas no effect of NBCn1 KO was observed on the rate of wound healing in absence of $CO_2/HCO_3$- (Figure 11F,G). Here and in subsequent experiments, $pH_o$ was kept constant at 7.4 when switching between different buffer solutions. Application of 5 μM S0859 inhibited wound healing in the presence of $CO_2/HCO_3$- (Figure 11D) but not in its nominal absence (Figure 11G). These findings demonstrate a key role of $Na^+,HCO_3^-$-cotransport via NBCn1 for wound healing.
We have previously shown that KO of NBCn1 inhibits rho-kinase-dependent signaling in VSMCs of mouse resistance arteries. A number of studies have suggested that rho-kinase activity enhances cell migration, although there are also studies showing an inverse relationship between rho-kinase activity and cell migration. In primary aortic explants, application of 10 μM rho-kinase inhibitor Y-27632 stimulated wound healing without affecting the relative difference between NBCn1 KO and WT mice (Figure 11E) supporting that altered rho-kinase activity does not explain the slower rate of wound healing in explants from NBCn1 KO mice.

*Proliferation of isolated VSMCs*

In addition to altered migration, a change in proliferative activity can alter the rate of wound healing. We investigated proliferation of VSMCs based on (a) the number of cell divisions observed in time-lapse recordings and (b) incorporation of thymidine-analogue BrdU.

Based on time-lapse imaging, we found a similar proportion of VSMCs from NBCn1 KO and WT mice undergoing mitosis during six hours observation in serum-containing culture medium (Figure 12A,B). Although the relative number of mitoses was reduced when VSMCs were investigated in PSS, we still saw no effect of NBCn1 KO or 5 μM S0859 under these conditions (Figure 12A). The unaltered proliferative activity of VSMCs from NBCn1 KO mice *in vitro* was confirmed by BrdU incorporation experiments: the relative number of cells containing recently synthesized DNA material after six hours incubation was not significantly different between explants from NBCn1 KO and WT mice (Figure 12C). As expected, the relative number of BrdU-positive cells after a six-hour incubation was approximately twice the relative number of cells undergoing mitosis during the same time period (Figure 12A,C). In contrast to the similar proliferative activities in explants from NBCn1 KO and WT mice and in the presence of 5 μM S0859, VSMC proliferation was significantly lower ($P<0.01$, two-way ANOVA) when explants were investigated in absence than presence of $CO_2/HCO_3^-$ (Figure 12A).
It is generally proposed that an alkaline global $pH_i$ stimulates cell proliferation and growth. When effects of NBCn1 KO, S0859, and $CO_2/HCO_3^-$ omission on VSMC proliferation (Figure 12A) and media hypertrophy (Figure 9D) were related to the global $pH_i$ measured in primary explants (Figure 10C,F) and arteries (Figure 8D), respectively, we found a significant positive correlation (Figure 12D) supporting that low global $pH_i$ inhibits VSMC proliferation.

*Cell density in primary explants*

Cell size is relevant for tissue structure and related to VSMC phenotype. Furthermore, due to its role in ion translocation, NBCn1 may contribute to local and/or global volume regulation. We measured average projected cell areas based on cell densities in confluent areas of primary explants investigated in serum-supplemented medium. As shown in Figure 12E, no significant difference was seen in projected cell areas between aortic explants from NBCn1 KO and WT mice.

*VSMC cell death*

Increased cell death would provide an alternative explanation for the decelerated wound healing in explants from NBCn1 KO mice and in explants treated with S0859. However, even after prolonged periods of investigation, cell integrity and morphology were typically well-maintained in aortic explants from NBCn1 KO and WT mice (Figure 12F) and during treatment with 5 μM S0859 (Figure 12G). The number of cells with normal morphology after 18 hours of time-lapse imaging relative to immediately after scratch infliction was also similar ($P$=0.40; unpaired, two-tailed Student's $t$-test) in carotid explants from NBCn1 KO (91±3%, n=11) and WT mice (96±4%, n=8). In contrast, treatment with 30 μM S0859 caused severe morphological changes over a period of 18 hours (Figure 12G and Supplemental Figure 9). Importantly, the morphological effects of 30 μM S0859 were independent of $HCO_3^-$ transport, since similar effects were observed in the absence of $CO_2/HCO_3^-$ (Figure 12H). These findings show that 30 $\mu$M S0859 has effects on viability, which are not explained by actions on $Na^+,HCO_3^-$-cotransport, whereas inhibition of $Na^+,HCO_3^-$-cotransport does not affect cell survival under the conditions of the assay.

*Local pH regulation in migrating VSMCs*

Migrating primary VSMCs were characterized by cell protrusions extending into the scratch-wound (Figure 13A). The presence of filopodia was substantially reduced in explants from NBCn1 KO compared to WT mice when investigated in serum-supplemented medium (Figure 13A,B) or PSS (Figure 13C) containing $CO_2/HCO_3^-$. When applied to $CO_2/HCO_3^-$-containing PSS, 5 $\mu$M S0859 reduced the presence of filopodia in explants from WT mice (Figure 13C). Under $CO_2/HCO_3^-$-free conditions, the presence of filopodia was similar in VSMCs from NBCn1 KO and WT mice and unaffected by 5 $\mu$M S0859 (Figure 13A,C).
Previous studies from non-vascular cell types propose that increased acid extrusion at the leading edge of migrating cells creates an axial $pH_i$ gradient, which can facilitate actin polymerization, filopodia formation, and cell migration. To investigate pH gradients within cells, we measured $pH_i$-differences between the tip of filopodia and the general (perinuclear) cytosol two hours after scratch infliction. These experiments-performed in PSS-confirmed that whereas VSMCs from WT mice showed a similar abundance of filopodia in presence (Figure 13D, first panel) and absence (Figure 13D, second panel) of $CO_2/HCO_3^-$, VSMCs from NBCn1 KO mice had substantially more or longer filopodia when incubated in absence than presence of $CO_2/HCO_3^-$ (Figure 13D, two right panels). With the intention of investigating local $pH_i$ dynamics in the region of the filopodia, we incubated primary explants in absence of $CO_2/HCO_3^-$ for two hours after wound infliction to allow for filopodia formation. We then investigated $pH_i$ after an additional 15 minutes with or without $CO_2/HCO_3^-$ or in the presence of pharmacological inhibitors (Figure 13E,F). In explants from WT mice, $pH_i$ at the tip of filopodia was -0.2 higher than in the perinuclear cytosol (Figure 13F). In presence of $CO_2/HCO_3^-$, this $pH_i$ gradient was absent in explants from NBCn1 KO mice (Figure 13E,F). Addition of 5 $\mu$M S0859 strongly acidified the migrating VSMCs and in particular shifted $pH_i$ at the tip of filopodia to very low levels (Figure 13F). Taken together, these findings support that under physiological conditions $Na^+,HCO_3^-$-cotransport via NBCn1 is required for establishing axial $pH_i$ gradients that facilitate filopodia formation.
In absence of $CO_2/HCO_3^-$, we detected axial $pH_i$ gradients in explants from both NBCn1 KO and WT mice (Figure 13F). This finding suggests that $CO_2/HCO_3^-$-independent acid-base transport can establish $pH_i$ gradients in VSMCs but only under artificial conditions without $CO_2/HCO_3^-$. We hypothesized that lower concentrations of mobile intracellular buffers in absence of $CO_2/HCO_3^-$ reduce the effective intracellular $H^+$ mobility and thereby permit $CO_2/HCO_3^-$-independent acid-base transporters to establish $pH_i$ gradients. Consistent with this hypothesis, we found that 100 $\mu$M of the cell-permeable carbonic anhydrase inhibitor acetazolamide-which has been shown to reduce the apparent intracellular $H^+$ mobility in cardiomyocytes-reestablishes axial $pH_i$ gradients in explants from NBCn1 KO mice in presence of $CO_2/HCO_3^-$ (Figure 13F).

*Role of carbonic anhydrases in cell migration and filopodia*

To test whether $pH_i$ gradients in primary aortic explants facilitate migration, we next studied the effects of acetazolamide on wound healing in explants from NBCn1 KO mice. As shown in Figure 14A, 100 $\mu$M acetazolamide increased the initial rate of wound healing. This effect is also illustrated in Figure 14B and is of a magnitude approximately similar to the difference between aortic explants from NBCn1 KO and WT mice in presence of $CO_2/HCO_3^-$ (Figure 11A,B). The effect of acetazolamide on wound healing in aortic explants from NBCn1 KO mice was paralleled by an increased abundance of filopodia (Figure 14C). The ability of acetazolamide to reestablish axial $pH_i$ gradients as well as filopodia and migration rates strongly supports their interdependency.

*Correlation between $pH_i$ gradients and cell migration*

To further investigate the importance of the axial $pH_i$ gradient, we plotted rates of wound healing as function of the size of the corresponding $pH_i$ gradients. We reduced variation between different experimental series by expressing rates of wound healing relative to explants from NBCn1 KO mice in presence of $CO_2/HCO_3^-$. As shown in Figure 14D, there was a strong correlation between the axial $pH_i$ gradient and the initial rate of wound healing.

Discussion

We show here that $Na^+,HCO_3^-$-cotransport via NBCn1 facilitates migration of VSMCs (Figure 11) and remodeling of conduit arteries in response to reduced shear stress (Figure 9). We demonstrate for the first time that migrating primary VSMCs display $pH_i$ gradients along their axis of translocation, and we corroborate that these axial $pH_i$ gradients promote

filopodia formation and migration (Figure 13,14).

Regulation of $pH_i$ is typically considered a homeostatic mechanism with the overall aim of maintaining constant $pH_i$ despite changes in metabolism and extracellular acid-base status. The current study supports that $pH_i$ changes can also serve signaling purposes and thus contribute to dynamic cell responses. By establishing $pH_i$ gradients between the leading and rear end of migrating cells, NBCn1 likely coordinates the activity of pH sensitive proteins (e.g., talin and cofilin) that facilitate cell migration by modulating actin polymerization and focal adhesions. Although $pH_i$ can fundamentally alter the function of almost all cellular proteins-and thus could appear too general for signaling purposes-the spatial restriction of $pH_i$ demonstrated in the current study (Figure 13E,F) allows for coordinated control of protein function specifically in a given region of the cell.

Whereas the role of $Na^+$,$HCO_3^-$-cotransporters for cell migration has been controversial, several studies-based primarily on non-vascular cells investigated without $CO_2$/$HCO_3^-$-have implicated $Na^+$/$H^+$-exchanger NHE1 in cell migration. Because vascular cells rely heavily on $CO_2$/$HCO_3^-$-dependent mechanisms, the use of $CO_2$/$HCO_3^-$-containing solutions is critical for studies of $pH_i$ regulation and its associated functional effects in the vascular system. In the current study, we investigated primary aortic and carotid artery explants. During cell culture, VSMCs are known to undergo phenotypic shift from a contractile (typical for healthy arteries) to a synthetic or proliferative phenotype that can also be observed during vascular remodeling and in atherosclerotic plaques. Our results suggest that $CO_2$/$HCO_3^-$ modifies local VSMC $pH_i$ by (a) changing relative acid-base transport activities at different subcellular locations and (b) increasing buffer mobility-and hence the effective intracellular $H^+$ mobility-which can dissipate $pH_i$ gradients (Figure 13F). We propose that spatial differences in local expression and/or activity of acid-base transporters and carbonic anhydrases-relative to the associated cytosolic volume-contribute to the dynamic formation and dissipation of pH gradients. It is possible that metabolic acid production also varies spatially: in endothelial cells, for instance, filopodia are rich in glycolytic enzymes but devoid of mitochondria. This compartmentalization of metabolism and the associated production of acidic waste products may contribute to the formation of axial $pH_i$ gradients.

It has been discussed whether $Na^+$/$H^+$-exchangers affect cell migration due to transport of acid-base equivalents or as a result of transport-independent functions (e.g., changes in intracellular signaling cascades or binding to cytoskeletal or extracellular matrix components). Since NBCn1 and NHE1 perform equivalent transport functions but are unlikely to mediate the same transport-independent effects, our finding that NBCn1 contributes to cell migration supports a role for transport of acid-base equivalents. The importance of acid-base transport is further supported by the inability of NBCn1 KO and 5 $\mu$M S0859 to interfere with cell migration in absence of $CO_2$/$HCO_3^-$ (Figure 11F,G) when the transport function of NBCn1 is already inhibited. Still, because interventions that interfere with acid-base transport activity may alter the ability of the transporters to link (e.g., via ezrin-radixin-moesin proteins) to the cytoskeleton, it cannot be excluded that disrupted binding to interacting proteins may contribute to the impaired cell migration after inhibition of NBCn1.

We show here that NBCn1 KO reduces media hypertrophy following carotid artery ligation *in vivo* (Figure 9D). In apparent contrast, NBCn1 KO did not affect cell proliferation or cell size *in vitro* (Figure 12A-E). This dissimilarity between *in vivo* and *in vitro* conditions may reflect limitations of 2-dimensional culture conditions compared to the complex 3-dimensional structure of the arterial wall, differences in the release of chemokines and growth factors caused by scratch wounds vs. altered flow conditions or the absence vs. presence of interactions with other cell types (e.g., immune cells). However, the larger NBCn1-dependent difference in global steady-state $pH_i$ (Figure 8D *vs.* 10C) and $Na^+$,$HCO_3^-$-cotransport activity (Figure 8C *vs.* 10A) for carotid arteries compared to primary explants suggests that functional upregulation of other $Na^+$,$HCO_3^-$-cotransporters during cell culture can account for the difference in NBCn1-dependent hypertrophy and proliferation. In congruence, omission of $CO_2$/$HCO_3^-$ and consequent inhibition all $HCO_3^-$-dependent transport mechanisms in primary explants produced a greater $pH_i$ decrease than KO of NBCn1 (Figure 10C) and moreover inhibited VSMC proliferation *in vitro* (Figure 12A). Together, our observations in the arterial wall as well as in primary explants show significant correlation between global VSMC $pH_i$, cell proliferation, and media hypertrophy (Figure 12D). This finding is in agreement with the recent finding that KO of NBCn1 inhibits cancer cell proliferation in primary breast carcinomas. Although it is difficult to extrapolate from 2-dimensional culture conditions to *in vivo* circumstances-and quantitative comparisons are at best speculative-the phenomenological congruence between our *in vivo* and *in vitro* observations supports a causative link between the decelerating effect of NBCn1 KO on wound healing *in vitro* and the reduced remodeling *in vivo.*

Pharmacological agents that selectively inhibit $Na^+$,$HCO_3^-$-cotransporters have been lacking, and although S0859 provides advantages compared to previously employed compounds (such as 4,4-diisothiocyanatostilbene-2,2'-disulphonate, DIDS), the current study shows that S0859 should be used with caution. We see convincing $CO_2$/$HCO_3^-$-dependency of S0859 only in the low-$\mu$M range. At higher concentrations, deleterious effects of S0859 on cell morphology and survival (Figure 12G,H) preclude meaningful conclusions regarding integrated cellular consequences of inhibiting $Na^+$,$HCO_3^-$-cotransport. The concentration required for complete inhibition of $Na^+$,$HCO_3^-$-cotransport is close to the concentration causing cell damage (compare Figure 10D,E and Figure 12G,H). These findings and previous evidence that S0859 binds to plasma and presumably extracellular connective tissue components limit the applicability of the compound to carefully controlled *in vitro* investigations of isolated cells.

In conclusion, we find that $Na^+,HCO_3^-$-cotransport mediated via NBCn1 plays a central role for VSMC acid extrusion and steady-state $pH_i$ control in mouse conduit arteries. In particular, we demonstrate that NBCn1 creates $pH_i$ gradients along the axis of migration, facilitates migration of VSMCs, and promotes remodeling of carotid arteries in response to altered shear stress. These findings provide new mechanistic insights on how transmembrane acid-base transport can modify remodeling of conduit arteries and identify NBCn1 as a novel target for occlusive artery disease, such as atherosclerosis and/or restenosis.

**Example 3**

Methods

*Development of polyclonal antibodies*

Based on predicted antigenic determinants in extracellular loop 3 of human NBCn1 (Figure 17), we selected two overlapping peptides covering the regions of high estimated antigenicity: NBCn1_EL3h1.1: [Hz]-HNNLDKLTSYSCVCTEPPNPSNETLAQWKKDNITA-amide (SEQ ID NO: 14) and NBCn1_EL3h2.1: [Hz]-LAQWKKDNITAHNISWRNLTVSECKKLRGVFLGSA-amide (SEQ ID NO: 15).
The hydrazine (Hz)-labelled peptides were synthesized and keyhole limpet hemocyanin (KLH)-conjugated by 21st Century Biochemicals (MA, USA). The KLH-conjugated peptides in Complete Freund's Adjuvant were each injected into two specific pathogen-free New Zealand rabbits. The rabbits were boosted through subsequent injections of the same KLH-conjugated peptide in Incomplete Freund's Adjuvant after 2, 4, 6 and 8 weeks. Serum was collected from blood sampled before first immunization (pre-immune serum) and at week 7, 8, 10 and 11.

*Binding assays and affinity purification*

The collected serum samples were tested for binding to the synthesized peptides conjugated to bovine serum albumin (BSA) based on enzyme-linked immunosorbent assay (ELISA) analysis. An anti-BSA antibody (A11133, Life Technologies) was used as positive control and pre-immune serum served as negative control. Flat-bottomed 96-well cell culture plates (734-2327, VWR) were coated overnight at 4°C with BSA-conjugated peptide (diluted to 2 μg/mL in phosphate buffered saline (PBS); 60 μL per well) and then blocked with 15% fetal bovine serum (FBS; #S0115, Biochrom AG; 75 μL per well) in PBS for 1 hour at 37°C. Between each step, the plates were washed 4 times with 0.05% Tween-20 in PBS. Sera and anti-BSA antibody were diluted in PBS, added to wells in duplicates (50 μL per well), and incubated for 1 hour at 37°C. After washing, the plates were incubated with horseradish peroxidase-conjugated secondary goat anti-rabbit IgG (#70745, Cell Signalling; 50 μL per well) for 1 hour at 37°C. After further washing, the plates were developed by incubation with the horseradish peroxidase substrate 3,3',5,5'-tetramethylbenzidine (002023, Life Technologies; 50 μL per well) for 30 minutes at 37°C. Reactions were stopped by addition of 100 μL 1 M $H_2SO_4$ to each well. Absorbances were measured at 450 nm on a PowerWave 340 microplate spectrophotometer (BioTek) using 620 nm readings as reference.
Sera with the highest titers were selected for affinity purification: we used pools of all 4 bleeds collected from rabbits injected with NBCn1_EL3h1.1 (B1-B4 in Figure 18A) and pools of the last two bleeds collected from rabbits injected with NBCn1_EL3h2.1 (B3 and B4 in Figure 18B). Affinity purification was performed by 21st Century Biochemicals using the synthetic peptides immobilized on optimized affinity resin. Lyophilized antibodies were stored at -20°C until use.

*Functional testing of affinity-purified antibodies*

The affinity-purified antibodies were tested functionally as inhibitors of $Na^+,HCO_3^-$-cotransport in MCF-7 human breast cancer cells. Because net acid extrusion from MCF-7 cells occurs through a combination of NBCn1-mediated $Na^+,HCO_3^-$-cotransport and NHE1-mediated $Na^+/H^+$-exchange (Lauritzen et al., 2010), we evaluated the degree of $Na^+,HCO_3^-$-cotransport inhibition by comparing the effect of the antibodies to that of the non-selective $Na^+,HCO_3^-$-cotransport inhibitor S0859 (Steinkamp et al., 2015, Larsen et al., 2012). Regulation of intracellular pH ($pH_i$) was also investigated in the absence of $CO_2/HCO_3^-$.
MCF-7 cells (Lauritzen et al., 2010) were grown in RPMI-1640 medium (#61870-010, Gibco) supplemented with 6% FBS (#S0115, Biochrom AG), 1% penicillin/streptomycin (#15140-122, Gibco), 1 μg/mL puromycin (#P9620, Sigma), and 200 μg/mL G418 (#G8168, Sigma). Cells were maintained at 37°C in an atmosphere of 5% $CO_2$ and used at passages 15-21. MCF-7 cells were seeded on cover glasses coated for 2 hours with 0.2% autoclaved gelatin (#G2625, Sigma) and investigated after 2-3 days. MCF-7 cells were loaded with 3 μM BCECF-AM (Invitrogen) for 20-30 minutes at 37°C. The preparations were mounted in a custom-built chamber on the stage of an Olympus IX70 or Nikon Diaphot 200 microscope and excited alternately at approximately 490 nm and 440 nm. Emission light was collected at 510 nm with CCD-based fluorescence imaging systems controlled through EasyRatioPro (Photon Technology International, NJ, USA) or VisiView (Visitron Systems, Germany) software.

Intracellular acidification was induced by the $NH_4^+$-prepulse technique (Boron and De Weer, 1976): 20 mM $NH_4Cl$ was added to the experimental bath solution for 15 minutes before it was washed out to a $Na^+$-free solution in order to first evaluate the rate of $Na^+$-independent net acid extrusion. Then, $Na^+$ was returned to the bath solution to activate $Na^+$-dependent net acid extrusion mechanisms. The BCECF fluorescence ratios were taken as a measure of $pH_i$. $Na^+$-dependent $pH_i$ recovery rates calculated over 30-second intervals were evaluated based on the initial increase in $pH_i$ recovery rate after addition of extracellular $Na^+$ and expressed relative to control conditions without antibody or S0859.

The affinity-purified anti-NBCn1 antibodies were solubilized in distilled water and added to the experimental solutions (at 1:100 dilution) from the time of $NH_4Cl$ addition until the end of the experiment. S0859 was solubilized in DMSO and added to the bath solution at a concentration of 30 $\mu$M from the time of $NH_4Cl$ washout until the end of the experiment. The final [DMSO] in the bath was kept below 0.1%.

The physiological saline solution used for $pH_i$ recordings contained (in mM): 140 $Na^+$, 4 $K^+$, 1.6 $Ca^{2+}$, 1.2 $Mg^{2+}$, 122 $Cl^-$, 24 $HCO_3^-$, 1.2 $SO_4^{2-}$, 1.18 $H_2PO_4^-$, 10 HEPES, 5.5 glucose, 0.03 EDTA. In $CO_2/HCO_3^-$-free solutions, $HCO_3^-$ was replaced with equimolar amounts of $Cl^-$. In $Na^+$-free solutions, $Na^+$ was replaced with equimolar amounts of N-methyl-D-glucammonium; except for $NaHCO_3$, which was replaced with choline-$HCO_3$. $CO_2/HCO_3^-$-containing solutions were aerated with 5% $CO_2$/balance air, whereas $CO_2/HCO_3^-$-free solutions were bubbled with nominally $CO_2$-free air. All solutions were adjusted to pH 7.4 at 37°C. Solutions contained 5 mM probenecid to inhibit BCECF extrusion by the organic anion transporter.

*Statistics*

Data are expressed as mean $\pm$ SEM, n equals number of experiments. Data were compared by one-way ANOVA followed by Dunnett's post-test or by unpaired, two-tailed Student's *t*-test. *P*<0.05 was considered statistically significant. Statistical analyses were performed using GraphPad Prism 5.02 software.

Results

Sera from rabbits immunized with the KLH-conjugated peptides (corresponding to fragments of the extracellular loop 3 of human NBCn1, see Figure 17) showed strong titers against the equivalent BSA-conjugated peptides (Figure 18).

*Biological testing for $Na^+$,$HCO_3^-$-cotransport inhibitory function*

Extracellular application of 20 mM $NH_4Cl$ to MCF-7 human breast cancer cells initially caused abrupt intracellular alkalinization (due to fast influx of $NH_3$) followed by gradual $pH_i$ recovery towards resting levels (due to $NH_4^+$ uptake and activation of base extrusion mechanisms; Figure 19A,B). Upon washout of $NH_4Cl$ from the experimental bath solution, $NH_3$ rapidly leaves the cells shifting the $NH_4^+ \rightleftarrows NH_3 + H^+$ equilibrium to the right, liberating $H^+$ and causing the desired intracellular acidification. We tested the ability of the MCF-7 cells to activate net acid extrusion mechanisms and recover $pH_i$ from this intracellular acidification in the presence and absence of the affinity-purified anti-NBCn1_EL3h1.1 and anti-NBCn1_EL3h2.1 polyclonal antibodies.

As shown in Figure 19A, we first evaluated the rate of $Na^+$-independent $pH_i$ recovery (under $Na^+$-free extracellular conditions) and then the increase in $pH_i$ recovery rate after $Na^+$ was returned to the experimental bath solution. The anti-NBCn1_EL3h1.1 and anti-NBCn1_EL3h2.1 antibodies both inhibited $Na^+$-dependent net acid extrusion from MCF-7 human breast cancer cells in the presence of $CO_2/HCO_3^-$ (Figure 19A,C). Most notably, the inhibition achieved with the affinity-purified anti-NBCn1_EL3h2.1 antibody (Figure 19A,C) was of similar magnitude to that observed with 30 $\mu$M S0859 (Figure 19B,D; see also (Larsen et al., 2012, Lauritzen et al., 2010, Steinkamp et al., 2015)) or following siRNA-mediated knockdown of NBCn1 (Lauritzen et al., 2010). Omission of $CO_2/HCO_3^-$ under control conditions reduced the $Na^+$-dependent $pH_i$ recovery rate to the level seen with the anti-NBCn1_EL3h2.1 antibody in the presence of $CO_2/HCO_3^-$ (Figure 19C). This was also the case at low $pH_i$ where $CO_2/HCO_3^-$ contributes only marginally to intracellular buffering. Taken together, these findings are consistent with the anti-NBCn1_EL3h2.1 antibody completely inhibiting NBCn1-mediated $Na^+$,$HCO_3^-$-cotransport in MCF-7 cells.

The remaining net acid extrusion after inhibition of $Na^+$,$HCO_3^-$-cotransport in MCF-7 cells has previously been shown to be due to $Na^+/H^+$-exchange, as it is inhibited by the $Na^+/H^+$-exchange inhibitor 5-(*N*-Ethyl-*N*-isopropyl)amiloride or by siRNA-mediated knockdown of NHE1 (Lauritzen et al., 2010). In agreement, no effect of the anti-NBCn1_EL3h2.1 antibody was seen on the $Na^+$-dependent $pH_i$ recovery rate in the absence of $CO_2/HCO_3^-$ (Figure 19C).

Discussion

We developed polyclonal anti-NBCn1 antibodies against the predicted extracellular loop 3 of human NBCn1. Comparing the degree to which our newly developed anti-NBCn1 antibodies inhibit net acid extrusion to previously reported inhibitory effects of S0859 and siRNA-mediated knockdown of NBCn1 (Lauritzen et al., 2010, Larsen et al., 2012, Steinkamp et al., 2015), we conclude that the developed anti-NBCn1_EL3h2.1 antibody is able to completely inhibit NBCn1-mediated $Na^+$,$HCO_3^-$-cotransport during intracellular acidification of MCF-7 human breast cancer cells. This conclusion is further supported by the observation that omission of $CO_2/HCO_3^-$ inhibited net acid extrusion to the same relative extent as addition of the anti-NBCn1_EL3h2.1 antibody in the presence of $CO_2/HCO_3^-$. Furthermore, the anti-NBCn1_EL3h2.1 antibody did not affect $Na^+/H^+$-exchange activity evaluated as the $Na^+$-dependent $pH_i$ recovery rate in the absence of $CO_2/HCO_3^-$.
In conclusion, our data demonstrate for the first time that it is possible to generate antibodies against extracellular loop 3 of human NBCn1 and that these antibodies have the capacity to fully inhibit NBCn1-mediated $Na^+$,$HCO_3^-$-cotransport. Considering the poor selectivity of S0859 (Heidtmann et al., 2015) (Figure 12) and the inability of S0859 to inhibit $Na^+$,$HCO_3^-$-cotransport in tissue preparations (Steinkamp et al., 2015, Larsen et al., 2012), the development of inhibitory antibodies against NBCn1 opens up new therapeutic possibilities.

**Reference List**

Boron, W. F. & De Weer, P. 1976. Intracellular pH transients in squid giant axons caused by CO2, NH3, and metabolic inhibitors. J Gen Physiol, 67, 91-112.

Heidtmann, H., Ruminot, I., Becker, H. M. & Deitmer, J. W. 2015. Inhibition of monocarboxylate transporter by N-cyanosulphonamide S0859. Eur J Pharmacol, 762, 344-349.

Larsen, A. M., Krogsgaard-Larsen, N., Lauritzen, G., Olesen, C. W., Honoré Hansen, S., Boedtkjer, E., Pedersen, S. F. & Bunch, L. 2012. Gram-scale solution-phase synthesis of selective sodium bicarbonate co-transport inhibitor S0859: in vitro efficacy studies in breast cancer cells. ChemMedChem, 7, 1808-1814.

Lauritzen, G., Jensen, M. B., Boedtkjer, E., Dybboe, R., Aalkjaer, C., Nylandsted, J. & Pedersen, S. F. 2010. NBCn1 and NHE1 expression and activity in ΔNErbB2 receptor-expressing MCF-7 breast cancer cells: Contributions to pHi regulation and chemotherapy resistance. Exp Cell Res, 316, 2538-2553.

Steinkamp, A.-D., Seling, N., Lee, S., Boedtkjer, E. & Bolm, C. 2015. Synthesis of N-cyano-substituted sulfilimine and sulfoximine derivatives of S0859 and their biological evaluation as sodium bicarbonate co-transport inhibitors. MedChemComm, 6, 2163-2169.

**Sequences**

SEQ ID NO: 1

Human NBCn1 DNA sequence variant a cloned from breast cancer tissue. The underlined 5'-region indicates a region, which differs from SEG ID NO: 2.

atggaaagatttcgtctggagaagaagttacctggtcctgatgaagaagctgttgtggatcttggcaaaactagctcaact
gtgaacaccaagtttgaaaaagaagaactagaaagtcatagagctgtatatattggtgttcacgtcccgtttagtaaagag
agtcgtcggcgtcataggcatcgcggacacaaacatcaccaccggagaagaaaagataaagaatcagataaagaa
gatggacgggaatctccttcttatgatacaccatcccagagagttcagtttatccttggtactgaagatgatgatgaagaac
atattccccatgatctcttcacggaaatggatgaactgtgttacagagatggagaagaatatgaatggaaagaaactgct
agatggctgaaatttgaagaggatgttgaagatggcggtgaccgatggagtaaaccttatgtggcaactctctctttgcac
agtctttttgaactaaggagttgcatcctcaatggaacagtcatgctggatatgagagcaagcactctagatgaaatagca
gatatggtattagacaacatgatagcttctggccaattagacgagtccatacgagagaatgtcagagaagctcttctgaa
gagacatcatcatcagaatgagaaaagattcaccagtcggattcctcttgttcgatcttttgcagatataggcaagaaacat
tctgaccctcacttgcttgaaaggaatggtattttggcctctccccagtctgctcctggaaacttggacaatagtaaaagtgg
agaaattaaaggtaatggaagtggtggaagcagagaaaatagtactgttgacttcagcaaggttgatatgaatttcatga
gaaaaattcctacgggtgctgaggcatccaacgtcctggtgggcgaagtagactttttggaaaggccaataattgcatttgt
gagactggctcctgctgtcctccttacagggttgactgaggtccctgttccaaccaggtttttgtttttgttattgggtccagcgg
gcaaggcaccacagtaccatgaaattggacgatcaatagccactctcatgacagatgagattttccatgatgtagcttata
aagcaaaagacagaaatgacctcttatctggaattgatgaatttttagatcaagtaactgtcctacctccaggagagtggg
atccttctatacgcatagaaccaccaaaaagtgtcccttctcaggaaaagagaaagattcctgtgtttcacaatggatctac
ccccacactgggtgagactcctaaagaggccgctcatcatgctgggcctgagctacagaggactggacggctttttggtg
gtttgatacttgacatcaaaaggaaagcaccttttttcttgagtgacttcaaggatgcattaagcctgcagtgcctggcctcg
attcttttcctatactgtgcctgtatgtctcctgtaatcacttttggagggctgcttggagaagctacagaaggcagaataagtg
caatagagtctctttttggagcatcattaactgggattgcctattcattgtttgctgggcaacctctaacaatattggggagcac
aggtccagttctagtgtttgaaaaaattttatataaattctgcagagattatcaactttcttatctgtctttaagaaccagtattggt
ctgtggacttctttttgtgcattgttttggttgcaacagatgcaagcagccttgtgtgttatattactcgatttacagaagaggctt
ttgcagcccttatttgcatcatattcatctacgaggctttggagaagctctttgatttaggagaaacatatgcatttaatatgcac
aacaacttagataaactgaccagctactcatgtgtatgtactgaacctccaaaccccagcaatgaaactctagcacaatg
gaagaaagataatataacagcacacaatatttcctggagaaatcttactgtttctgaatgtaaaaaacttcgtggtgtattctt
ggggtcagcttgtggtcatcatggaccttatattccagatgtgctcttttggtgtgtcatcttgtttttcacaacatttttctgtcttca
ttcctcaagcaatttaagaccaagcgttactttcctaccaaggtgcgatcgacaatcagtgattttgctgtatttctcacaatag
taataatggttacaattgactaccttgtaggagttccatctcctaaacttcatgttcctgaaaaatttgagcctactcatccaga
gagagggtggatcataagcccactgggagataatccttggtggaccttattaatagctgctattcctgctttgctttgtaccatt

ctcatctttatggatcaacaaatcacagctgtaattataaacagaaaggaacacaaattgaagaaaggagctggctatc
accttgatttgctcatggttggcgttatgttgggagtttgctctgtcatgggacttccatggtttgtggctgcaacagtgttgtcaat
aagtcatgtcaacagcttaaaagttgaatctgaatgttctgctccaggggaacaacccaagtttttgggaattcgtgaacag
cgggttacagggctaatgatttttattctaatgggcctctctgtgttcatgacttcagtcctaaagtttattccaatgcctgttctgt
atggtgttttcctttatatgggagtttcctcattaaaaggaatccagttatttgaccgtataaaattatttggaatgcctgctaagc
atcagcctgatttgatatacctccgttatgtgccgctctggaaggtccatattttcacagtcattcagcttacttgtttggtcctttta
tgggtgataaaagtttcagctgctgcagtggtttttcccatgatggttcttgcattagtgtttgtgcgcaaactcatggacctgtgt
ttcacgaagagagaacttagttggcttgatgatcttatgccagaaagtaagaaaaagaaagaagatgacaaaaagaa
aaaagagaaagaggaagctgaacggatgcttcaagatgatgatgatactgtgcaccttccatttgaagggggaagtctc
ttgcaaattccagtcaaggccctaaaatatagtcctgataaacctgtgagtgtgaaaataagttttgaagatgaaccaaga
aagaaatacgtggatgctgaaacttcattatagaattgaaccaagaggcattatacatatagatatatacatatgtaatgtg
tgcgtatcatgtcactatatataagaatattgtatgtcatgctgtttatgtgtgactaccgggtttttaaaagtagt

SEQ ID NO: 2

Human NBCn1 DNA sequence variant b cloned from breast cancer tissue. The underlined 5'-region indicates a region, which differs from SEG ID NO: 1.

atggaggctgatggggccggcgagcagatgagaccgctactcacccggggtcctgatgaagaagctgttgtggatcttg
gcaaaactagctcaactgtgaacaccaagtttgaaaaagaagaactagaaagtcatagagctgtatatattggtgttcac
gtcccgtttagtaaagagagtcgtcggcgtcataggcatcgcggacacaaacatcaccaccggagaagaaaagataa
agaatcagataaagaagatggacgggaatctccttcttatgatacaccatcccagagagttcagtttatccttggtactgaa
gatgatgatgaagaacatattccccatgatctcttcacggaaatggatgaactgtgttacagagatggagaagaatatga
atggaaagaaactgctagatggctgaaatttgaagaggatgttgaagatggcggtgaccgatggagtaaaccttatgtg
gcaactctctctttgcacagtctttttgaactaaggagttgcatcctcaatggaacagtcatgctggatatgagagcaagca
ctctagatgaaatagcagatatggtattagacaacatgatagcttctggccaattagacgagtccatacgagagaatgtc
agagaagctcttctgaagagacatcatcatcagaatgagaaaagattcaccagtcggattcctcttgttcgatcttttgcag
atataggcaagaaacattctgaccctcacttgcttgaaaggaatggtattttggcctctccccagtctgctcctggaaacttg
gacaatagtaaaagtggagaaattaaaggtaatggaagtggtggaagcagagaaaatagtactgttgacttcagcaag
gttgatatgaatttcatgagaaaaattcctacgggtgctgaggcatccaacgtcctggtgggcgaagtagactttttggaaa
ggccaataattgcatttgtgagactggctcctgctgtcctccttacagggttgactgaggtccctgttccaaccaggtttttgtttt
tgttattgggtccagcgggcaaggcaccacagtaccatgaaattggacgatcaatagccactctcatgacagatgagatt
ttccatgatgtagcttataaagcaaaagacagaaatgacctcttatctggaattgatgaatttttagatcaagtaactgtccta
cctccaggagagtgggatccttctatacgcatagaaccaccaaaaagtgtcccttctcaggaaaagagaaagattcctgt
gtttcacaatggatctacccccacactgggtgagactcctaaagaggccgctcatcatgctgggcctgagctacagagg
actggacggctttttggtggtttgatacttgacatcaaaaggaaagcacctttttttcttgagtgacttcaaggatgcattaagcc
tgcagtgcctggcctcgattcttttcctatactgtgcctgtatgtctcctgtaatcacttttggagggctgcttggagaagctaca
gaaggcagaataagtgcaatagagtctctttttggagcatcattaactgggattgcctattcattgtttgctgggcaacctcta

acaatattggggagcacaggtccagttctagtgtttgaaaaaattttatataaattctgcagagattatcaactttcttatctgtc
tttaagaaccagtattggtctgtggacttctttttgtgcattgttttggttgcaacagatgcaagcagccttgtgtgttatattactc
gatttacagaagaggcttttgcagcccttatttgcatcatattcatctacgaggctttggagaagctctttgatttaggagaaac
atatgcatttaatatgcacaacaacttagataaactgaccagctactcatgtgtatgtactgaacctccaaaccccagcaat
gaaactctagcacaatggaagaaagataatataacagcacacaatatttcctggagaaatcttactgtttctgaatgtaaa
aaacttcgtggtgtattcttggggtcagcttgtggtcatcatggaccttatattccagatgtgctcttttggtgtgtcatcttgtttttc
acaacatttttctgtcttcattcctcaagcaatttaagaccaagcgttactttcctaccaaggtgcgatcgacaatcagtgattt
tgctgtatttctcacaatagtaataatggttacaattgactaccttgtaggagttccatctcctaaacttcatgttcctgaaaaatt
tgagcctactcatccagagagagggtggatcataagcccactgggagataatccttggtggaccttattaatagctgctatt
cctgctttgctttgtaccattctcatctttatggatcaacaaatcacagctgtaattataaacagaaaggaacacaaattgaa
gaaaggagctggctatcaccttgatttgctcatggttggcgttatgttgggagtttgctctgtcatgggacttccatggtttgtgg
ctgcaacagtgttgtcaataagtcatgtcaacagcttaaaagttgaatctgaatgttctgctccaggggaacaacccaagtt
tttgggaattcgtgaacagcgggttacagggctaatgatttttattctaatgggcctctctgtgttcatgacttcagtcctaaagtt
tattccaatgcctgttctgtatggtgttttcctttatatgggagtttcctcattaaaaggaatccagttatttgaccgtataaaattat
ttggaatgcctgctaagcatcagcctgatttgatatacctccgttatgtgccgctctggaaggtccatattttcacagtcattca
gcttacttgtttggtccttttatgggtgataaaagtttcagctgctgcagtggtttttcccatgatggttcttgcattagtgtttgtgcg
caaactcatggacctgtgtttcacgaagagagaacttagttggcttgatgatcttatgccagaaagtaagaaaaagaaag
aagatgacaaaaagaaaaaagagaaagaggaagctgaacggatgcttcaagatgatgatgatactgtgcaccttcca
tttgaagggggaagtctcttgcaaattccagtcaaggccctaaaatatagtgttgatccctcaattgttaacatatcagatga
aatggccaaaactgcacagtggaaggcactttccatgaatactgagaatgccaaagtaaccagatctaacatgagtcct
gataaacctgtgagtgtgaaaataagttttgaagatgaaccaagaaagaaatacgtggatgctgaaacttcattatagaa
ttgaaccaagaggcattatacatatagatatatacatatgtaatgtgtgcgtatcatgtcactatatataagaatattgtatgtc
atgctgtttatgtgtgactaccgggtttttaaaagtagtgtctggagtttgtaatgagcaccgtggagactatgtatttaatgaa
atgctctctttgaagtgaggtacatggttctt

SEQ ID NO: 3

Human NBCn1 Peptide sequence (variant a) cloned from breast cancer tissue. The underlined N-terminal region indicates where SEG ID NO: 3 differ from SEG ID NO: 4.

MERFRLEKKLPGPDEEAVVDLGKTSSTVNTKFEKEELESHRAVYIGVHVPFSKESRRR
HRHRGHKHHHRRRKDKESDKEDGRESPSYDTPSQRVQFILGTEDDDEEHIPHDLFTE
MDELCYRDGEEYEWKETARWLKFEEDVEDGGDRWSKPYVATLSLHSLFELRSCILN
GTVMLDMRASTLDEIADMVLDNMIASGQLDESIRENVREALLKRHHHQNEKRFTSRIP
LVRSFADIGKKHSDPHLLERNGILASPQSAPGNLDNSKSGEIKGNGSGGSRENSTVDF
SKVDMNFMRKIPTGAEASNVLVGEVDFLERPIIAFVRLAPAVLLTGLTEVPVPTRFLFLL
LGPAGKAPQYHEIGRSIATLMTDEIFHDVAYKAKDRNDLLSGIDEFLDQVTVLPPGEW
DPSIRIEPPKSVPSQEKRKIPVFHNGSTPTLGETPKEAAHHAGPELQRTGRLFGGLILDI

KRKAPFFLSDFKDALSLQCLASILFLYCACMSPVITFGGLLGEATEGRISAIESLFGASL
TGIAYSLFAGQPLTILGSTGPVLVFEKILYKFCRDYQLSYLSLRTSIGLWTSFLCIVLVAT
DASSLVCYITRFTEEAFAALICIIFIYEALEKLFDLGETYAFNMHNNLDKLTSYSCVCTEP
PNPSNETLAQWKKDNITAHNISWRNLTVSECKKLRGVFLGSACGHHGPYIPDVLFWC
VILFFTTFFLSSFLKQFKTKRYFPTKVRSTISDFAVFLTIVIMVTIDYLVGVPSPKLHVPEK
FEPTHPERGWIISPLGDNPWWTLLIAAIPALLCTILIFMDQQITAVIINRKEHKLKKGAGY
HLDLLMVGVMLGVCSVMGLPWFVAATVLSISHVNSLKVESECSAPGEQPKFLGIREQ
RVTGLMIFILMGLSVFMTSVLKFIPMPVLYGVFLYMGVSSLKGIQLFDRIKLFGMPAKH
QPDLIYLRYVPLWKVHIFTVIQLTCLVLLWVIKVSAAAVVFPMMVLALVFVRKLMDLCFT
KRELSWLDDLMPESKKKKEDDKKKKEKEEAERMLQDDDDTVHLPFEGGSLLQIPVKA
LKYSPDKPVSVKISFEDEPRKKYVDAETSL*

SEQ ID NO: 4

Human NBCn1 Peptide sequence (variant b) cloned from breast cancer tissue. The underlined N-terminal region indicates where SEG ID NO: 3 differ from SEG ID NO: 4.

MEADGAGEQMRPLLTRGPDEEAVVDLGKTSSTVNTKFEKEELESHRAVYIGVHVPFS
KESRRRHRHRGHKHHHRRRKDKESDKEDGRESPSYDTPSQRVQFILGTEDDDEEHI
PHDLFTEMDELCYRDGEEYEWKETARWLKFEEDVEDGGDRWSKPYVATLSLHSLFE
LRSCILNGTVMLDMRASTLDEIADMVLDNMIASGQLDESIRENVREALLKRHHHQNEK
RFTSRIPLVRSFADIGKKHSDPHLLERNGILASPQSAPGNLDNSKSGEIKGNGSGGSR
ENSTVDFSKVDMNFMRKIPTGAEASNVLVGEVDFLERPIIAFVRLAPAVLLTGLTEVPV
PTRFLFLLLGPAGKAPQYHEIGRSIATLMTDEIFHDVAYKAKDRNDLLSGIDEFLDQVTV
LPPGEWDPSIRIEPPKSVPSQEKRKIPVFHNGSTPTLGETPKEAAHHAGPELQRTGRL
FGGLILDIKRKAPFFLSDFKDALSLQCLASILFLYCACMSPVITFGGLLGEATEGRISAIE
SLFGASLTGIAYSLFAGQPLTILGSTGPVLVFEKILYKFCRDYQLSYLSLRTSIGLWTSFL
CIVLVATDASSLVCYITRFTEEAFAALICIIFIYEALEKLFDLGETYAFNMHNNLDKLTSYS
CVCTEPPNPSNETLAQWKKDNITAHNISWRNLTVSECKKLRGVFLGSACGHHGPYIP
DVLFWCVILFFTTFFLSSFLKQFKTKRYFPTKVRSTISDFAVFLTIVIMVTIDYLVGVPSP
KLHVPEKFEPTHPERGWIISPLGDNPWWTLLIAAIPALLCTILIFMDQQITAVIINRKEHKL
KKGAGYHLDLLMVGVMLGVCSVMGLPWFVAATVLSISHVNSLKVESECSAPGEQPKF
LGIREQRVTGLMIFILMGLSVFMTSVLKFIPMPVLYGVFLYMGVSSLKGIQLFDRIKLFG
MPAKHQPDLIYLRYVPLWKVHIFTVIQLTCLVLLWVIKVSAAAVVFPMMVLALVFVRKL
MDLCFTKRELSWLDDLMPESKKKKEDDKKKKEKEEAERMLQDDDDTVHLPFEGGSL
LQIPVKALKYSVDPSIVNISDEMAKTAQWKALSMNTENAKVTRSNMSPDKPVSVKISF
EDEPRKKYVDAETSL*

SEQ ID NO: 5

EL2, human and mouse: SPVITFGGLLGEATEGRISAIESLFGASLT

SEQ ID NO: 6

EL3, human:

KLFDLGETYAFNMHNNLDKLTSYSCVCTEPPNPSNETLAQWKKDNITAHNISWRNLTV
SECKKLRGVFLGSACGHHGP

SEQ ID NO: 7

EL3, mouse:

Unformatted residues are fully conserved, bold strongly conserved, italicized weakly conserved, and underlined without consensus between the mouse and human sequence.

KLF*H*LGEIYAFNMHNNLD**E**LTSY**T**CVC**A**EP*S*NPSNETLELWK**R***K*NITA**Y***S***V**SWGNLTV
SECK***T*FH**G**M**F**V**GSACGPHGP

SEQ ID NO: 8

EL4, human: PSPKLHVPEKFEPTHPERGWIISPLGDNPW

SEQ ID NO: 9

EL4, mouse: PSPKLHVPEKFEPTDPSRGWIISPLGDNPW

SEQ ID NO: 10

EL5, human and mouse: SISHVNSLKVESECSAPGEQPKFLGIREQR

SEQ ID NO: 11

human

MERFRLEKKLPGPDEEAVVDLGKTSSTVNTKFEKEELESHRAVYIGVHVPFSKESRRR
HRHRGHKHHHRRRKDKESDKEDGRESPSYDTPSQRVQFILGTEDDDEEHIPHDLFTE
MDELCYRDGEEYEWKETARWLKFEEDVEDGGDRWSKPYVATLSLHSLFELRSCILN
GTVMLDMRASTLDEIADMVLDNMIASGQLDESIRENVREALLKRHHHQNEKRFTSRIP
LVRSFADIGKKHSDPHLLERNGEGLSASRHSLRTGLSASNLSLRGESPLSLLLGHLLP
SSRAGTPAGSRCTTPVPTPQNSPPSSPSISRLTSRSSQESQRQAPELLVSPASDDIPT
VVIHPPEEDLEAALKGEEQKNEENVDLTPGILASPQSAPGNLDNSKSGEIKGNGSGGS
RENSTVDFSKVDMNFMRKIPTGAEASNVLVGEVDFLERPIIAFVRLAPAVLLTGLTEVP
VPTRFLFLLLGPAGKAPQYHEIGRSIATLMTDEIFHDVAYKAKDRNDLLSGIDEFLDQVT
VLPPGEWDPSIRIEPPKSVPSQEKRKIPVFHNGSTPTLGETPKEAAHHAGPELQRTGR
LFGGLILDIKRKAPFFLSDFKDALSLQC

SEQ ID NO: 12

human

ATVLSISHVNSLKVESECSAPGEQPKFLGIREQRVT

SEQ ID NO: 13

human

DRIKLFGMPAKHQPDLIYLRYVPLWKVHIFTVIQLTC

SEQ ID NO: 14

NBCn1_EL3h1.1: [Hz]-HNNLDKLTSYSCVCTEPPNPSNETLAQWKKDNITA-amide (SEQ ID NO: 14)

SEQ ID NO: 15

NBCn1_EL3h2.1: [Hz]-LAQWKKDNITAHNISWRNLTVSECKKLRGVFLGSA-amide (SEQ ID NO: 15).

## Claims

1. An antibody or antigen binding fragment thereof specifically recognizing and binding an extracellular polypeptide region of human NBCn1, wherein said extracellular polypeptide region comprises or consists of a sequence selected from any one of SEQ ID NO: 14 and 15, and wherein said antibody or antigen binding fragment thereof is capable of inhibiting cellular NBCn1-mediated Na+- dependent HCO3- transport, and wherein said antigen binding fragment is (i) a Fab fragment, a monovalent fragment consisting of the VL, VH, CL and CH1 domains; (ii) a F(ab')2 fragment, a bivalent fragment comprising two Fab fragments linked by a disulfide bridge at the hinge region; (iii) a Fd fragment consisting of the VH and CH1 domains; (iv) a Fv fragment consisting of the VL and VH domains of a single arm of an antibody or (v) a dAb fragment, which consists of a VH domain.

**2.** A method of selecting an antibody, said method comprising

a) Providing an antibody library,
b) Providing one or more antigens from an extracellular polypeptide region of human NBCn1, wherein said extracellular polypeptide region comprises or consists of a sequence selected from any one of SEQ ID NO: 14 and 15
c) Contacting said antibody library with said one or more antigens, and
d) Selecting antibodies, which bind said one or more antigens and are capable of inhibiting cellular NBCn1-mediated $Na^+$-dependent $HCO_3^-$ transport.

**3.** A method of producing an antibody specifically recognizing and binding an extracellular polypeptide region of human NBCn1, wherein said antibody is capable of inhibiting cellular NBCn1-mediated $Na^+$-dependent $HCO_3^-$ transport, said method comprising the steps of administering to an animal, preferably a mammal, an extracellular polypeptide fragment of human NBCn1 or a homolog at least 98% identical thereto, wherein said extracellular polypeptide region comprises or consists of a sequence selected from any one of SEQ ID NO: 14 and 15, isolating antibodies from said mammal, testing whether antibodies are capable of inhibiting cellular NBCn1-mediated $Na^+$-dependent $HCO_3^-$ transport and selecting antibodies capable of inhibiting cellular NBCn1-mediated $Na^+$-dependent $HCO_3^-$ transport.

**4.** An antibody specifically recognizing and binding an extracellular polypeptide region of human NBCn1, wherein said extracellular polypeptide region comprises or consists of a sequence selected from any one of SEQ ID NO: 14 and 15, and wherein said antibody is capable of inhibiting cellular NBCn1-mediated $Na^+$-dependent $HCO^{3-}$ transport for use as medicament.

**5.** The antibody according to claim 4 for use in the treatment of breast cancer, atherosclerosis and/or restenosis.

**6.** The antibody according to claim 5, wherein said breast cancer is a triple negative breast cancer, an estrogen receptor-positive breast cancers or a HER2-positive breast cancer.

**7.** The antibody according to any of the preceding claims 5 and 6, where said use is combined with an estrogen-receptor antibody and/or a HER2 antibody.

## Patentansprüche

**1.** Antikörper oder Antigen-bindendes Fragment davon, der bzw. das spezifisch eine extrazelluläre Polypeptidregion von humanem NBCn1 erkennt und daran bindet, wobei die extrazelluläre Polypeptidregion eine Sequenz umfasst oder aus einer Sequenz besteht, die aus einer beliebigen aus SEQ ID NO: 14 und 15 ausgewählt ist, und wobei der Antikörper oder das Antigen-bindende Fragment davon dazu in der Lage ist, zellulären NBCnl-vermittelten $Na^+$-abhängigen $HCO_3^-$-Transport zu hemmen, und wobei das Antigen-bindende Fragment Folgendes ist: (i) ein Fab-Fragment, ein monovalentes Fragment, das aus der VL-, VH-, CL- und CH1-Domäne besteht; (ii) ein F(ab')2-Fragment, ein bivalentes Fragment, das zwei Fab-Fragmente umfasst, die durch eine Disulfidbrücke an der Gelenkregion verbunden sind; (iii) ein Fd-Fragment, das aus der VH- und CH1-Domäne besteht; (iv) ein Fv-Fragment, das aus der VL- und VH-Domäne eines einzelnen Arms eines Antikörpers besteht, oder (v) ein dAb-Fragment, das aus einer VH-Domäne besteht.

**2.** Verfahren zum Auswählen eines Antikörpers, wobei das Verfahren Folgendes umfasst:

a) Bereitstellen einer Antikörperbibliothek,
b) Bereitstellen von einem oder mehreren Antigenen aus einer extrazellulären Polypeptidregion von humanem NBCn1, wobei die extrazelluläre Polypeptidregion eine Sequenz umfasst oder aus einer Sequenz besteht, die aus einer beliebigen aus SEQ ID NO: 14 und 15 ausgewählt ist,
c) Kontaktieren der Antikörperbibliothek mit dem einen oder den mehreren Antigenen und
d) Auswählen von Antikörpern, die an das eine oder die mehreren Antigene binden und dazu in der Lage sind, zellulären NBCnl-vermittelten $Na^+$-abhängigen $HCO_3^-$-Transport zu hemmen.

**3.** Verfahren zum Herstellen eines Antikörpers, der spezifisch eine extrazelluläre Polypeptidregion von humanem NBCn1 erkennt und daran bindet, wobei der Antikörper dazu in der Lage ist, zellulären NBCnl-vermittelten $Na^+$-abhängigen $HCO_3^-$-Transport zu hemmen, wobei das Verfahren die folgenden Schritte umfasst: Verabreichen eines extrazellulären Polypeptidfragments von humanem NBCn1 oder eines Homologs, das zu mindestens 98 % damit identisch ist, an ein Tier, bevorzugt ein Säugetier, wobei die extrazelluläre Polypeptidregion eine Sequenz umfasst oder aus einer Sequenz besteht, die aus einer beliebigen aus SEQ ID NO: 14 und 15 ausgewählt ist, Isolieren von Antikörpern aus dem Säugetier, Testen, ob Antikörper dazu in der Lage sind, zellulären NBCnl-vermittelten $Na^+$-abhängigen $HCO_3^-$-Transport zu hemmen, und Auswählen von Antikörpern, die dazu in der Lage sind, zellulären NBCnl-vermittelten $Na^+$-abhängigen $HCO_3^-$-Transport zu hemmen.

**4.** Antikörper, der spezifisch eine extrazelluläre Polypeptidregion von humanem NBCn1 erkennt und daran bindet, wobei die extrazelluläre Polypeptidregion eine Sequenz umfasst oder aus einer Sequenz besteht, die aus einer beliebigen aus SEQ ID NO: 14 und 15 ausgewählt ist, und wobei der Antikörper dazu in der Lage ist, zellulären NBCnl-vermittelten $Na^+$-abhängigen $HCO_3^-$-Transport zu hemmen, zur Verwendung als Medikament.

**5.** Antikörper nach Anspruch 4 zur Verwendung bei der Behandlung von Brustkrebs, Atherosklerose und/oder Restenose.

**6.** Antikörper nach Anspruch 5, wobei der Brustkrebs ein dreifach negativer Brustkrebs, ein Östrogenrezeptor-positiver Brustkrebs oder ein HER2-positiver Brustkrebs ist.

**7.** Antikörper nach einem der vorhergehenden Ansprüche 5 und 6, wobei die Verwendung mit einem Östrogenrezeptor-Antikörper und/oder einem HER2-Antikörper kombiniert wird.

## Revendications

**1.** Anticorps ou fragment de liaison à l'antigène de celui-ci reconnaissant et se liant spécifiquement à une région polypeptidique extracellulaire de NBCn1 humain, dans lequel ladite région polypeptidique extracellulaire comprend ou est constituée d'une séquence choisie parmi l'une quelconque de SEQ ID NO: 14 et 15, et dans lequel ledit anticorps ou fragment de liaison à l'antigène de celui-ci est capable d'inhiber le transport cellulaire de HCO3-dépendant de Na+ médié par NBCn1, et dans lequel ledit fragment de liaison à l'antigène est (i) un fragment Fab, un fragment monovalent constitué des domaines VL, VH, CL et CH1 ; (ii) un fragment F(ab')2, un fragment bivalent comprenant deux fragments Fab liés par un pont disulfure au niveau de la région charnière ; (iii) un fragment Fd constitué des domaines VH et CH1 ; (iv) un fragment Fv constitué des domaines VL et VH d'un seul bras d'un anticorps ou (v) un fragment dAb, qui est constitué d'un domaine VH.

**2.** Procédé de sélection d'un anticorps, ledit procédé comprenant

a) la fourniture d'une bibliothèque d'anticorps,
b) la fourniture d'un ou de plusieurs antigènes d'une région polypeptidique extracellulaire de NBCn1 humain, dans lequel ladite région polypeptidique extracellulaire comprend ou est constituée d'une séquence choisie parmi l'une quelconque de SEQ ID NO: 14 et 15
c) la mise en contact de ladite bibliothèque d'anticorps avec lesdits un ou plusieurs antigènes, et
d) la sélection d'anticorps, qui se lient auxdits un ou plusieurs antigènes et sont capables d'inhiber le transport cellulaire de $HCO_3^-$ dépendant de $Na^+$ médié par NBCn1.

**3.** Procédé de production d'un anticorps reconnaissant et se liant spécifiquement à une région polypeptidique extracellulaire de NBCn1 humain, dans lequel ledit anticorps est capable d'inhiber le transport cellulaire de $HCO_3^-$ dépendant de $Na^+$ médié par NBCn1, ledit procédé comprenant les étapes d'administration à un animal, de préférence un mammifère, d'un fragment polypeptidique extracellulaire de NBCn1 humain ou d'un homologue identique à au moins 98 % à celui-ci, dans lequel ladite région polypeptidique extracellulaire comprend ou est constituée d'une séquence choisie parmi l'une quelconque de SEQ ID NO: 14 et 15, d'isolation d'anticorps dudit mammifère, de test pour déterminer si les anticorps sont capables d'inhiber le transport cellulaire de $HCO_3^-$ dépendant de $Na^+$ médié par NBCn1 et de sélection des anticorps capables d'inhiber le transport cellulaire de $HCO_3^-$ dépendant de $Na^+$ médié par NBCn1.

**4.** Anticorps reconnaissant et se liant spécifiquement à une région polypeptidique extracellulaire de NBCn1 humain, dans lequel ladite région polypeptidique extracellulaire comprend ou est constituée d'une séquence choisie parmi l'une quelconque de SEQ ID NO: 14 et 15, et dans lequel ledit anticorps est capable d'inhiber le transport cellulaire de $HCO^{3-}$ dépendant de $Na^{+}$ médié par NBCn1 pour une utilisation comme médicament.

**5.** Anticorps selon la revendication 4 destiné à être utilisé dans le traitement du cancer du sein, de l'athérosclérose et/ou de la resténose.

**6.** Anticorps selon la revendication 5, dans lequel ledit cancer du sein est un cancer du sein triple négatif, un cancer du sein positif aux récepteurs d'œstrogène ou un cancer du sein HER2 positif.

**7.** Anticorps selon l'une quelconque des revendications 5 et 6 précédentes, dans lequel ladite utilisation est combinée avec un anticorps récepteur d'œstrogène et/ou un anticorps HER2.

Figure 1

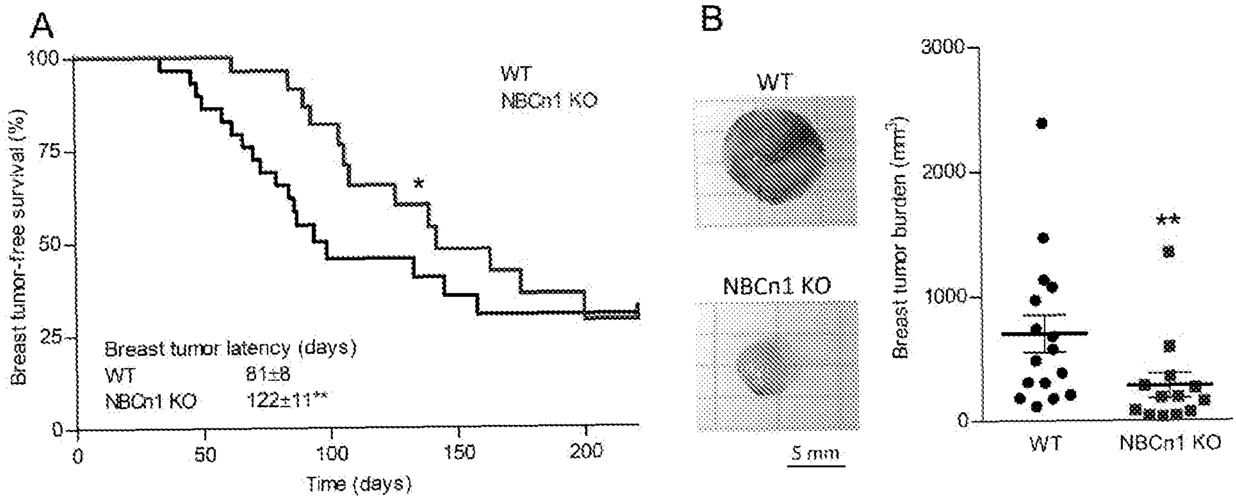
A
100
75
50
25
0
Breast tumor-free survival (%)
WT
NBCn1 KO
*
Breast tumor latency (days)
WT 81±8
NBCn1 KO 122±11**
0
50
100
150
200
Time (days)
B
WT
NBCn1 KO
5 mm
3000
2000
1000
0
Breast tumor burden (mm3)
**
WT
NBCn1 KO

Figure 2

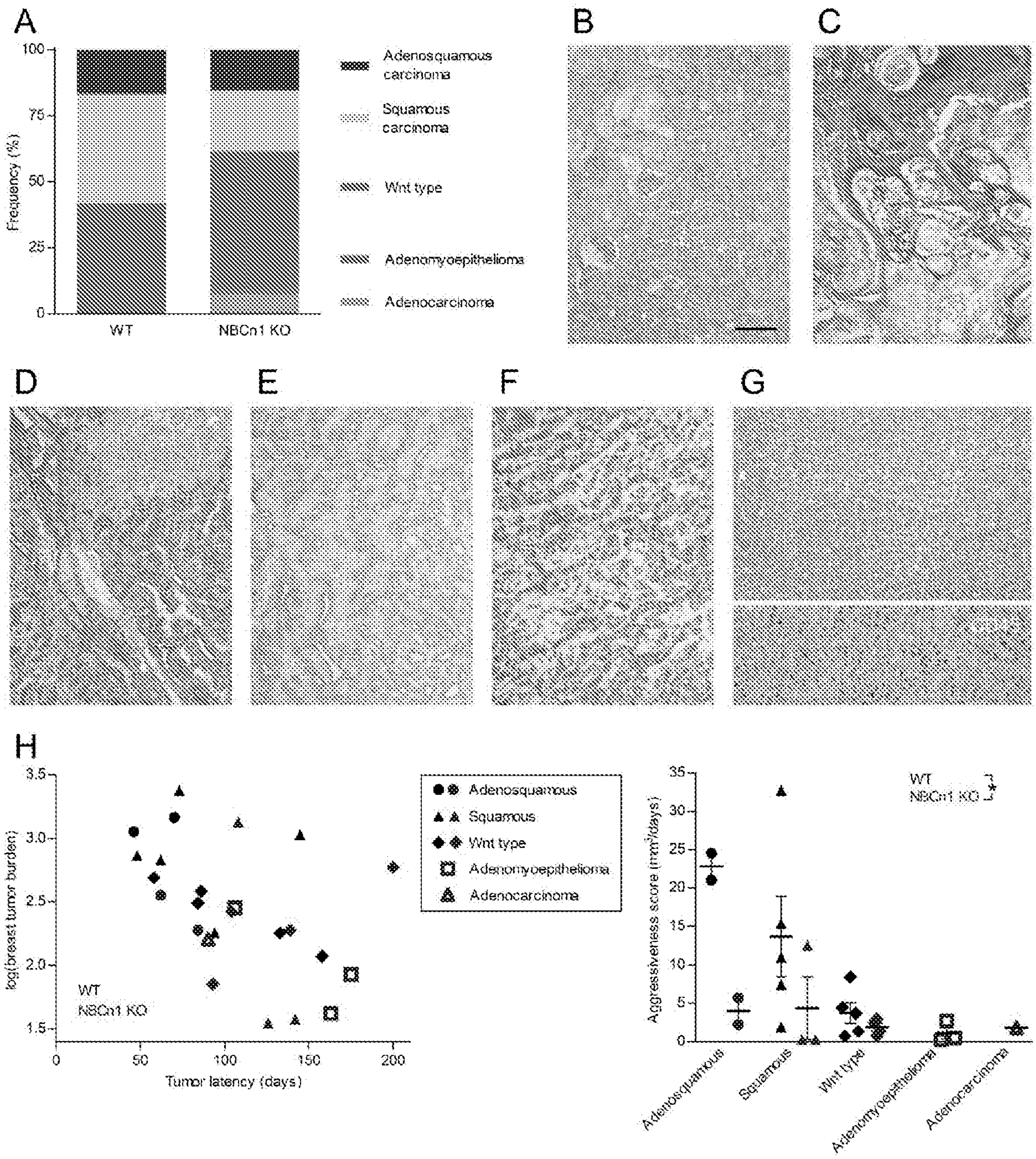
A
B
C
D
E
F
G
H
100
75
50
25
0
Frequency (%)
WT
NBCn1 KO
Adenosquamous carcinoma
Squamous carcinoma
Wnt type
Adenomyoepithelioma
Adenocarcinoma
3.5
3.0
2.5
2.0
1.5
log(breast tumor burden)
0
50
100
150
200
Tumor latency (days)
WT
NBCn1 KO
Adenosquamous
Squamous
Wnt type
Adenomyoepithelioma
Adenocarcinoma
35
30
25
20
15
10
5
0
Aggressiveness score (mm³/days)
WT
NBCn1 KO
*
Adenosquamous
Squamous
Wnt type
Adenomyoepithelioma
Adenocarcinoma

Figure 3

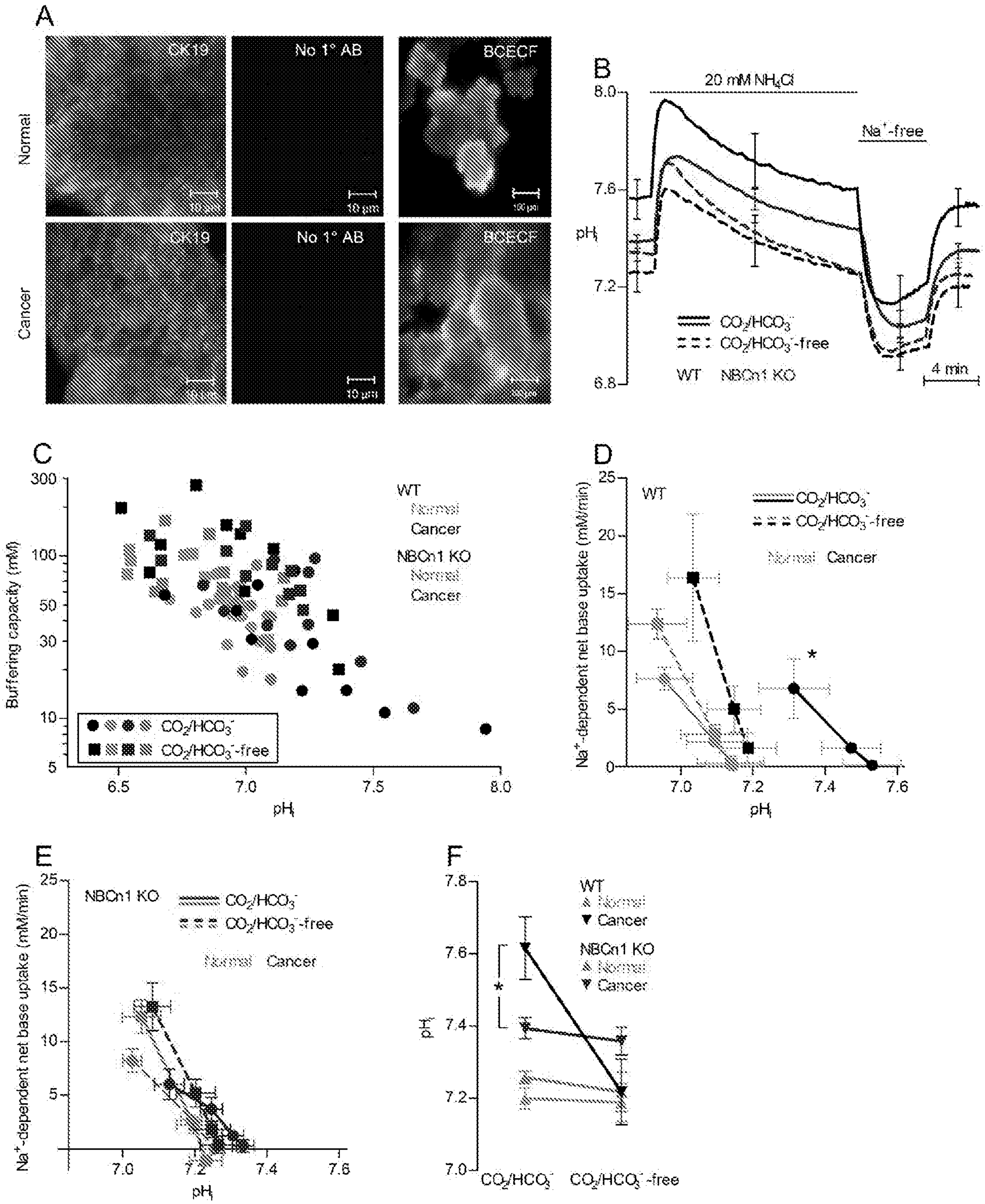

A
CK19
No 1° AB
BCECF
Normal
Cancer
10 µm
100 µm
B
20 mM $NH_4Cl$
$Na^+$-free
$pH_i$
$CO_2/HCO_3^-$
$CO_2/HCO_3^-$-free
WT
NBCn1 KO
4 min
C
Buffering capacity (mM)
WT
Normal
Cancer
NBCn1 KO
Normal
Cancer
$CO_2/HCO_3^-$
$CO_2/HCO_3^-$-free
$pH_i$
D
WT
$CO_2/HCO_3^-$
$CO_2/HCO_3^-$-free
Normal
Cancer
$Na^+$-dependent net base uptake (mM/min)
$pH_i$
E
NBCn1 KO
$CO_2/HCO_3^-$
$CO_2/HCO_3^-$-free
Normal
Cancer
$Na^+$-dependent net base uptake (mM/min)
$pH_i$
F
WT
Normal
Cancer
NBCn1 KO
Normal
Cancer
$pH_i$
$CO_2/HCO_3^-$
$CO_2/HCO_3^-$-free

Figure 4

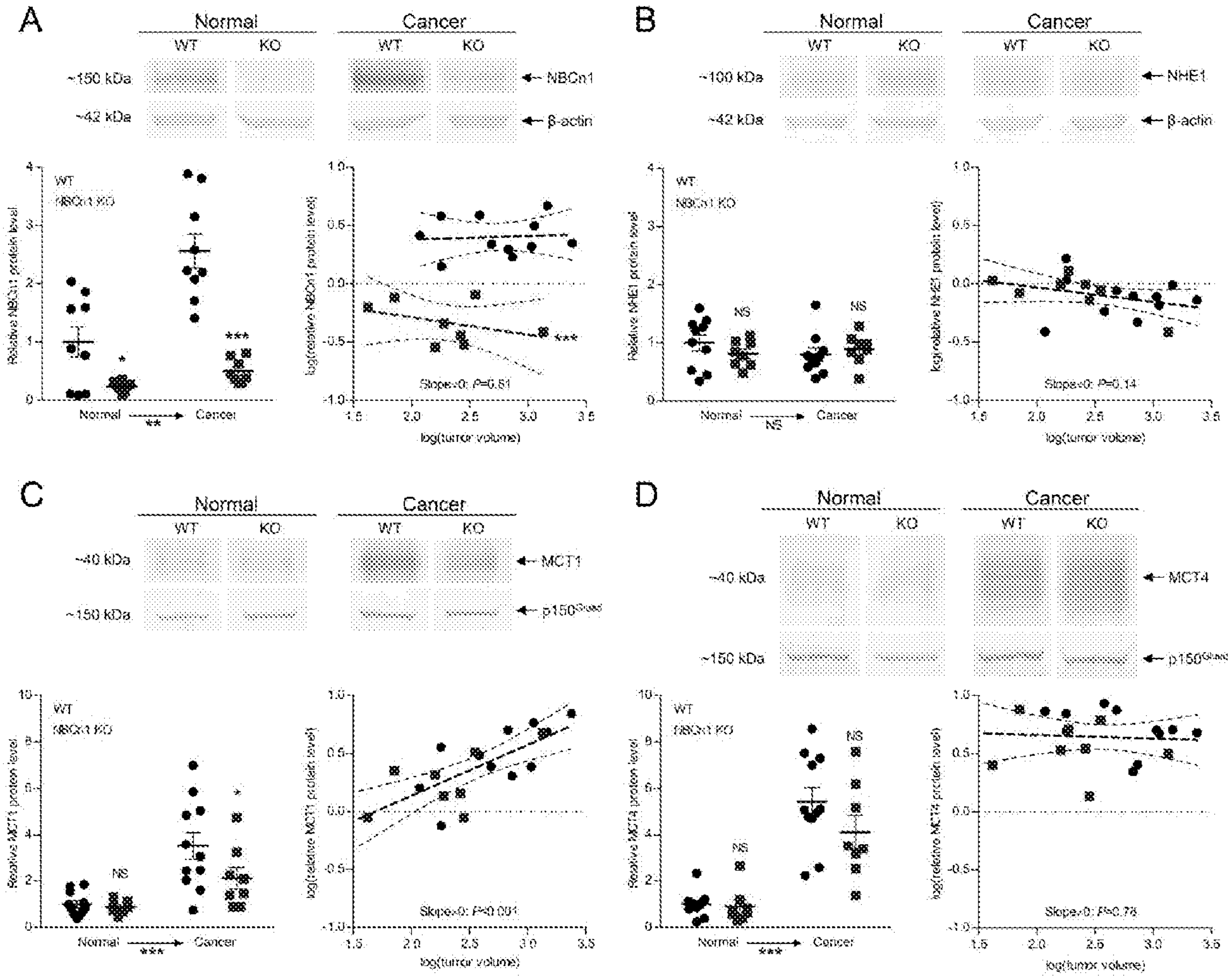
A
Normal
Cancer
WT
KO
~150 kDa
~42 kDa
NBCn1
β-actin
B
~100 kDa
NHE1
C
~40 kDa
MCT1
~150 kDa
D
MCT4
Normal
Cancer
log(tumor volume)
NS

Figure 5

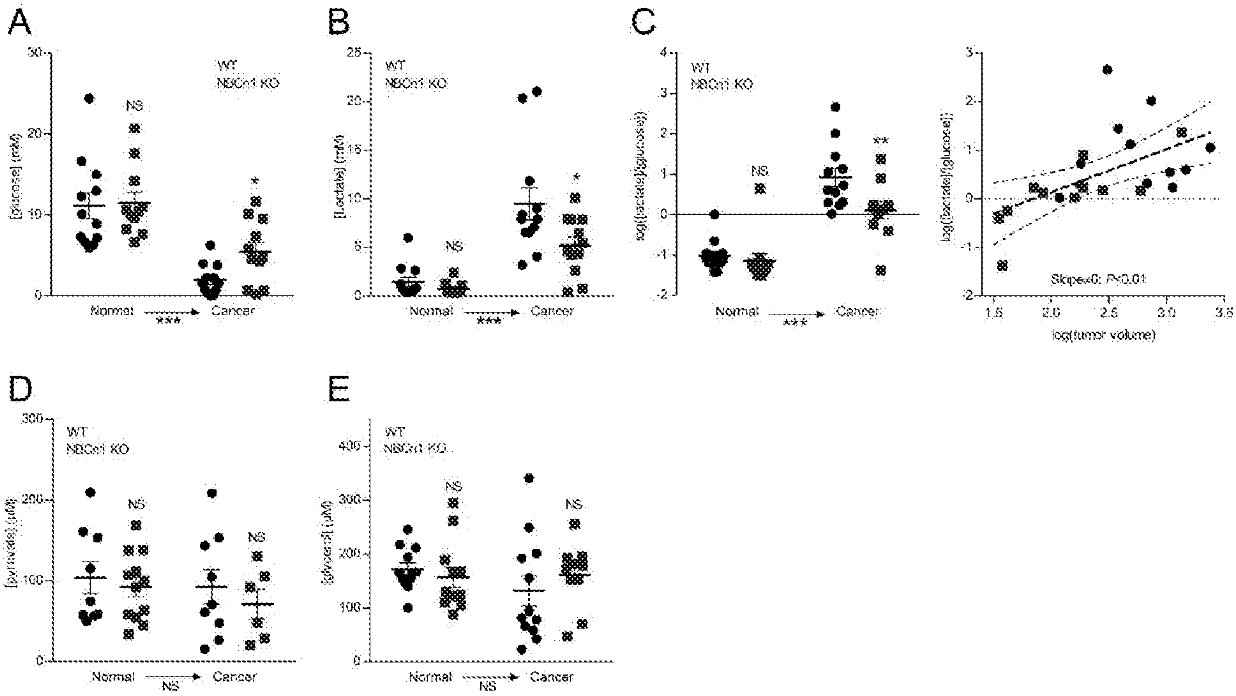
A
B
C
D
E
WT
NBCn1 KO
NS
Normal
Cancer
***
Slope≠0: P<0.01
log(tumor volume)

Figure 6

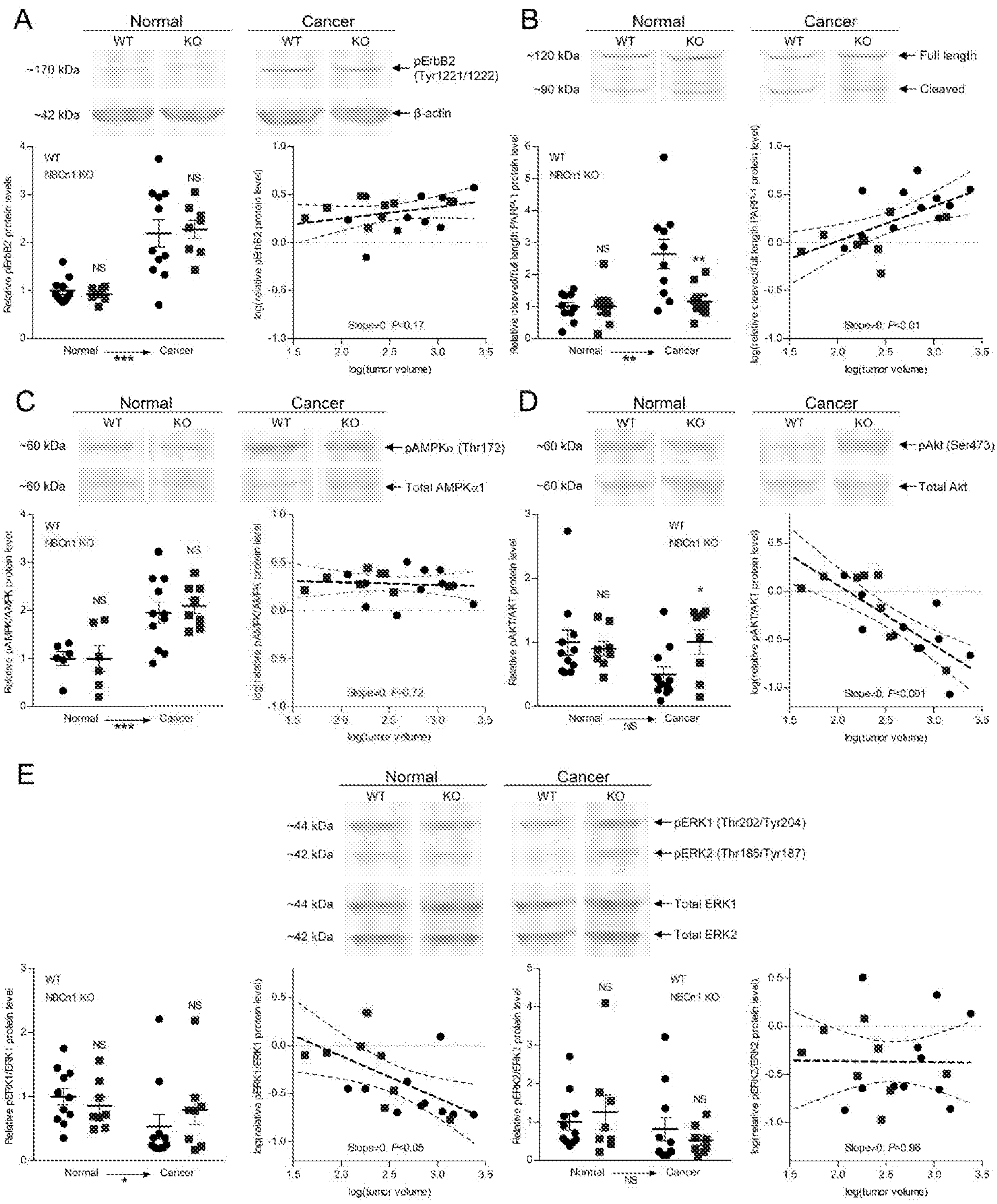
A
Normal
Cancer
WT
KO
~170 kDa
~42 kDa
pErbB2 (Tyr1221/1222)
β-actin
Slope≠0: P=0.17
log(tumor volume)
B
~120 kDa
~90 kDa
Full length
Cleaved
Slope≠0: P<0.01
C
~60 kDa
pAMPKα (Thr172)
Total AMPKα1
Slope≠0: P=0.72
D
pAkt (Ser473)
Total Akt
Slope≠0: P<0.001
E
~44 kDa
~42 kDa
pERK1 (Thr202/Tyr204)
pERK2 (Thr185/Tyr187)
Total ERK1
Total ERK2
Slope≠0: P<0.05
Slope≠0: P=0.96

Figure 7

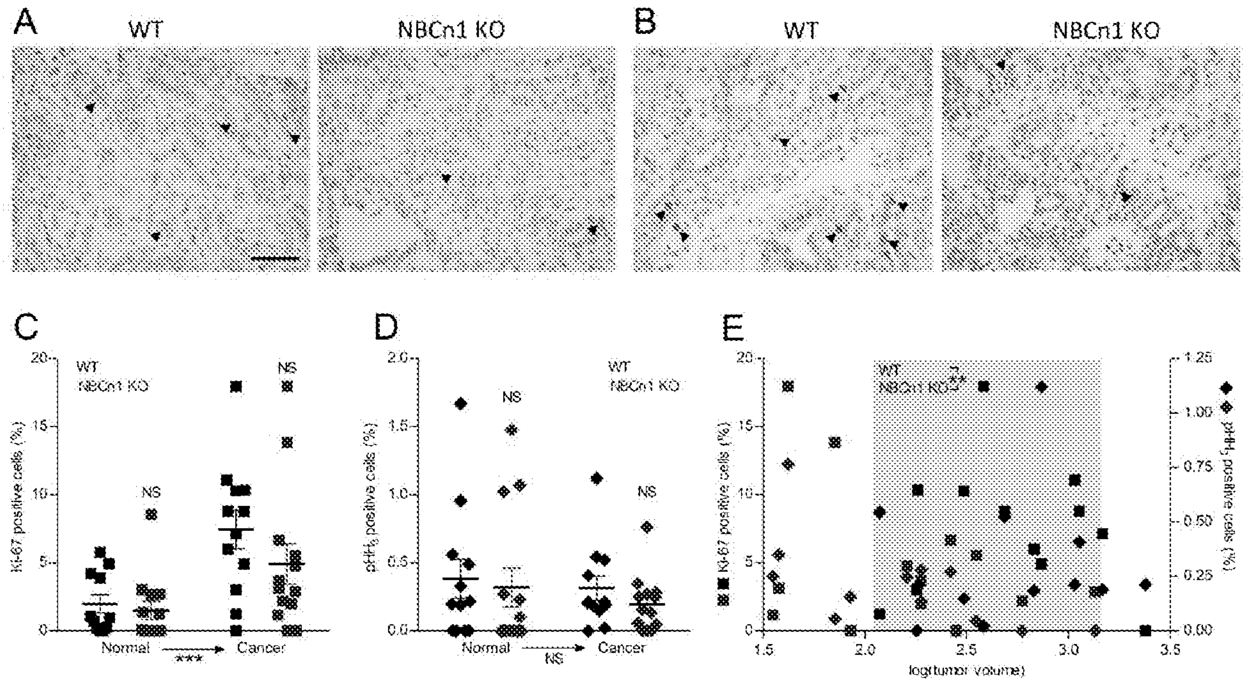
A
WT
NBCn1 KO
B
WT
NBCn1 KO
C
WT
NBCn1 KO
NS
NS
Ki-67 positive cells (%)
Normal
***
Cancer
D
WT
NBCn1 KO
NS
NS
pHH3 positive cells (%)
Normal
NS
Cancer
E
WT
NBCn1 KO
**
Ki-67 positive cells (%)
pHH3 positive cells (%)
log(tumor volume)

Figure 8

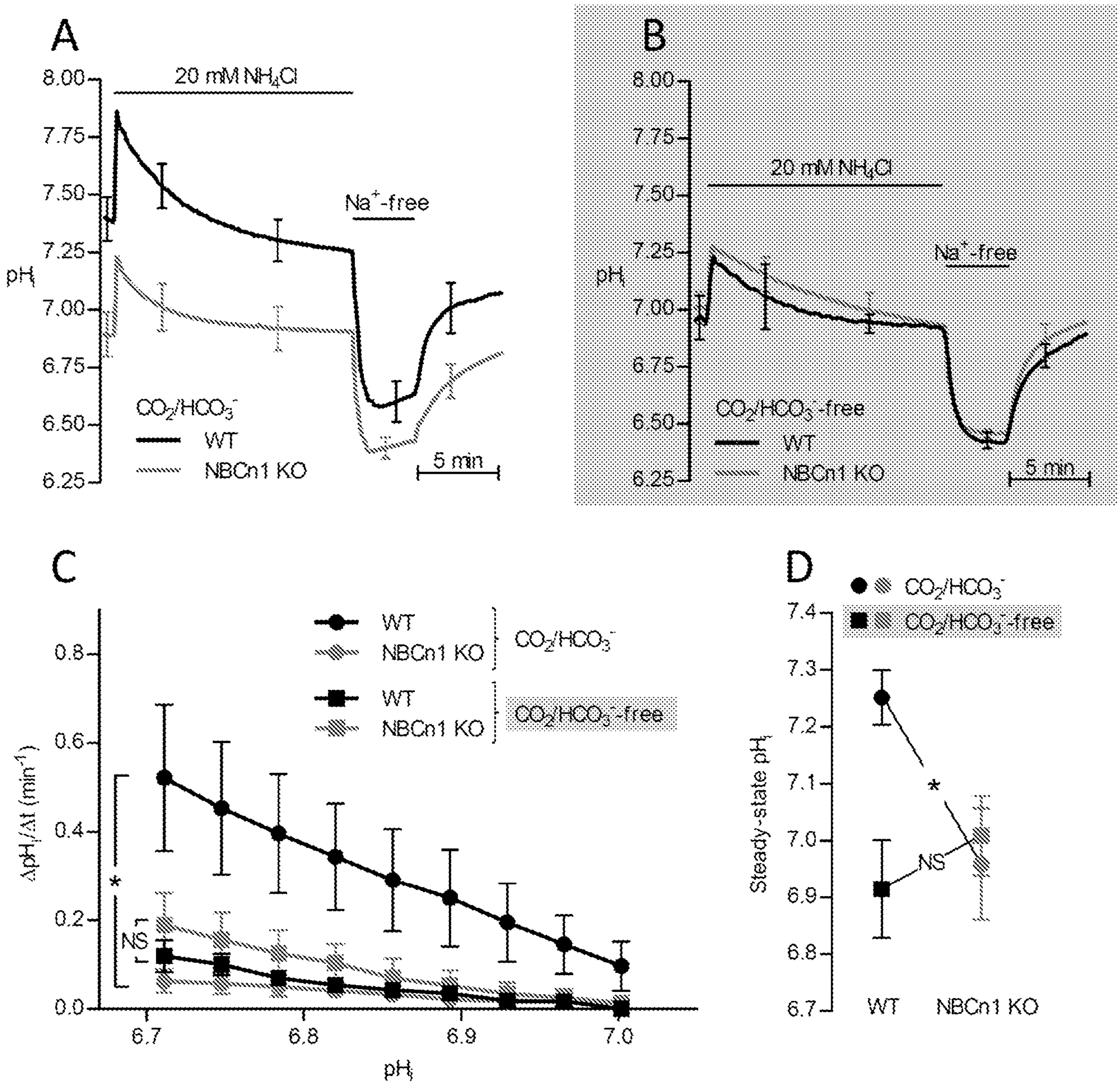
A
20 mM NH4Cl
Na+-free
pHi
CO2/HCO3-
WT
NBCn1 KO
5 min
B
20 mM NH4Cl
Na+-free
CO2/HCO3--free
C
WT
NBCn1 KO
CO2/HCO3-
CO2/HCO3--free
ΔpHi/Δt (min-1)
NS
*
D
CO2/HCO3-
CO2/HCO3--free
Steady-state pHi
WT
NBCn1 KO

Figure 9

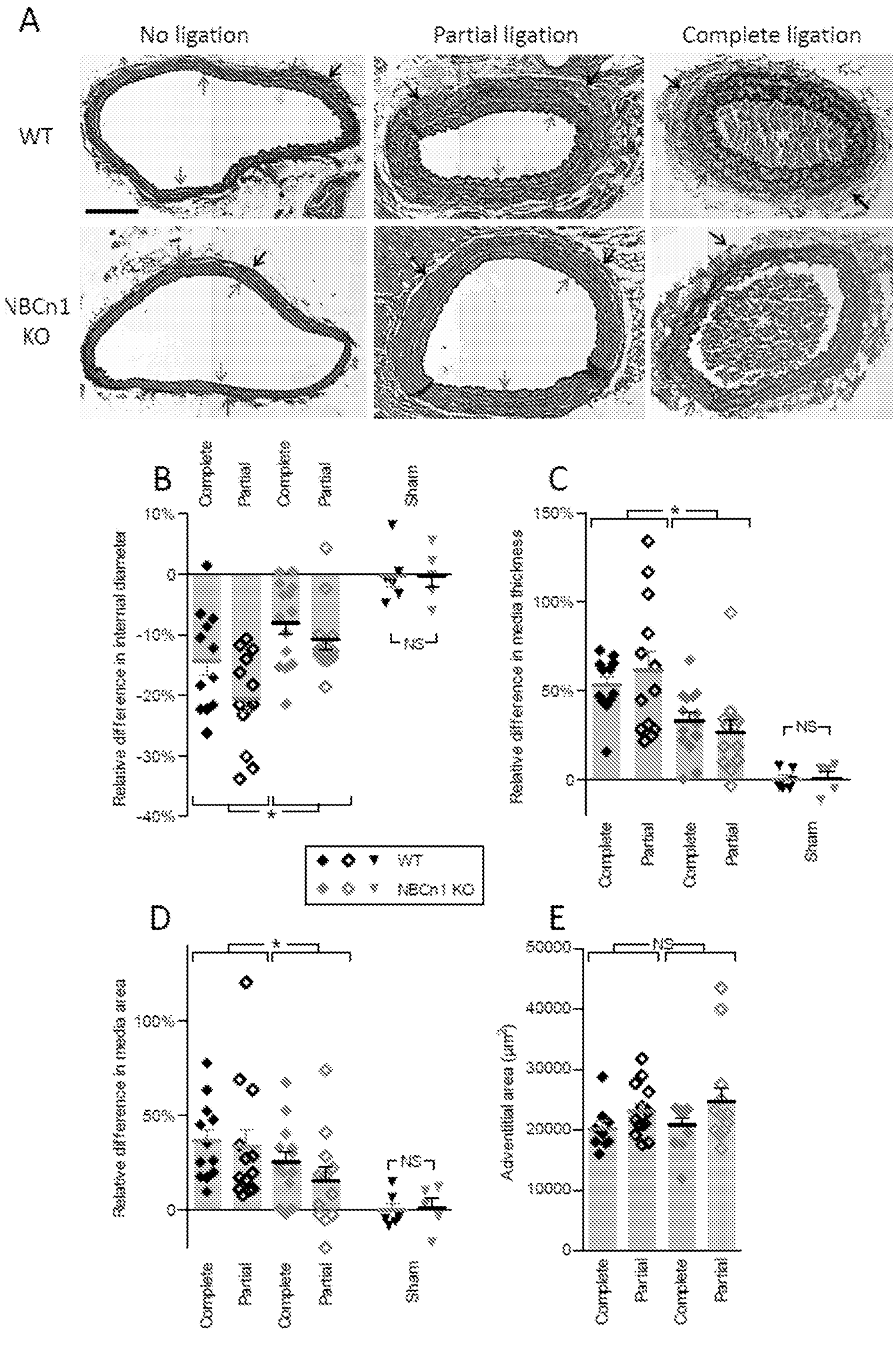
A
No ligation
Partial ligation
Complete ligation
WT
NBCn1 KO
B
C
D
E
Complete
Partial
Sham
Relative difference in internal diameter
Relative difference in media thickness
Relative difference in media area
Adventitial area (µm²)
NS
*
WT
NBCn1 KO

Figure 10

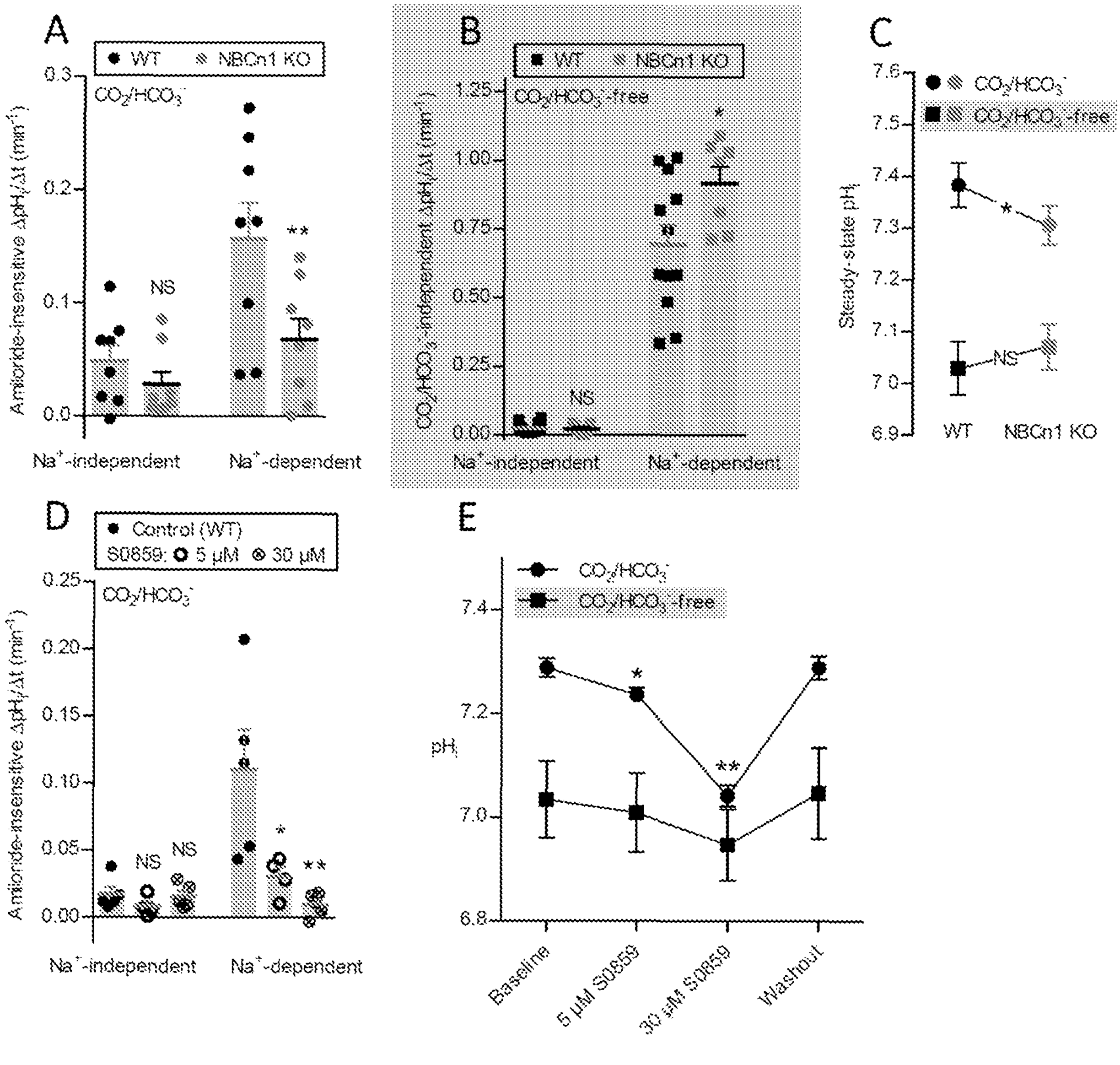
A
WT
NBCn1 KO
CO2/HCO3-
Amiloride-insensitive ΔpHi/Δt (min-1)
NS
**
Na+-independent
Na+-dependent
B
WT
NBCn1 KO
CO2/HCO3- -free
CO2/HCO3- -independent ΔpHi/Δt (min-1)
NS
*
Na+-independent
Na+-dependent
C
CO2/HCO3-
CO2/HCO3- -free
Steady-state pHi
*
NS
WT
NBCn1 KO
D
Control (WT)
S0859: 5 μM 30 μM
CO2/HCO3-
Amiloride-insensitive ΔpHi/Δt (min-1)
NS
NS
*
**
Na+-independent
Na+-dependent
E
CO2/HCO3-
CO2/HCO3- -free
pHi
Baseline
5 μM S0859
30 μM S0859
Washout

Figure 11

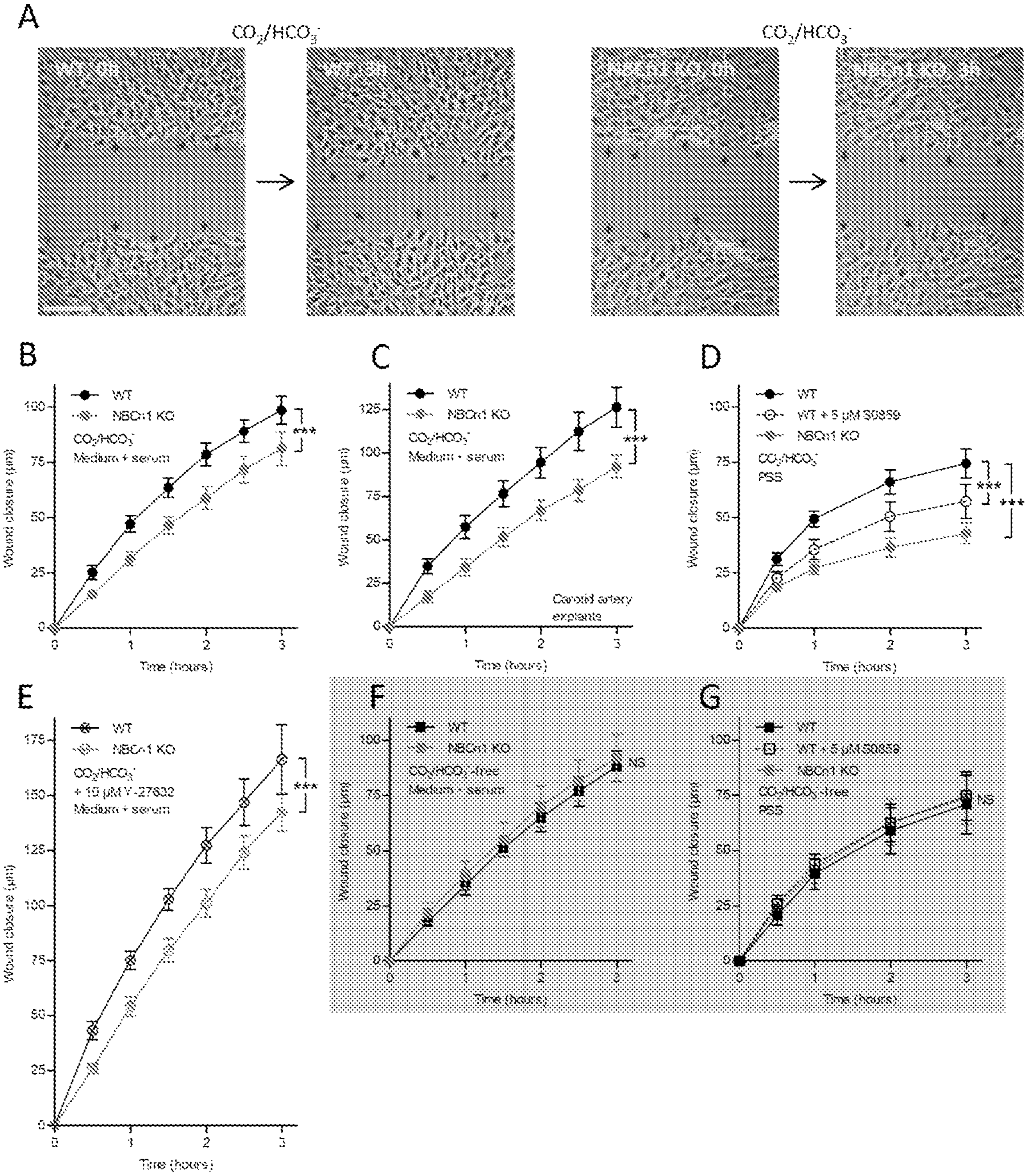
A
$CO_2/HCO_3^-$
$CO_2/HCO_3^-$
B
WT
NBCn1 KO
$CO_2/HCO_3^-$
Medium + serum
Wound closure (µm)
Time (hours)
***
C
WT
NBCn1 KO
$CO_2/HCO_3^-$
Medium + serum
Carotid artery explants
Wound closure (µm)
Time (hours)
***
D
WT
WT + 5 µM S0859
NBCn1 KO
$CO_2/HCO_3^-$
PSS
Wound closure (µm)
Time (hours)
***
***
E
WT
NBCn1 KO
$CO_2/HCO_3^-$
+ 10 µM Y-27632
Medium + serum
Wound closure (µm)
Time (hours)
***
F
WT
NBCn1 KO
$CO_2/HCO_3^-$-free
Medium + serum
Wound closure (µm)
Time (hours)
NS
G
WT
WT + 5 µM S0859
NBCn1 KO
$CO_2/HCO_3^-$-free
PSS
Wound closure (µm)
Time (hours)
NS

Figure 12

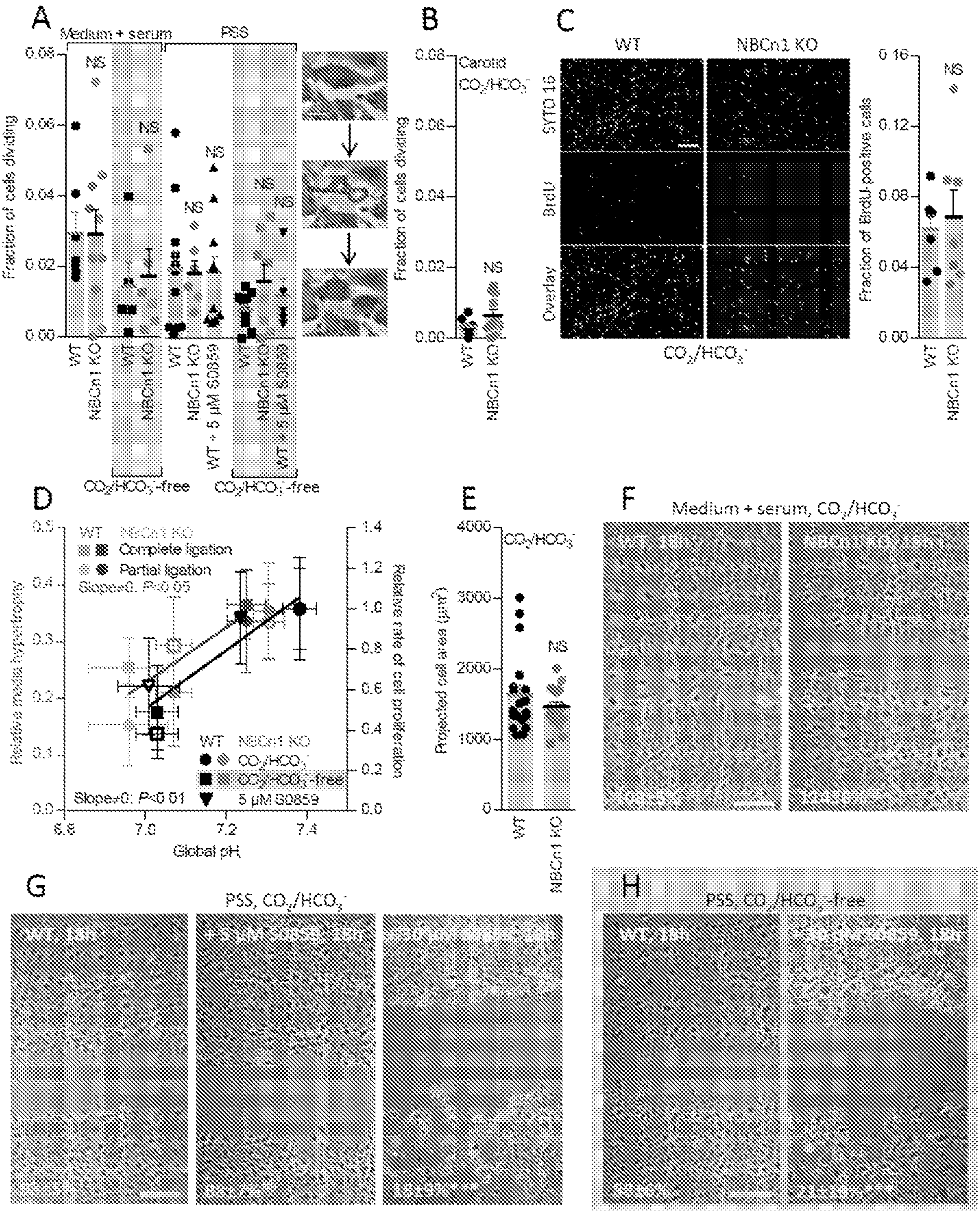
A
Medium + serum
PSS
Fraction of cells dividing
NS
WT
NBCn1 KO
WT + 5 µM S0859
CO2/HCO3- -free
B
Carotid
CO2/HCO3-
C
WT
NBCn1 KO
SYTO 16
BrdU
Overlay
CO2/HCO3-
Fraction of BrdU positive cells
D
Complete ligation
Partial ligation
Slope≠0: P<0.05
Slope≠0: P<0.01
Relative media hypertrophy
Relative rate of cell proliferation
Global pHi
5 µM S0859
E
Projected cell area (µm²)
F
Medium + serum, CO2/HCO3-
G
PSS, CO2/HCO3-
H
PSS, CO2/HCO3- -free

Figure 13

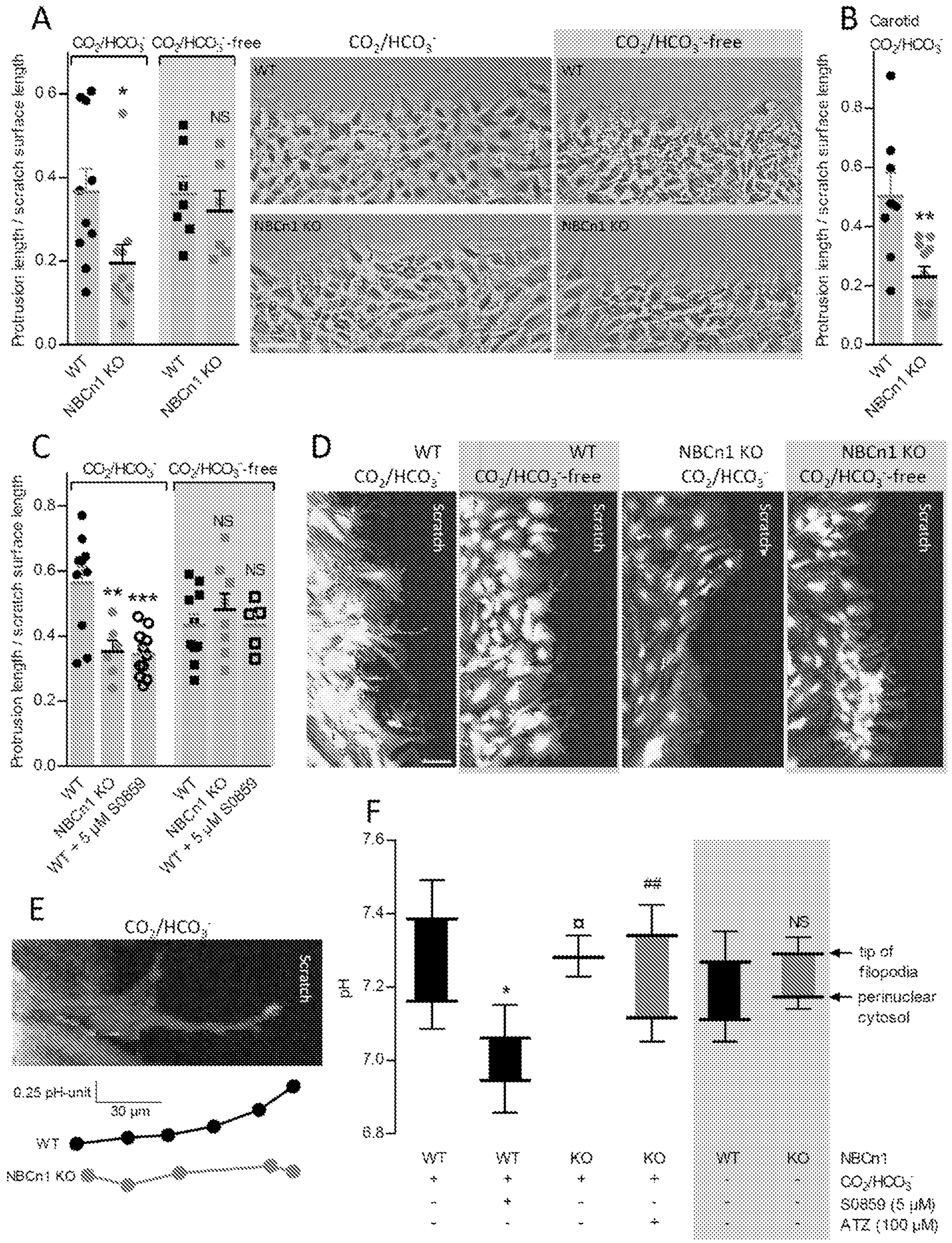
A
$CO_2/HCO_3^-$
$CO_2/HCO_3^-$-free
Protrusion length / scratch surface length
WT
NBCn1 KO
NS
B
Carotid
C
WT + 5 µM S0859
D
Scratch
E
0.25 pH-unit
30 µm
F
pH
tip of filopodia
perinuclear cytosol
NBCn1
S0859 (5 µM)
ATZ (100 µM)

Figure 14

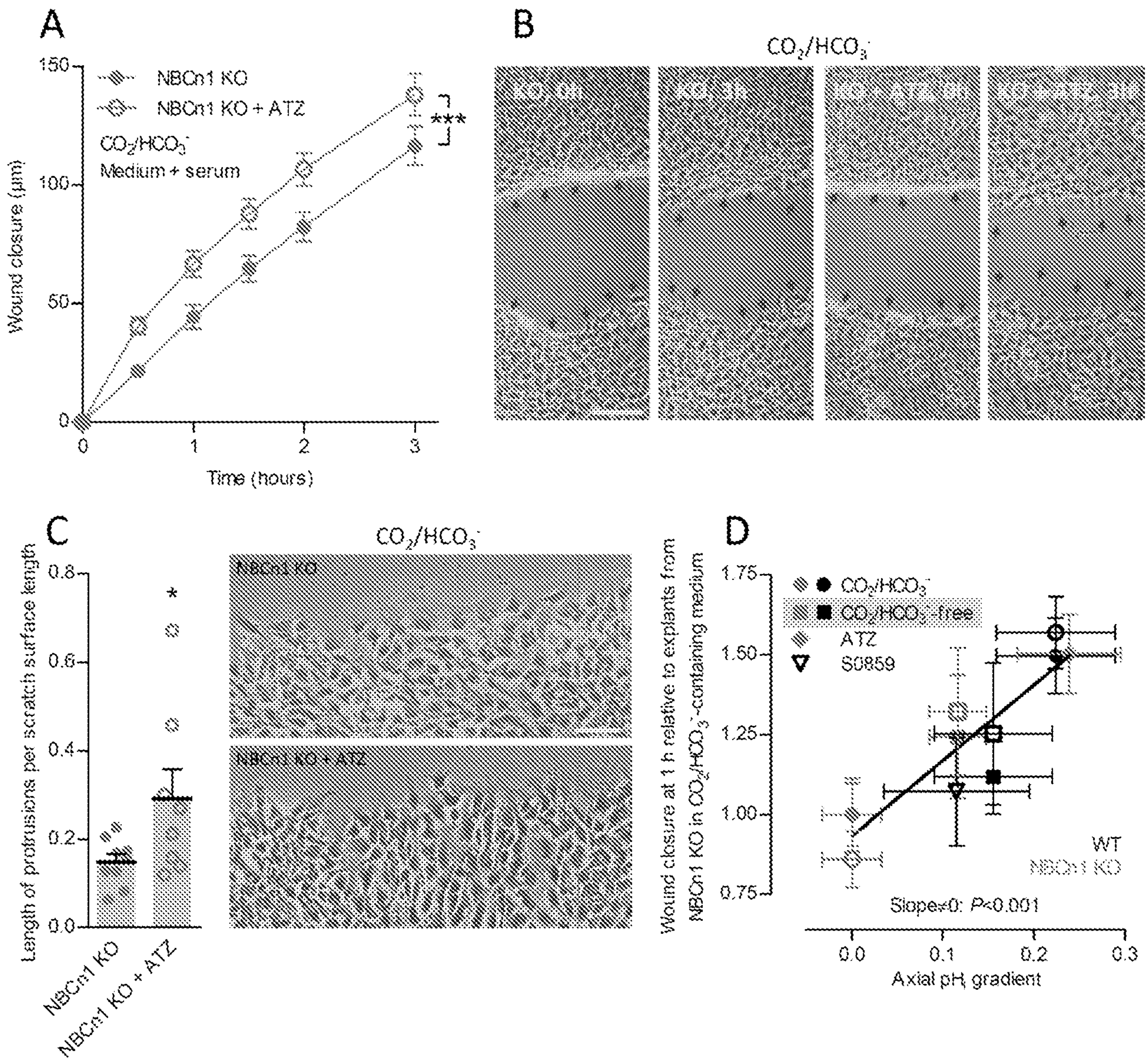
A
NBCn1 KO
NBCn1 KO + ATZ
$CO_2/HCO_3^-$
Medium + serum
***
Wound closure (μm)
Time (hours)
B
$CO_2/HCO_3^-$
C
$CO_2/HCO_3^-$
NBCn1 KO
NBCn1 KO + ATZ
Length of protrusions per scratch surface length
*
D
$CO_2/HCO_3^-$
$CO_2/HCO_3^-$-free
ATZ
S0859
WT
NBCn1 KO
Slope≠0: $P<0.001$
Wound closure at 1 h relative to explants from NBCn1 KO in $CO_2/HCO_3^-$-containing medium
Axial $pH_i$ gradient

Figure 15

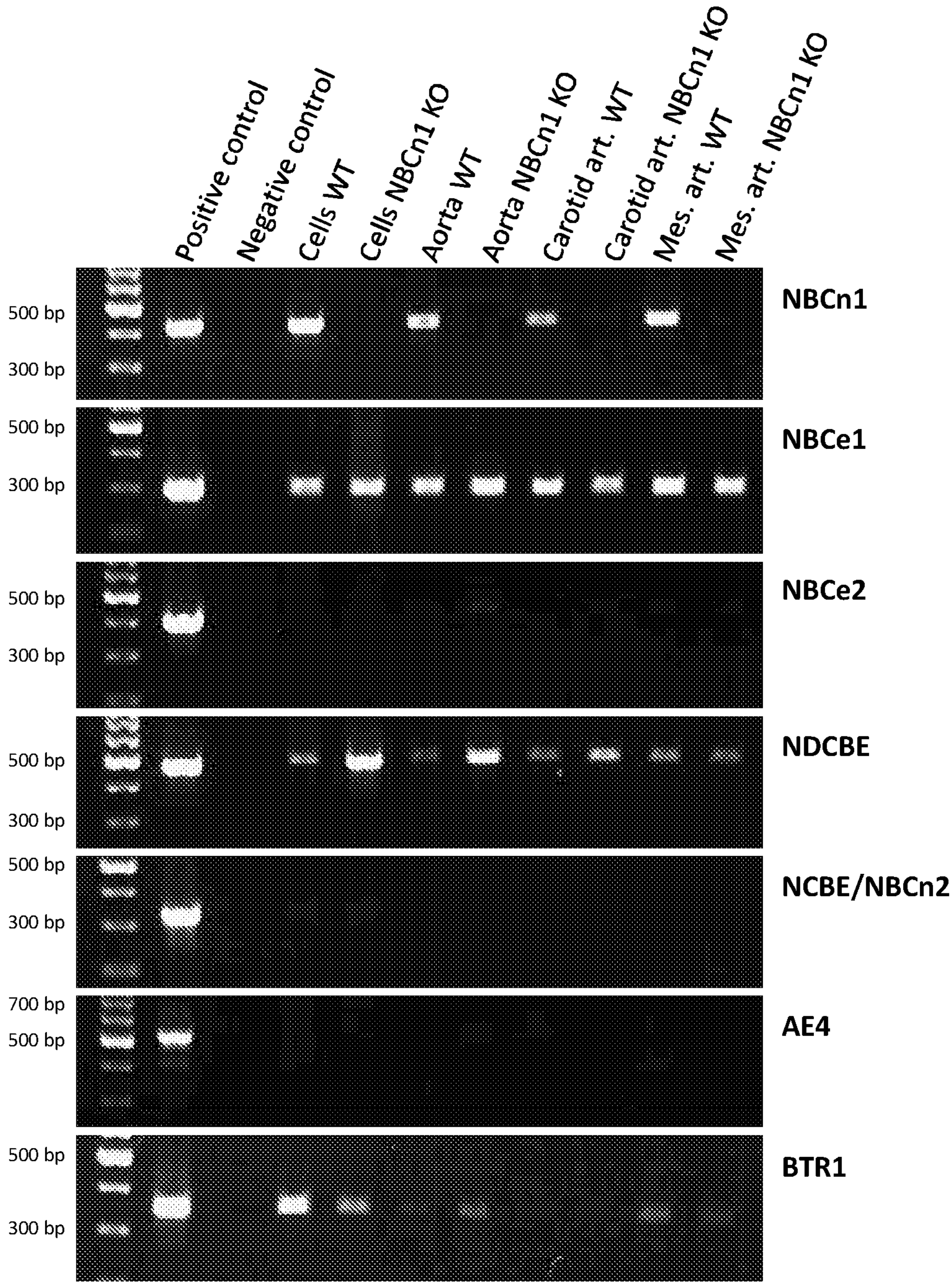
Positive control
Negative control
Cells WT
Cells NBCn1 KO
Aorta WT
Aorta NBCn1 KO
Carotid art. WT
Carotid art. NBCn1 KO
Mes. art. WT
Mes. art. NBCn1 KO
500 bp
300 bp
NBCn1
500 bp
300 bp
NBCe1
500 bp
300 bp
NBCe2
500 bp
300 bp
NDCBE
500 bp
300 bp
NCBE/NBCn2
700 bp
500 bp
AE4
500 bp
300 bp
BTR1

Figure 16

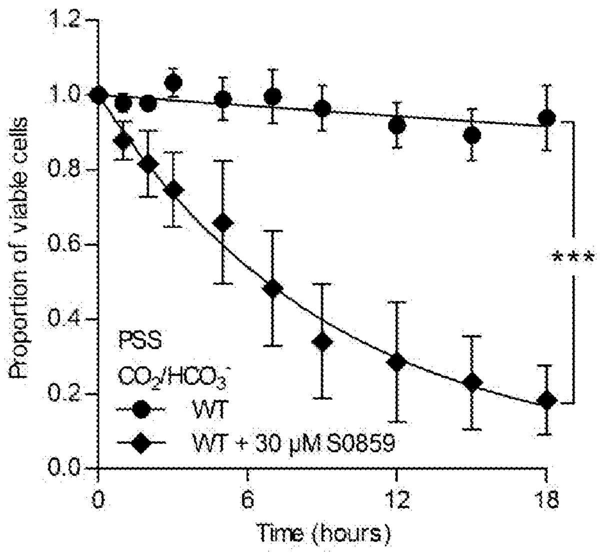
1.2
1.0
0.8
0.6
0.4
0.2
0.0
Proportion of viable cells
0
6
12
18
Time (hours)
PSS
$CO_2/HCO_3^-$
WT
WT + 30 μM S0859
***

Figure 17

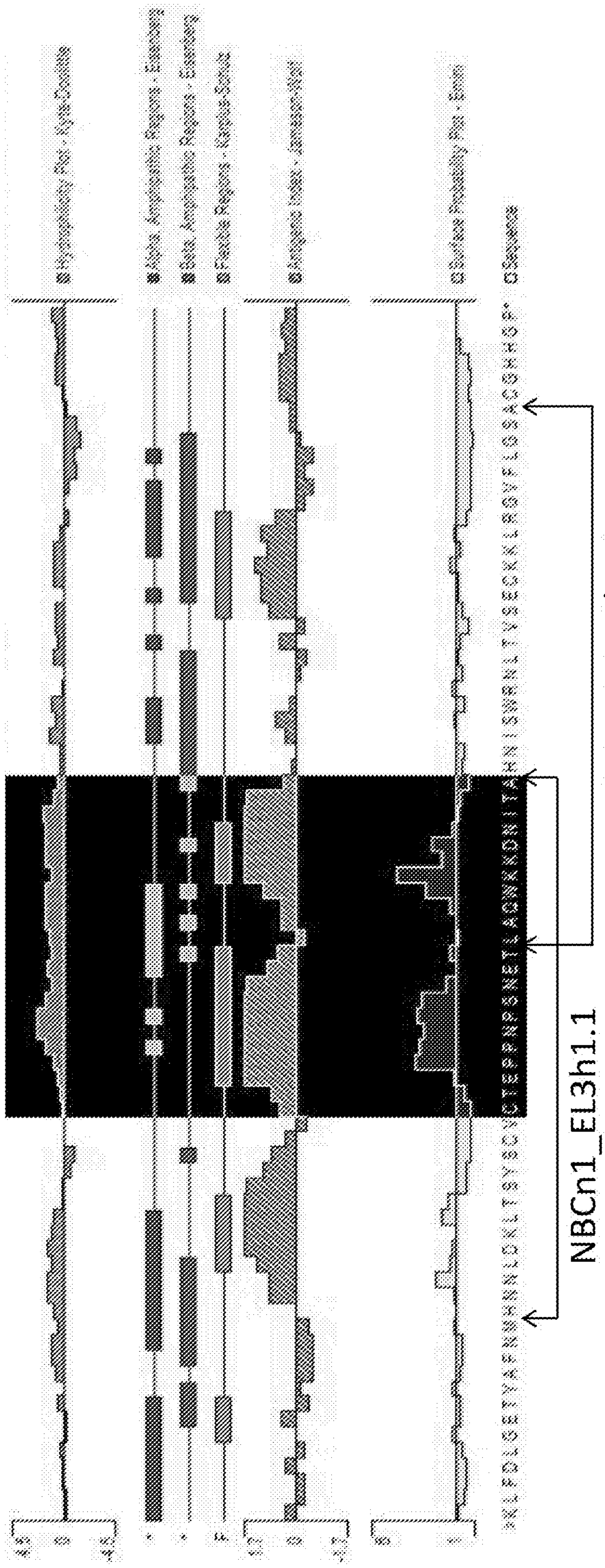
Hydrophobicity Plot - Kyte-Doolittle
Alpha, Amphipathic Regions - Eisenberg
Beta, Amphipathic Regions - Eisenberg
Flexible Regions - Karplus-Schulz
Antigenic Index - Jameson-Wolf
Surface Probability Plot - Emini
Sequence
NBCn1_EL3h1.1
NBCn1_EL3h2.1

Figure 18

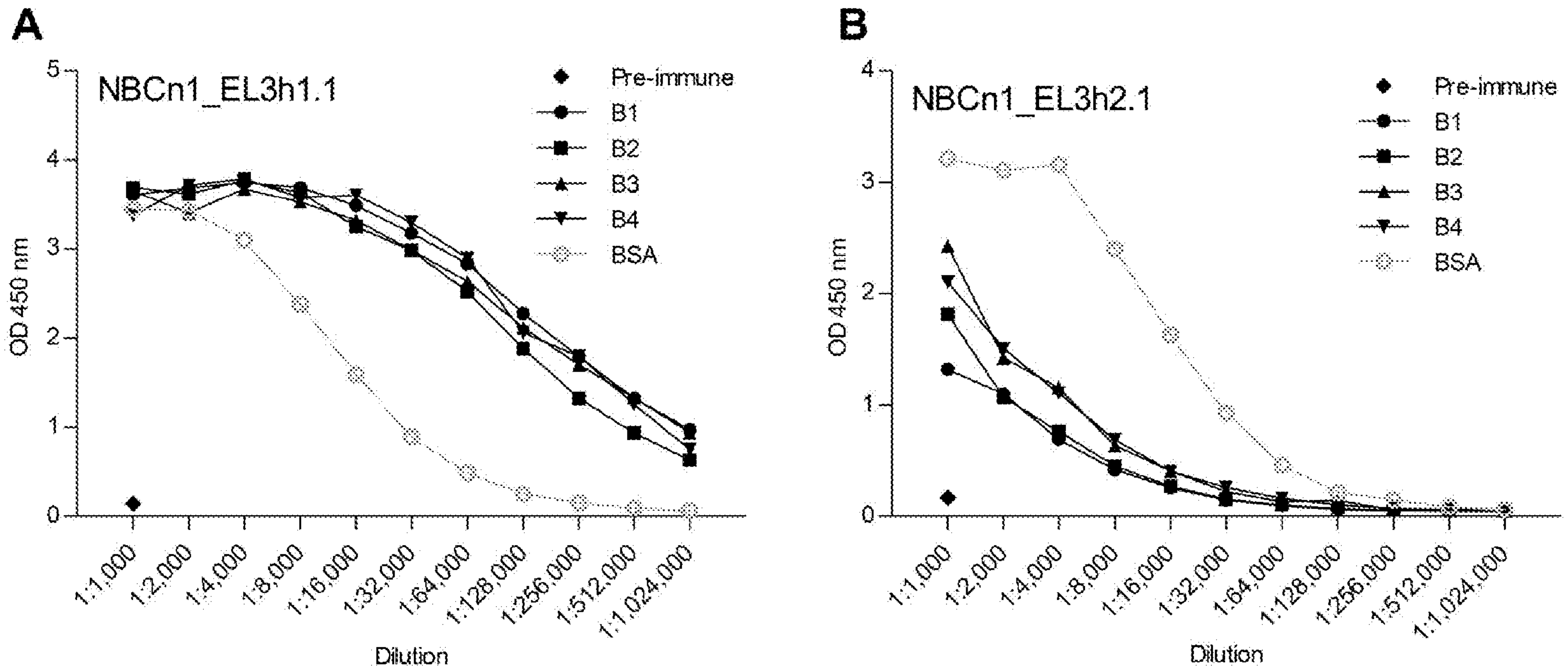
A
NBCn1_EL3h1.1
OD 450 nm
Pre-immune
B1
B2
B3
B4
BSA
Dilution
1:1,000
1:2,000
1:4,000
1:8,000
1:16,000
1:32,000
1:64,000
1:128,000
1:256,000
1:512,000
1:1,024,000
B
NBCn1_EL3h2.1

Figure 19

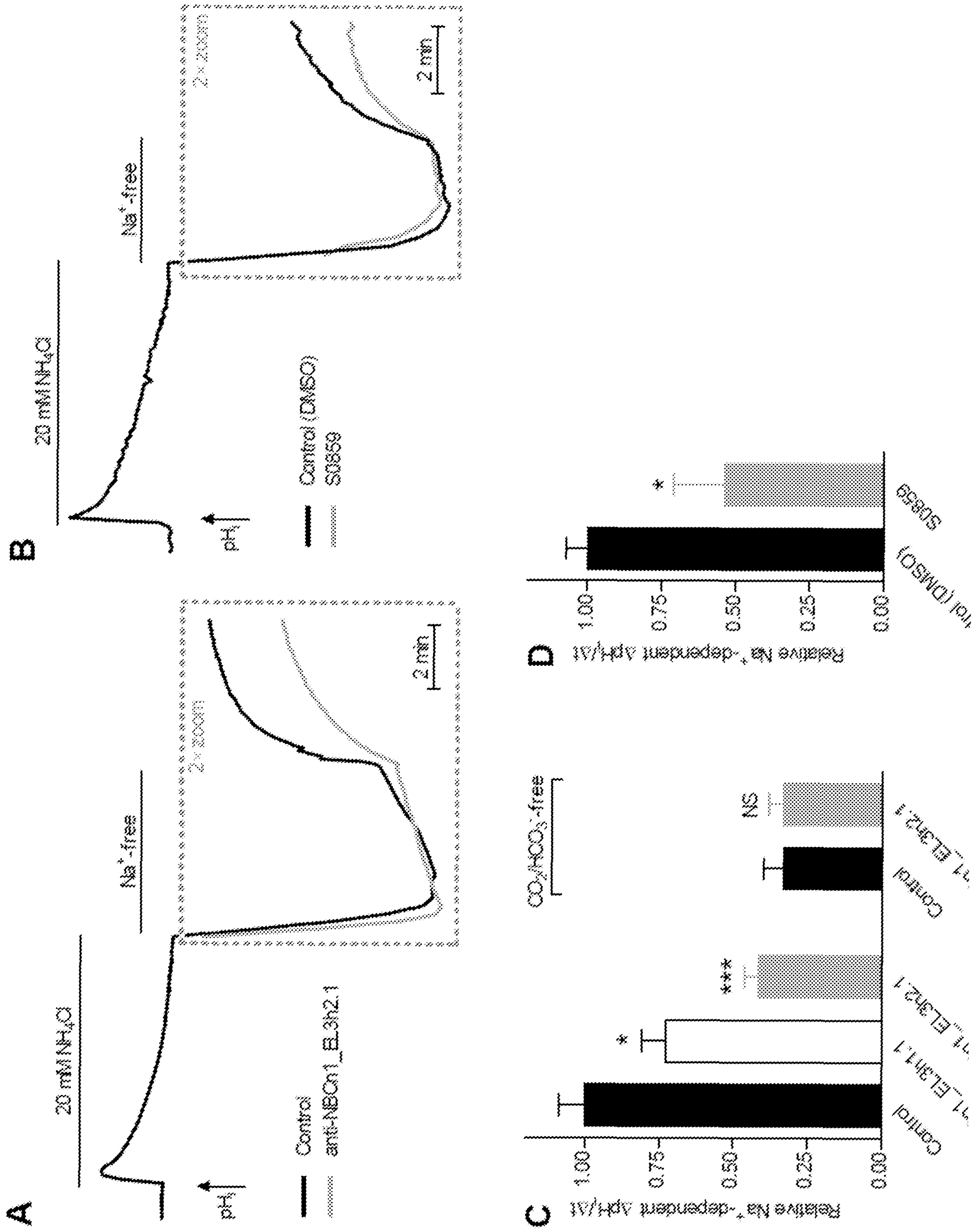
A
20 mM NH$_4$Cl
Na$^+$-free
2× zoom
2 min
pH$_i$
Control
anti-NBCn1_EL3h2.1
B
20 mM NH$_4$Cl
Na$^+$-free
2× zoom
2 min
pH$_i$
Control (DMSO)
S0859
C
Relative Na$^+$-dependent ΔpH/Δt
1.00
0.75
0.50
0.25
0.00
*
***
NS
CO$_2$/HCO$_3^-$-free
Control
Control
D
Relative Na$^+$-dependent ΔpH/Δt
1.00
0.75
0.50
0.25
0.00
*
S0859

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9409131 A
- WO 9413806 A
- WO 0243478 A
- US 20030118592 A
- US 20030133939 A
- EP 404097 A
- WO 9311161 A
- WO 9111465 A, Goldenberg
- US 4331647 A
- US 4946778 A, Ladner
- US 5223409 A
- US 5403484 A, Ladner
- US 5571698 A, Ladner
- US 5693762 A, Queen
- US 5208146 A, Irie
- US 5637677 A, Greene


### Non-patent literature cited in the description


- *Curr. Pharm. Des,* 2012, vol. 18, 1372-1382
- **CHICHE J et al.** In vivo pH in metabolic-defective Ras-transformed fibroblast tumors: key role of the monocarboxylate transporter, MCT4, for inducing an alkaline intracellular pH. *Int. J. Cancer,* 2012, vol. 130, 1511-1520
- **DE MILITO A ; MARINO ML ; FAIS S.** A rationale for the use of proton pump inhibitors as antineoplastic agents. *Curr. Pharm. Des,* 2012, vol. 18, 1395-1406
- **SILVA AS ; YUNES JA ; GILLIES RJ ; GATENBY RA.** The potential role of systemic buffers in reducing intratumoral extracellular pH and acid-mediated invasion. *Cancer Res.,* 2009, vol. 69, 2677-2684
- **ROBERTSON N ; POTTER C ; HARRIS AL.** Role of carbonic anhydrase IX in human tumor cell growth, survival, and invasion. *Cancer Res.,* 2004, vol. 64, 6160-6165
- **SWIETACH P ; PATIAR S ; SUPURAN CT ; HARRIS AL ; VAUGHAN-JONES RD.** The role of carbonic anhydrase 9 in regulating extracellular and intracellular ph in three-dimensional tumor cell growths. *J. Biol. Chem.,* 2009, vol. 284, 20299-20310
- **BOEDTKJER E et al.** Contribution of Na+,HCO3--cotransport to cellular pH control in human breast cancer: a role for the breast cancer susceptibility locus NBCn1 (SLC4A7). *Int. J. Cancer,* 2013, vol. 132, 1288-1299
- **DAMKIER, H.H. ; NIELSEN, S. ; PRAETORIUS, J.** An anti-NH2-terminal antibody localizes NBCn1 to heart endothelia and skeletal and vascular smooth muscle cells. *Am J Physiol Heart Circ Physiol,* 2006, vol. 290, H172-80
- **KOHLER ; MILSTEIN.** *Nature,* 1975, vol. 256, 495
- Antibodies: A Laboratory Manual. Cold Spring Harbor Laboratory Press, 1988
- **GREENBERG, A. S. ; AVILA, D. ; HUGHES, M. ; HUGHES, A. ; MCKINNEY, E. C. ; FLAJNIK, M. F.** *Nature,* 1995, vol. 374, 168-173
- **LONBERG, N. et al.** *Nature,* 1994, vol. 368 (6474), 856-859
- **BIRD et al.** *Science,* 1988, vol. 242, 423-426
- **HUSTON et al.** *Proc. Natl. Acad. Sci. USA,* 1988, vol. 85, 5879-5883
- **WARD.** *Nature,* 1989, vol. 341, 544-546
- **HOLLINGER et al.** *Proc. Natl. Acad Sci. USA,* 1993, vol. 90, 6444-6448
- **MANDRUP OA ; FRIIS NA ; LYKKEMARK S ; JUST J ; KRISTENSEN P.** A novel heavy domain antibody library with functionally optimized complementarity determining regions. *PLoS One,* 2013, vol. 8, e76834
- **SORENSEN MD ; AGERHOLM IE ; CHRISTENSEN B ; KOLVRAA S ; KRISTENSEN P.** Microselection--affinity selecting antibodies against a single rare cell in a heterogeneous population. *J Cell Mol Med,* 2010, vol. 14, 1953-1961
- **SORENSEN MD ; KRISTENSEN P.** Selection of antibodies against a single rare cell present in a heterogeneous population using phage display. *Nat Protoc,* 2011, vol. 6, 509-522
- **SORENSEN MD ; MELCHJORSEN CJ ; MANDRUP OA ; KRISTENSEN P.** Raising antibodies against circulating foetal cells from maternal peripheral blood. *Prenat Diagn,* 2013, vol. 33, 284-291
- Production of Polyclonal Antisera. **GREEN et al.** Immunochemical Protocols. Humana Press, 1992, 1-5
- Expression of foreign proteins in E. coli using plasmid vectors and purification of specific polyclonal antibodies. **WILLIAMS et al.** DNA Cloning 2: Expression Systems. Oxford University Press, 1995, 15

- **LOSMAN et al.** *Int. J. Cancer,* 1990, vol. 46, 310
- **KOHLER et al.** *Nature,* 1975, vol. 256, 495
- Current Protocols in Immunology. John Wiley & Sons, 1991, vol. 1
- Production of monoclonal antibodies against proteins expressed in E. coli. **PICKSLEY et al.** DNA Cloning 2: Expression Systems. Oxford University Press, 1995, 9
- **GREEN et al.** *Nature Genet,* 1994, vol. 7, 13
- **LONBERG et al.** *Nature,* 1994, vol. 368, 856
- **TAYLOR et al.** *Int. Immun,* 1994, vol. 6, 579
- Purification of Immunoglobulin G (IgG). **BAINES et al.** Methods in Molecular Biology. The Humana Press, Inc, 1992, vol. 10, 79-104
- **NISONOFF et al.** *Arch Biochem. Biophys.,* 1960, vol. 89, 230
- **PORTE.** *Biochem. J.,* 1959, vol. 73, 119
- **EDELMAN ; COLIGAN et al.** Methods in Enzymology. Academic Press, 1967, vol. 1
- **INBAR et al.** *Proc. Nat'l Acad. Sci. USA,* 1972, vol. 69, 2659
- **SANDHU.** *Crit. Rev. Biotech,* 1992, vol. 12, 437
- **WHITLOW et al.** *Methods: A Companion to Methods in Enzymology,* 1991, vol. 2, 97
- **BIRD et al.** *Science,* 1988, vol. 242, 423
- **PACK et al.** *Bio/Technology,* 1993, vol. 11, 1271
- **KAY et al.** Phage Display of Peptides and Proteins. Academic Press, Inc, 1996
- **LARRICK et al.** *Methods: A Companion to Methods in Enzymology,* 1991, vol. 2, 106
- Genetic Manipulation of Monoclonal Antibodies. **COURTENAY-LUCK et al.** Monoclonal Antibodies: Production, Engineering and Clinical Application. Cambridge University Press, 1995, 166
- Genetic Manipulation and Expression of Antibodies. **WARD et al.** Monoclonal Antibodies: Principles and Applications. Wiley-Liss, Inc, 1995, 137
- **ORLANDI et al.** *Proc. Nat'l Acad. Sci. USA,* 1989, vol. 86, 3833
- **JONES et al.** *Nature,* 1986, vol. 321, 522
- **CARTER et al.** *Proc. Nat'l Acad. Sci. USA,* 1992, vol. 89, 4285
- **SANDHU.** *Crit. Rev. Biotech.,* 1992, vol. 12, 437
- **SINGER et al.** *J. Immun.,* 1993, vol. 150, 2844
- Antibody Engineering Protocols. Humana Press, Inc, 1995
- Engineering Therapeutic Antibodies. **KELLEY et al.** Protein Engineering: Principles and Practice. John Wiley & Sons, Inc, 1996, 399-434
- Production of Polyclonal Antisera. **GREEN et al.** Methods In Molecular Biology. Humana Press, 1992, 1-12
- **VARTHAKAVI ; MINOCHA.** *J. Gen. Virol.,* 1996, vol. 77, 1875
- **HULIKOVA A ; HARRIS AL ; VAUGHAN-JONES RD ; SWIETACH P.** Regulation of intracellular pH in cancer cell lines under normoxia and hypoxia. *J Cell Physio,* 2013, vol. 228, 743-752
- **BORON, W. F. ; DE WEER, P.** Intracellular pH transients in squid giant axons caused by CO2, NH3, and metabolic inhibitors. *J Gen Physiol,* 1976, vol. 67, 91-112
- **HEIDTMANN, H. ; RUMINOT, I. ; BECKER, H. M. ; DEITMER, J. W.** Inhibition of monocarboxylate transporter by N-cyanosulphonamide S0859. *Eur J Pharmacol,* 2015, vol. 76, 344-349
- **LARSEN, A. M. ; KROGSGAARD-LARSEN, N. ; LAURITZEN, G. ; OLESEN, C. W. ; HONORÉ HANSEN, S. ; BOEDTKJER, E. ; PEDERSEN, S. F. ; BUNCH, L.** Gram-scale solution-phase synthesis of selective sodium bicarbonate co-transport inhibitor S0859: in vitro efficacy studies in breast cancer cells. *ChemMedChem,* 2012, vol. 7, 1808-1814
- **LAURITZEN, G. ; JENSEN, M. B. ; BOEDTKJER, E. ; DYBBOE, R. ; AALKJAER, C. ; NYLANDSTED, J. ; PEDERSEN, S. F.** NBCn1 and NHE1 expression and activity in ΔNErbB2 receptor-expressing MCF-7 breast cancer cells: Contributions to pHi regulation and chemotherapy resistance. *Exp Cell Res,* 2010, vol. 316, 2538-2553
- **STEINKAMP, A.-D. ; SELING, N. ; LEE, S. ; BOEDTKJER, E. ; BOLM, C.** Synthesis of N-cyano-substituted sulfilimine and sulfoximine derivatives of S0859 and their biological evaluation as sodium bicarbonate co-transport inhibitors. *MedChemComm,* 2015, vol. 6, 2163-2169